# EUROPEAN PATENT APPLICATION

(11) **EP 3 882 266 A1**
(43) Date of publication of application: **22.09.2021**
(21) Application number: 19872549.1
(22) Date of filing: 17.10.2019
(51) Int. Cl.: C07K 16/28, A61P 35/00, A61P 37/00, A61K 47/68, A61K 39/00

(54) **BINDING MOLECULE SPECIFIC TO LRIG-1 PROTEIN, AND USE THEREOF**

(30) Priority: 17.10.2018 KR 20180123858
(71) Applicant: Good T Cells, Inc., Seoul 03722 (KR)
(72) Inventor: KIM, Jung Ho, Seoul 04136 (KR); KIM, Beom Seok, Seoul 04029 (KR)
(74) Representative: Schnappauf, Georg
(86) International application number: PCT/KR2019/013666
(87) International publication number: WO 2020/080853

(57) **Abstract**

The present invention relates to a binding molecule capable of specifically binding to Lrig-1 protein, which is located on the surface of a regulatory T cell. The binding molecule provided in the present invention can activate the function of regulatory T cells to effectively prevent, ameliorate, or treat diseases caused by excessive activation or expression of various immune cells and inflammatory cells, for example, immune-related diseases such as autoimmune diseases, graft-versus-host diseases, organ transplant rejection, asthma, atopy, and acute or chronic inflammatory diseases. In addition, the binding molecule, preferably the antibody, specific for the Lrig-1 protein according to the present invention has advantages of more effectively targeting the Lrig-1 protein as compared with antibodies against Lrig-1 which are previously commercially available, and also possessing very good binding capacity thereto.

## Description

### Technical Field

The present invention relates to a binding molecule capable of specifically binding to leucine-rich and immunoglobulin-like domains 1 (Lrig-1) protein, which is a protein present on the surface of regulatory T cells (Treg cells), and a use thereof.

### Background Art

One of the most important traits in all normal individuals is to have the ability to recognize and eliminate non-self antigens, while not detrimentally responding to antigenic substances that make up the self. As such, non-response of the living body to self antigens is called immunologic unresponsiveness or tolerance. Self-tolerance occurs by eliminating lymphocytes that may have specific receptors for self antigens, or by self-inactivation of the ability to respond after contacting self antigens. In a case where a problem arises in inducing or maintaining self-tolerance, an immune response to self antigens occurs, and the disease resulting therefrom is called an autoimmune disease.

For the treatment of the autoimmune disease, a concept of suppressor T cells suggesting the possibility of presence of T cells capable of controlling and suppressing the effector function of conventional T cells was introduced and presented for the first time by Gershon in the early 1970s (R. K. Gershon and K. Kondo, Immunology, 1970, 18: 723-37). Since then, studies have been conducted to elucidate biological properties and functions of regulatory T cells in many areas of immunology.

In this connection, it has been reported that the regulatory T cells (Treg cells) play an important role in naturally preventing occurrence of excessive inflammation and immune responses; however, in a case where an autoimmune disease and a chronic inflammatory disease occur, the function and the number of the regulatory T cells are remarkably decreased. Therefore, in a case of patients with immune and inflammatory diseases, it is important that the regulatory T cells are produced at a normal level, which can be one of the treatments for these diseases.

Until now, studies on genes and proteins which are present specifically in regulatory T cells have been conducted, and it has been presented that substances such as CD25, CTLA4, CD62L, CD38, CD103, GITR, and CD45RB may correspond to marker substances. However, there are no genes and proteins that can target only the regulatory T cells alone.

On the other hand, there are three hypervariable regions called complementarity determining regions (hereinafter referred to as "CDRs") and four framework regions. The CDRs primarily serve to bind to an epitope on an antigen. The CDRs of each chain are typically referred to as CDR1, CDR2, and CDR3 sequentially starting from the N-terminus, and are also distinguished by the chain where particular CDRs are located.

### Technical Problem

An object of the present invention is to provide a binding molecule specific for Lrig-1 protein present on the surface of regulatory T cells (Treg cells).

Another object of the present invention is to provide a nucleic acid molecule which encodes the binding molecule according to the present invention.

Yet another object of the present invention is to provide an expression vector into which the nucleic acid molecule according to the present invention is inserted.

Still yet another object of the present invention is to provide a host cell line transfected with the expression vector according to the present invention.

Still yet another object of the present invention is to provide an antibody-drug conjugate according to the present invention.

Still yet another object of the present invention is to provide a pharmaceutical composition for preventing or treating an immune-related disease, comprising the binding molecule according to the present invention.

Still yet another object of the present invention is to provide a method for preventing or treating an immune-related disease, comprising a step of administering, to an individual, a pharmaceutically effective amount of the binding molecule or the antibody-drug conjugate (ADC) provided in the present invention.

However, the technical problem to be solved by the present invention is not limited to the above-mentioned problems, and other problems which are not mentioned will be clearly understood by those skilled in the art from the following description.

### Solution to Problem

The present inventors have discovered Lrig-1 protein that is present specifically on the surface of regulatory T cells, have selected an epitope on the protein, and have produced a monoclonal antibody capable of specifically binding to the Lrig-1 protein, thereby completing the present invention.

According to an embodiment of the present invention, there is provided a binding molecule which specifically binds to leucine-rich and immunoglobulin-like domains 1 (Lrig-1) protein.

As used herein, the term "binding molecule" refers to a variable domain comprising an intact immunoglobulin that includes a monoclonal antibody, such as a chimeric, humanized, or human monoclonal antibody, or an immunoglobulin that binds to an antigen, for example, an immunoglobulin fragment that competes with intact immunoglobulins for binding to monomeric HA or trimeric HA of influenza A virus. Regardless of the structure, an antigen-binding fragment binds to the same antigen recognized by intact immunoglobulins. The antigen-binding fragment may include a peptide or polypeptide which contains, out of the amino acid sequence of the binding molecule, an amino acid sequence of two or more contiguous residues, 20 or more contiguous amino acid residues, 25 or more contiguous amino acid residues, 30 or more contiguous amino acid residues, 35 or more contiguous amino acid residues, 40 or more contiguous amino acid residues, 50 or more contiguous amino acid residues, 60 or more contiguous amino acid residues, 70 or more contiguous amino acid residues, 80 or more contiguous amino acid residues, 90 or more contiguous amino acid residues, 100 or more contiguous amino acid residues, 125 or more contiguous amino acid residues, 150 or more contiguous amino acid residues, 175 or more contiguous amino acid residues, 200 or more contiguous amino acid residues, or 250 or more contiguous amino acid residues. The term "antigen-binding fragment", in particular, includes Fab, F(ab'), F(ab')2, Fv, dAb, Fd, complementarity determining region (CDR) fragments, single-chain antibodies (scFvs), bivalent single-chain antibodies, single-chain phage antibodies, unibodies, diabodies, triabodies, tetrabodies, polypeptides containing at least one fragment of immunoglobulin which is sufficient for a particular antigen to bind to the polypeptide, and the like. The fragment may be produced synthetically or by enzymatic or chemical digestion of a complete immunoglobulin, or may be produced by genetic engineering methods using recombinant DNA techniques. Production methods are well known in the art.

In the present invention, the Lrig-1 protein is a transmembrane protein consisting of 1091 amino acids present on the surface of regulatory T cells, and is composed of leucine-rich repeats (LRRs) and three immunoglobulin-like domains on the extracellular or lumen side, a cell transmembrane sequence, and a cytoplasmic tail portion. The LRIG gene family includes LRIG1, LRIG2, and LRIG3, and the amino acids therebetween are highly conserved. The LRIG1 gene is highly expressed in normal skin and can be expressed in basal and hair follicle cells to regulate proliferation of epithelial stem cells. Therefore, the LRIG1 gene plays an important role in maintaining homeostasis of the epidermis, and its absence may develop psoriasis or skin cancer. It has been reported that in a case where chromosome 3p14.3 portion in which LRIG1 is located is cut off, there is a possibility of developing into cancer cells. In fact, it was identified that expression of LRIG1 is greatly decreased in renal cell carcinoma and cutaneous squamous cell carcinoma. Recently, it has been also found that Lrig-1 is expressed in only about 20 to 30% of cancers. On the other hand, for the purpose of the present invention, the Lrig-1 protein may be, but is not limited to, a protein present in humans or mice.

In the present invention, the Lrig-1 protein may be, but is not limited to, a human-derived polypeptide represented by SEQ ID NO: 1 or a mouse-derived polypeptide represented by SEQ ID NO: 3.

In addition, in the present invention, the Lrig-1 protein represented by SEQ ID NO: 1 may be encoded by a polynucleotide represented by SEQ ID NO: 2, but is not limited thereto.

In addition, in the present invention, the Lrig-1 protein represented by SEQ ID NO: 3 may be encoded by a polynucleotide represented by SEQ ID NO: 4, but is not limited thereto.

In the present invention, the binding molecule may be a binding molecule, comprising:
a heavy chain variable region that contains a heavy chain CDR1 consisting of an amino acid sequence selected from the group consisting of SEQ ID NOs: 5, 13, 21, and 29; a heavy chain CDR2 consisting of an amino acid sequence selected from the group consisting of SEQ ID NOs: 6, 14, 22, and 30; a heavy chain CDR3 consisting of an amino acid sequence selected from the group consisting of SEQ ID NOs: 7, 15, 23, and 31; and
a light chain variable region that contains a light chain CDR1 consisting of an amino acid sequence selected from the group consisting of SEQ ID NOs: 8, 16, 24, and 32; a light chain CDR2 consisting of an amino acid sequence selected from the group consisting of SEQ ID NOs: 9, 17, 25, and 33; a light chain CDR3 consisting of an amino acid sequence selected from the group consisting of SEQ ID NO: 10, 18, 26, and 34.

In the present invention, the binding molecule may be a binding molecule, comprising:
a heavy chain variable region, selected from the group consisting of the following (a) to (d):
   (a) a heavy chain variable region that contains a heavy chain CDR1 represented by SEQ ID NO: 5, a heavy chain CDR2 represented by SEQ ID NO: 6, and a heavy chain CDR3 represented by SEQ ID NO: 7;
   (b) a heavy chain variable region that contains a heavy chain CDR1 represented by SEQ ID NO: 13, a heavy chain CDR2 represented by SEQ ID NO: 14, and a heavy chain CDR3 represented by SEQ ID NO: 15;
   (c) a heavy chain variable region that contains a heavy chain CDR1 represented by SEQ ID NO: 21, a heavy chain CDR2 represented by SEQ ID NO: 22, and a heavy chain CDR3 represented by SEQ ID NO: 23; and
   (d) a heavy chain variable region that contains a heavy chain CDR1 represented by SEQ ID NO: 29, a heavy chain CDR2 represented by SEQ ID NO: 30, and a heavy chain CDR3 represented by SEQ ID NO: 31; and
a light chain variable region, selected from the group consisting of the following (e) to (h):
   (e) a light chain variable region that contains a light chain CDR1 represented by SEQ ID NO: 8, a light chain CDR2 represented by SEQ ID NO: 9, and a light chain CDR3 represented by SEQ ID NO: 10;
   (f) a light chain variable region that contains a light chain CDR1 represented by SEQ ID NO: 16, a light chain CDR2 represented by SEQ ID NO: 17, and a light chain CDR3 represented by SEQ ID NO: 18;
   (g) a light chain variable region that contains a light chain CDR1 represented by SEQ ID NO: 24, a light chain CDR2 represented by SEQ ID NO: 25, and a light chain CDR3 represented by SEQ ID NO: 26;
   (h) a light chain variable region that contains a light chain CDR1 represented by SEQ ID NO: 32, a light chain CDR2 represented by SEQ ID NO: 33, and a light chain CDR3 represented by SEQ ID NO: 34.

In the present invention, the binding molecule may be a binding molecule selected from the group consisting of the following (1) to (4):
(1) a binding molecule comprising a heavy chain variable region that contains a heavy chain CDR1 represented by SEQ ID NO: 5, a heavy chain CDR2 represented by SEQ ID NO: 6, and a heavy chain CDR3 represented by SEQ ID NO: 7; and a light chain variable region that contains a light chain CDR1 represented by SEQ ID NO: 8, a light chain CDR2 represented by SEQ ID NO: 9, and a light chain CDR3 represented by SEQ ID NO: 10;
(2) a binding molecule comprising a heavy chain variable region that contains a heavy chain CDR1 represented by SEQ ID NO: 13, a heavy chain CDR2 represented by SEQ ID NO: 14, and a heavy chain CDR3 represented by SEQ ID NO: 15; and a light chain variable region that contains a light chain CDR1 represented by SEQ ID NO: 16, a light chain CDR2 represented by SEQ ID NO: 17, and a light chain CDR3 represented by SEQ ID NO: 18;
(3) a binding molecule comprising a heavy chain variable region that contains a heavy chain CDR1 represented by SEQ ID NO: 21, a heavy chain CDR2 represented by SEQ ID NO: 22, and a heavy chain CDR3 represented by SEQ ID NO: 23; and a light chain variable region that contains a light chain CDR1 represented by SEQ ID NO: 24, a light chain CDR2 represented by SEQ ID NO: 25, and a light chain CDR3 represented by SEQ ID NO: 26;
(4) a binding molecule comprising a heavy chain variable region that contains a heavy chain CDR1 represented by SEQ ID NO: 29, a heavy chain CDR2 represented by SEQ ID NO: 30, and a heavy chain CDR3 represented by SEQ ID NO: 31; and a light chain variable region that contains a light chain CDR1 represented by SEQ ID NO: 32, a light chain CDR2 represented by SEQ ID NO: 33, and a light chain CDR3 represented by SEQ ID NO: 34.

In the present invention, the binding molecule may be a binding molecule, comprising:
a heavy chain variable region consisting of any one amino acid sequence selected from the group consisting of SEQ ID NOs: 11, 19, 27, and 35; and
a light chain variable region consisting of any one amino acid sequence selected from the group consisting of SEQ ID NO: 12, 20, 28, and 36.

In the present invention, the binding molecule may be a binding molecule selected from the group consisting of the following binding molecules:
(i) a binding molecule comprising a heavy chain variable region represented by SEQ ID NO: 11, and a light chain variable region represented by SEQ ID NO: 12;
(ii) a binding molecule comprising a heavy chain variable region represented by SEQ ID NO: 19, and a light chain variable region represented by SEQ ID NO: 20;
(iii) a binding molecule comprising a heavy chain variable region represented by SEQ ID NO: 27, and a light chain variable region represented by SEQ ID NO: 28; and
(iv) a binding molecule comprising a heavy chain variable region represented by SEQ ID NO: 35, and a light chain variable region represented by SEQ ID NO: 36.

In the present invention, the binding molecule may further comprise a fragment crystallization (Fc) region or a constant region. Here, the Fc region may be an Fc region of an IgG1, IgG2, IgG3, or IgG4 antibody, or may be derived therefrom. Alternatively, the Fc region may be a hybrid Fc region.

In the present invention, the Fc region may be an Fc region of a mammalian-derived IgG1, IgG2, IgG3, or IgG4 antibody, and may preferably be an Fc region of a human-derived IgG1, IgG2, IgG3, or IgG4 antibody. However, the present invention is not limited thereto.

As an example of the present invention, the constant region may be a mouse-derived IgG2a constant region represented by SEQ ID NO: 37, but is not limited thereto.

As an example of the present invention, the constant region may be a mouse-derived immunoglobulin kappa constant region represented by SEQ ID NO: 38, but is not limited thereto.

As an example of the present invention, the constant region may be a human-derived IgG1 constant region represented by SEQ ID NO: 39 or 53, but is not limited thereto.

As an example of the present invention, the constant region may be a human-derived immunoglobulin kappa constant region represented by SEQ ID NO: 40, but is not limited thereto.

As an example of the present invention, the constant region may be a human-derived IgG2 constant region represented by SEQ ID NO: 41, but is not limited thereto.

As an example of the present invention, the constant region may be a human-derived IgG3 constant region represented by SEQ ID NO: 42, but is not limited thereto.

As an example of the present invention, the constant region may be a human-derived IgG4 constant region represented by SEQ ID NO: 43, but is not limited thereto.

As an example of the present invention, the Fc region may be a human-derived immunoglobulin lambda constant region, but is not limited thereto.

In the present invention, the "hybrid Fc" may be derived from a combination of human IgG subclasses or a combination of human IgD and IgG. In a case where the hybrid Fc binds to a biologically active molecule, polypeptide, or the like, the hybrid Fc has effects of not only increasing a serum half-life of the biologically active molecule, but also increasing an expression level of the polypeptide when a nucleotide sequence encoding the Fc-polypeptide fusion protein is expressed.

As an example of the present invention, the hybrid Fc region may be a hybrid Fc represented by SEQ ID NO: 44, but is not limited thereto.

In the binding molecule of the present invention, the Fc or constant region may be linked, via a linker, to the variable region. Here, the linker may be linked to the C-terminus of the Fc or constant region, and the N-terminus of the binding molecule of the present invention may be linked to the linker. However, the present invention is not limited thereto.

In the present invention, the "linker" may contain a sequence that can be cleaved by an enzyme that is overexpressed in a tissue or cell having a target disease. In a case where the linker may be cleaved by the overexpressed enzyme as described above, it is possible to effectively prevent activity of a polypeptide from decreasing due to the Fc or constant region. In the present invention, an example of the linker may be preferably a peptide linker consisting of 33 amino acids located in the 282^{nd} to 314^{th} portion of human albumin which is most abundantly present in the blood, and more preferably a peptide linker consisting of 13 amino acids located in the 292^{nd} to 304^{th} portion of the human albumin. Such portions are portions which are mostly exposed to the outside in three-dimensional structure, and thus have a minimum possibility of inducing an immune response in the body. However, the linker is not limited thereto.

The binding molecule of the present invention may further comprise a heavy chain constant region consisting of an amino acid sequence selected from the group consisting of SEQ ID NOs: 37, 39, 41, 42, 43, 44, and 53.

The binding molecule of the present invention may further comprise a light chain constant region consisting of an amino acid sequence represented by SEQ ID NO: 38 or 40.

The binding molecule of the present invention may further comprise:
a heavy chain constant region consisting of an amino acid sequence represented by SEQ ID NO: 37; and
a light chain constant region consisting of an amino acid sequence represented by SEQ ID NO: 38.

The binding molecule of the present invention may further comprise:
a heavy chain constant region consisting of an amino acid sequence represented by SEQ ID NO: 39, 41, 42, 43, and 53; and
a light chain constant region consisting of an amino acid sequence represented by SEQ ID NO: 40.

The binding molecule of the present invention may further comprise:
a heavy chain constant region consisting of an amino acid sequence represented by SEQ ID NO: 44.

The binding molecule of the present invention may be a binding molecule selected from the group of the following binding molecules:
a binding molecule comprising a heavy chain represented by SEQ ID NO: 45, and a light chain represented by SEQ ID NO: 46;
a binding molecule comprising a heavy chain represented by SEQ ID NO: 47, and a light chain represented by SEQ ID NO: 48;
a binding molecule comprising a heavy chain represented by SEQ ID NO: 49, and a light chain represented by SEQ ID NO: 50; and
a binding molecule comprising a heavy chain represented by SEQ ID NO: 51, and a light chain represented by SEQ ID NO: 52. The binding molecule of the present invention is characterized by being an antibody, but is not limited thereto. The antibody includes all of a monoclonal antibody, a full-length antibody, or an antibody fragment which is a portion of an antibody, has the ability to bind to Lrig-1 protein, and can compete with the binding molecule of the present invention in binding to an epitope on Lrig-1.

As used herein, the term "antibody" refers to a protein molecule which serves as a receptor that specifically recognizes an antigen, including an immunoglobulin molecule that is immunologically reactive with a particular antigen. For the purpose of the present invention, the antigen may be Lrig-1 protein present on the surface of regulatory T cells. Preferably, the antibody may specifically recognize the leucine-rich region or immunoglobulin-like domain of the Lrig-1 protein, but is not limited thereto.

In the present invention, the "immunoglobulin" has a heavy chain and a light chain, and each of the heavy chain and the light chain comprises a constant region and a variable region. The variable region of each of the light chain and the heavy chain contains three hypervariable regions called complementarity determining regions (hereinafter referred to as "CDRs") and four framework regions. The CDRs primarily serve to bind to an epitope on an antigen. The CDRs of each chain are typically referred to as CDR1, CDR2, and CDR3 sequentially starting from the N-terminus, and are also distinguished by the chain where particular CDRs are located.

In addition, as used herein, the term "monoclonal antibody" refers to an antibody molecule of a single molecular composition which is obtained from substantially the same antibody population, and exhibits single binding specificity and affinity for a particular epitope.

In the present invention, the "full-length antibody" has a structure with two full-length light chains and two full-length heavy chains in which each light chain is linked to a heavy chain by disulfide bond, and includes IgA, IgD, IgE, IgM, and IgG. The IgG includes, as subtypes thereof, IgG1, IgG2, IgG3, and IgG4.

In addition, as used herein, the term "antigen fragment " refers to a fragment that retains an antigen-binding function, and includes Fab, Fab', F(ab')₂, Fv, and the like. The Fab has a structure with variable regions of light and heavy chains, a constant region of the light chain, and a first constant region (CH1 domain) of the heavy chain, and has one antigen-binding site. In addition, Fab' is different from Fab in that Fab' has a hinge region containing at least one cysteine residue at the C-terminus of the heavy chain CH1 domain. F(ab')₂ antibodies are produced with cysteine residues at the hinge region of Fab' forming disulfide bond. Fv (variable fragment) refers to the smallest antibody fragment having only a heavy chain variable region and a light chain variable region. Double-chain Fv (dsFv) is configured to be such that a heavy chain variable region and a light chain variable region are linked to each other by disulfide bond, and single-chain Fv (scFv) is configured to be such that a heavy chain variable region and a light chain variable region are covalently linked to each other, in general, via a peptide linker. The antibody fragment may be obtained as Fab or F(ab')₂ fragment in a case where a proteolytic enzyme, for example, papain or pepsin is used, and may be produced through a genetic recombinant technique.

In addition, in the present invention, the antibody may be, but is not limited to, a chimeric antibody, a humanized antibody, a bivalent, bispecific molecule, a minibody, a domain antibody, a bispecific antibody, an antibody mimetic, a unibody, a diabody, a triabody, or a tetrabody, or a fragment thereof.

In the present invention, the "chimeric antibody" is an antibody which is obtained by recombination of a variable region of a mouse antibody and a constant region of a human antibody, and has a greatly improved immune response as compared with the mouse antibody.

In addition, as used herein, the term "humanized antibody" refers to an antibody obtained by modifying a protein sequence of an antibody derived from a non-human species so that the protein sequence is similar to an antibody variant naturally produced in humans. For example, the humanized antibody may be prepared as follows. Mouse-derived CDRs may be recombined with a human antibody-derived FR to prepare a humanized variable region, and the humanized variable region may be recombined with a constant region of a preferred human antibody to prepare a humanized antibody.

In the present invention, the binding molecule may be provided as a bispecific antibody or a bispecific antigen-binding fragment which is capable of binding to Lrig-1 protein and also binding to another protein.

In the present invention, the bispecific antibody and the bispecific antigen-binding fragment may comprise the binding molecule according to the present invention. As an example of the present invention, the bispecific antibody and the bispecific antigen-binding fragment comprise an antigen-binding domain capable of binding to Lrig-1 protein, wherein the antigen-binding domain capable of binding to Lrig-1 protein may comprise or consist of the binding molecule according to the present invention.

The bispecific antibody and the bispecific antigen-binding fragment provided in the present invention comprise an antigen-binding domain, which is a binding molecule capable of binding to Lrig-1 protein according to the present invention, and an antigen-binding domain capable of binding to another target protein. Here, the antigen-binding domain capable of binding another target protein may be an antigen-binding domain capable of binding to a protein other than Lrig-1 protein, for example, PD-1 or a cell surface receptor. However, the antigen-binding domain is not limited thereto.

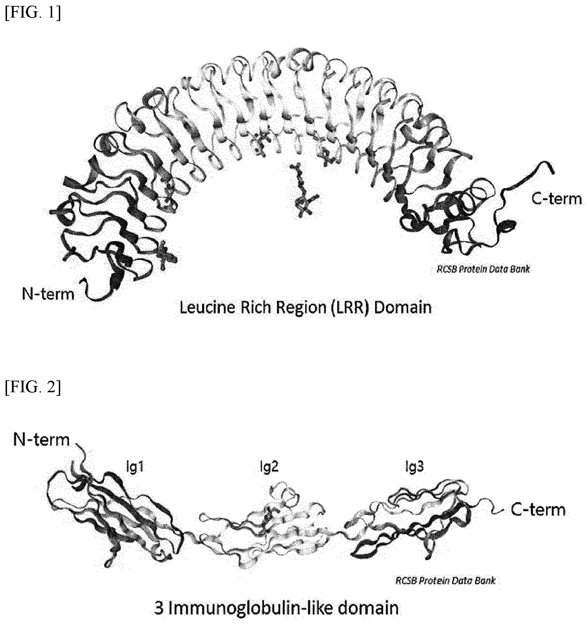

The bispecific antibody and the bispecific antigen-binding fragment according to the present invention may be provided in any suitable format, for example, that described in Kontermann MAbs 2012, 4(2): 182-197, which is incorporated herein by reference in its entirety. For example, the bispecific antibody or the bispecific antigen-binding fragment may be a bispecific antibody conjugate (for example, IgG2, F(ab')2, or CovX-body), a bispecific IgG or IgG-like molecule (for example, IgG, scFv4-Ig, IgG-scFv, scFv-IgG, DVD-Ig, IgG-sVD, sVD-IgG, or 2 in 1-IgG, mAb2, or Tandemab common LC), an asymmetric bispecific IgG or IgG-like molecule (for example, kih IgG, kih IgG common LC, CrossMab, kih IgG-scFab, mAb-Fv, charge pair, or SEED-body), a small bispecific antibody molecule (for example, diabody (Db), dsDb, DART, scDb, tandAb, tandem scFv (taFv), tandem dAb/VHH, triple body, triple head, Fab-scFv, or F(ab')2-scFv2), a bispecific Fc and CH3 fusion protein (for example, taFv-Fc, di-diabody, scDb-CH3, scFv-Fc-scFv, HCAb-VHH, scFv-kih-Fc, or scFv-kih-CH3), or a bispecific fusion protein (for example, scFv2-albumin, scDb-albumin, taFv-toxin, DNL-Fab3, DNL-Fab4-IgG, DNL-Fab4-IgG-cytokine 2). See, in particular, FIG. 2 in Kontermann MAbs 2012, 4(2): 182-19. The bispecific antibody and the bispecific antigen-binding fragment according to the invention may be designed and prepared by those skilled in the art.

A method for producing the bispecific antibody in the present invention comprises forming a reducing disulfide or non-reducing thioether bond, and chemical crosslinking of an antibody or antibody fragment as described, for example, in Segal and Bast, 2001. Production of Bispecific Antibodies. Current Protocols in Immunology. 14:IV:2.13:2.13.1-2.13.16, which is incorporated herein by reference in its entirety. For example, N-succinimidyl-3-(-2-pyridyldithio)-propionate (SPDP) may be used, for example, for chemically crosslinking an Fab fragment through an SH-group at the hinge region, to generate a disulfide-linked bispecific F(ab)2 heterodimer.

In addition, an alternative method for producing the bispecific antibody in the present invention comprises fusing an antibody-producing hybridoma with, for example, polyethylene glycol, to produce quadroma cells capable of secreting bispecific antibodies, as described, for example, in D. M. and Bast, B. J. 2001. Production of Bispecific Antibodies. Current Protocols in Immunology. 14:IV:2.13:2.13.1-2.13.16.

The bispecific antibody and the bispecific antigen-binding fragment according to the invention may also be, for example, recombinantly produced by expression from a nucleic acid construct that encodes a polypeptide for an antigen-binding molecule, as described, for example, in Antibody Engineering: Methods and Protocols, Second Edition (Humana Press, 2012), at Chapter 40: Production of Bispecific Antibodies: Diabodies and Tandem scFv (Hornig and Farber-Schwarz), or French, How to make bispecific antibodies, Methods Mol. Med. 2000; 40:333-339, both of which are incorporated herein by reference in their entireties.

For example, a DNA construct that contains a sequence encoding light and heavy chain variable domains for two antigen-binding domains (that is, light and heavy chain variable domains for an antigen-binding domain capable of binding to PD-1 or the like, and light and heavy chain variable domains for an antigen-binding domain capable of binding to another target protein), and a sequence encoding a suitable linker or dimerization domain between the antigen-binding domains may be prepared by molecular cloning techniques. Subsequently, a recombinant bispecific antibody may be produced by expression of the construct (for example, *in vitro*) in a suitable host cell (for example, a mammalian host cell), and then the expressed recombinant bispecific antibody may be optionally purified.

Antibodies may be produced by an affinity maturation process in which a modified antibody with improved affinity for an antigen as compared with an unmodified parent antibody is produced. An affinity matured antibody may be produced by a procedure known in the art, for example, in Marks et al., Rio/Technology 10:779-783 (1992); Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-159 (1995); and Hawkins et al, J. Mol. Biol. 226:889-896 (1992).

In addition, the binding molecule provided in the present invention may include a variant of the amino acid sequence as long as the variant can specifically bind to Lrig-1 protein. For example, in order to improve binding affinity and/or other biological properties of an antibody, modifications may be made to an amino acid sequence of the antibody. Such modifications include, for example, deletions, insertions, and/or substitutions of amino acid sequence residues of the antibody.

Such amino acid variations are made based on relative similarity of amino acid side chain substituents such as hydrophobicity, hydrophilicity, charge, and size. According to analysis on sizes, shapes, and types of amino acid side chain substituents, it can be seen that arginine, lysine, and histidine are all positively charged residues; alanine, glycine, and serine have similar sizes; and phenylalanine, tryptophan, and tyrosine have similar shapes. Thus, based on these considerations, it can be said that arginine, lysine, and histidine; alanine, glycine, and serine; and phenylalanine, tryptophan, and tyrosine are biologically functional equivalents.

In introducing variations, the hydropathic index of amino acids may be considered. Each amino acid has been assigned hydropathic index depending on its hydrophobicity and charge: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). The hydropathic amino acid index is very important in conferring the interactive biological function on a protein. It is known that substitution with an amino acid having similar hydropathic index allows a protein to retain similar biological activity. In a case where variations are introduced with reference to the hydropathic index, substitutions are made between amino acids that exhibit a hydropathic index difference of preferably within ± 2, more preferably within ± 1, and even more preferably within ± 0.5.

Meanwhile, it is also well known that substitutions between amino acids having similar hydrophilicity values result in proteins with equivalent biological activity. As disclosed in US Pat. No. 4,554,101, respective amino acid residues have been assigned the following hydrophilicity values: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). In a case where variations are introduced with reference to the hydrophilicity values, substitutions may be made between amino acids that exhibit a hydrophilicity value difference of preferably within ± 2, more preferably within ± 1, and even more preferably within ± 0.5.

Amino acid exchanges in proteins which do not entirely alter activity of a molecule are known in the art (H. Neurath, R.L.Hill, The Proteins, Academic Press, New York (1979)). The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Gln/Glu.

Given the above-described variations with biologically equivalent activity, it is interpreted that the binding molecule of the present invention also includes sequences that exhibit substantial identity with the sequences listed in the Sequence Listing.

As used herein, the term "substantial identity" refers to a sequence showing at least 61% homology, more preferably 70% homology, even more preferably 80% homology, and most preferably 90% homology when the sequence of the present invention is aligned with any other sequence so that they maximally correspond to each other, and the aligned sequence is analyzed by using an algorithm typically used in the art. Alignment methods for comparison of sequences are known in the art. Various methods and algorithms for alignment are disclosed in Smith and Waterman, Adv. Appl. Math. 2:482(1981); Needleman and Wunsch, J. Mol. Bio.48:443(1970); Pearson and Lipman, Methods in Mol. Biol. 24: 307-31(1988); Higgins and Sharp, Gene 73:237-44(1988); Higgins and Sharp, CABIOS 5:151-3(1989); Corpet et al., Nuc. Acids Res. 16:10881-90(1988); Huang et al., Comp. Appl. BioSci. 8:155-65(1992); and Pearson et al., Meth. Mol. Biol. 24:307-31(1994). NCBI Basic Local Alignment Search Tool (BLAST) (Altschul et al., J. Mol. Biol. 215: 403-10 (1990)) is accessible from the National Center for Biological Information (NBCI), or the like, and may be used in conjunction with sequencing programs, such as blastp, blasm, blastx, tblastn, and tblastx, on the internet. BLSAT is accessible at http://www.ncbi.nlm.nih.gov/BLAST/. Sequence homology comparison methods using this program can be identified online (http://www.ncbi.nlm.nih.gov/BLAST/blast_help.html).

In the present invention, the binding molecule, preferably the antibody, may be produced by a conventional method for producing an antibody, and may be produced by affinity maturation.

As used herein, the term "affinity maturation" refers to a process in which antibodies having increased affinity for an antigen are produced by activated B cells in the course of an immune response. For the purpose of the present invention, the affinity maturation allows antibodies or antibody fragments to be produced due to affinity maturation based on the principles of mutation and selection, in the same process that occurs in nature.

The binding molecule, preferably the antibody, provided in the present invention can activate the function, particularly of regulatory T immune cells (Treg cells), among immune cells; increase the number of the Treg cells; and regulate immunological tolerance, thereby effectively preventing, ameliorating, or treating an immune-related disease.

In the present invention, the "immune-related disease" may be a disease induced by excessive activation and expression of various immune cells and inflammatory cells. The immune-related disease may, for example, include autoimmune diseases; graft-versus-host diseases; organ transplant rejection; asthma; atopy; or acute or chronic inflammatory diseases, but is not limited thereto.

In addition, in the present invention, the "autoimmune disease" may be, but is not limited to, one or more selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barre syndrome, chronic thyroiditis, multiple sclerosis, multiple myositis, ankylosing spondylitis, fibrositis, and polyarteritis nodosa.

According to another embodiment of the present invention, there is provided a nucleic acid molecule encoding the binding molecule provided in the present invention.

The nucleic acid molecule of the present invention includes all nucleic acid molecules obtained by translating the amino acid sequences of the binding molecules provided in the present invention to polynucleotide sequences, as known to those skilled in the art. Therefore, various polynucleotide sequences may be prepared by an open reading frame (ORF), and all of these polynucleotide sequences are also included in the nucleic acid molecule of the present invention.

According to yet another embodiment of the present invention, there is provided an expression vector into which the isolated nucleic acid molecule provided in the present invention is inserted.

In the present invention, the "vector" is a nucleic acid molecule capable of transporting another nucleic acid linked thereto. One type of vector is a "plasmid," which refers to circular double-stranded DNA into which an additional DNA segment can be ligated. Another type of vector is a phage vector. Yet another type of vector is a viral vector, where an additional DNA segment can be ligated into the viral genome. Certain vectors are capable of autonomous replication in a host cell into which they are introduced (for example, bacterial vectors having a bacterial origin of replication and episomal mammalian vectors). Other vectors (for example, non-episomal mammalian vectors) can be integrated into the genome of a host cell upon introduction into the host cell, and thus are replicated along with the host genome. In addition, certain vectors are capable of directing expression of genes to which they are operatively linked. Such vectors are referred to herein as "recombinant expression vectors" or simply "expression vectors." In general, expression vectors useful in recombinant DNA techniques are often in the form of plasmids. In the present specification, "plasmid" and "vector" may be used interchangeably as the plasmid is the most commonly used form of vector.

Specific examples of the expression vector in the present invention may be selected from, but are not limited to, the group consisting of commercially widely used pCDNA vectors, F, R1, RP1, Col, pBR322, ToL, Ti vectors; cosmids; phages such as lambda, lambdoid, M13, Mu, p1 P22, Qµµ, T-even, T2, T3, T7; plant viruses. Any expression vector known, to those skilled in the art, as expression vectors can be used in the present invention, and the expression vector is selected depending on the nature of the target host cell. Introduction of a vector into a host cell may be performed by calcium phosphate transfection, viral infection, DEAE-dextran-mediated transfection, lipofectamine transfection, or electroporation. However, the present invention is not limited thereto, and those skilled in the art may adopt and use an introduction method appropriate for the expression vector and the host cell which are used. The vector may preferably contain at least one selection marker. However, the present invention is not limited thereto, and selection can be made using the vector that contains no selection marker, depending on whether or not a product is produced. The selection marker is selected depending on the target host cell, which is done using methods already known to those skilled in the art, and thus the present invention has no limitation thereon.

In the present invention, to facilitate purification of the nucleic acid molecule, a tag sequence may be inserted into and fused to an expression vector. The tag includes, but is not limited to, hexa-histidine tag, hemagglutinin tag, myc tag, or flag tag, and any tag known to those skilled in the art which facilitates purification can be used in the present invention.

According to still yet another embodiment of the present invention, there is provided a host cell line transfected with the expression vector provided in the present invention.

In the present invention, the "host cell" includes individual cells or cell cultures which may be or have been recipients of the vector(s) for incorporation of a polypeptide insert. The host cell includes progeny of a single host cell, and the progeny may not necessarily be completely identical (in morphology or in genomic DNA complement) to the original parent cell due to natural, accidental, or intentional mutation. The host cell includes cells transfected *in vivo* with the polynucleotide(s) herein.

In the present invention, the host cell may include cells of mammalian, plant, insect, fungal, or cellular origin, and may be, for example, bacterial cells such as E. coli, Streptomyces, Salmonella typhimurium; fungal cells such as yeast cells and Pichia pastoris; insect cells such as Drosophila and Spodoptera Sf9 cells; animal cells such as Chinese hamster ovary (CHO) cells, SP2/0 (mouse myeloma), human lymphoblastoid, COS, NSO (mouse myeloma), 293T, Bowes melanoma cells, HT-1080, baby hamster kidney (BHK) cells, human embryonic kidney (HEK) cells, or PERC.6 (human retinal cells); or plant cells. However, the host cell is not limited thereto, and any cell known to those skilled in the art which can be used as a host cell line is available.

In the present invention, the transfection method may be any method of injecting a desired vector into the host cell, and include any known method capable of injecting a vector into a host cell. Examples of the transfection method may include, but are not limited to, CaCh-mediated transfection, electroporation, microinjection, calcium phosphate precipitation, electroporation, liposome-mediated transfection, DEAE-dextran treatment, gene bombardment, and virus-mediated transfection.

According to still yet another embodiment of the present invention, there is provided an antibody-drug conjugate (ADC) comprising the antibody provided in the present invention and a drug.

As used herein, the term "antibody-drug conjugate (ADC)" refers to a form in which the drug and the antibody are chemically linked to each other without degrading biological activity of the antibody and the drug. In the present invention, the antibody-drug conjugate denotes a form in which the drug is bound to an amino acid residue at the N-terminus of the heavy and/or light chain of the antibody, specifically, a form in which the drug is bound to an α-amine group at the N-terminus of the heavy and/or light chain of the antibody.

As used herein, the term "drug" may mean any substance having a certain biological activity for a cell, which is a concept including DNA, RNA, or a peptide. The drug may be in a form which contains a reactive group capable of reacting and crosslinking with an α-amine group, and also includes a form which contains a reactive group capable of reacting and crosslinking with an α-amine group and to which a linker is linked.

In the present invention, examples of the reactive group capable of reacting and crosslinking with the α-amine group are not particularly limited in terms of type as long as the reactive group can react and crosslink with an α-amine group at the N-terminus of a heavy or light chain of an antibody. The reactive group includes all types of groups known in the art which react with an amine group. The reactive group may, for example, be any one of isothiocyanate, isocyanate, acyl azide, NHS ester, sulfonyl chloride, aldehyde, glyoxal, epoxide, oxirane, carbonate, aryl halide, imidoester, carbodiimide, anhydride, and fluorophenyl ester, but is not limited thereto.

In the present invention, the drug includes any drug regardless of type as long as the drug can treat diseases targeted by the Lrig-1 antibody, and may preferably include therapeutic agents for immune-related diseases, for example, autoimmune diseases, graft versus host diseases, organ transplant rejection, asthma, atopy, acute or chronic inflammatory diseases, or the like.

According to still yet another embodiment of the present invention, there is provided a pharmaceutical composition for preventing or treating an immune-related disease, comprising, as an active ingredient, the binding molecule or antibody-drug conjugate (ADC) provided in the present invention.

The binding molecule, preferably the antibody, provided in the present invention can activate the function, particularly of regulatory T immune cells (Treg cells), among immune cells; increase the number of the Treg cells; and regulate immunological tolerance, thereby effectively preventing, ameliorating, or treating an immune-related disease.

In the present invention, the "immune-related disease" may be a disease induced by excessive activation and expression of various immune cells and inflammatory cells. The immune-related disease may, for example, include autoimmune diseases; graft-versus-host diseases; organ transplant rejection; asthma; atopy; or acute or chronic inflammatory diseases, but is not limited thereto.

In addition, in the present invention, the "autoimmune disease" may be, but is not limited to, one or more selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barre syndrome, chronic thyroiditis, multiple sclerosis, multiple myositis, ankylosing spondylitis, fibrositis, and polyarteritis nodosa.

On the other hand, in the present invention, the "prevention" may include, without limitation, any act of blocking symptoms of a disease, or suppressing or delaying the symptoms, using the pharmaceutical composition of the present invention.

In addition, in the present invention, the "treatment" may include, without limitation, any act of ameliorating or beneficially altering symptoms of a disease, using the pharmaceutical composition of the present invention.

In the present invention, the pharmaceutical composition may be characterized by being in the form of capsules, tablets, granules, injections, ointments, powders, or beverages, and the pharmaceutical composition may be characterized by being targeted to humans.

In the present invention, the pharmaceutical composition may be formulated in the form of oral preparations such as powders, granules, capsules, tablets, and aqueous suspensions, preparations for external use, suppositories, and sterile injectable solutions, respectively, according to conventional methods, and used. However, the pharmaceutical composition is not limited thereto. The pharmaceutical composition of the present invention may further comprise a pharmaceutically acceptable carrier. As the pharmaceutically acceptable carrier, a binder, a glidant, a disintegrant, an excipient, a solubilizer, a dispersant, a stabilizer, a suspending agent, a pigment, a flavor, and the like may be used for oral administration; a buffer, a preserving agent, a pain-relieving agent, a solubilizer, an isotonic agent, a stabilizer, and the like may be used in admixture for injections; and a base, an excipient, a lubricant, a preserving agent, and the like may be used for topical administration. The preparations of the pharmaceutical composition of the present invention may be prepared in various ways by being mixed with the pharmaceutically acceptable carrier as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. Alternatively, the pharmaceutical composition may be formulated into solutions, suspensions, tablets, capsules, sustained-release preparations, or the like.

Meanwhile, as examples of carriers, diluents, or excipients suitable for making preparations, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, or the like may be used. In addition, a filler, an anti-coagulant, a lubricant, a wetting agent, a fragrance, an emulsifier, a preservative, and the like may further be included.

The route of administration of the pharmaceutical composition of the present invention includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred.

In the present invention, the "parenteral" includes subcutaneous, intradermal, intravenous, intramuscular, intraarticular, intrabursal, intrasternal, intradural, intralesional, and intracranial injection or infusion techniques. The pharmaceutical composition of the present invention may also be administered in the form of suppositories for rectal administration.

The pharmaceutical composition of the present invention may vary depending on a variety of factors, including activity of a certain compound used, the patient's age, body weight, general health status, sex, diet, time of administration, route of administration, rate of excretion, drug combination, and severity of a certain disease to be prevented or treated. A dose of the pharmaceutical composition may vary depending on the patient's condition, body weight, severity of disease, drug form, route of administration, and duration, and may be appropriately selected by those skilled in the art. The pharmaceutical composition may be administered in an amount of 0.0001 to 50 mg/kg or 0.001 to 50 mg/kg, per day. Administration may be made once a day or several times a day. The dose is not intended to limit the scope of the invention in any case. The pharmaceutical composition according to the present invention may be formulated in the form of pills, sugar-coated tablets, capsules, liquids, gels, syrups, slurries, or suspensions.

According to still yet another embodiment of the present invention, there is provided a method for preventing, ameliorating, or treating an immune-related disease, comprising a step of administering, to an individual, a pharmaceutically effective amount of the binding molecule or antibody-drug conjugate (ADC) provided in the present invention.

The binding molecule, preferably the antibody, provided in the present invention can activate the function, particularly of regulatory T immune cells (Treg cells), among immune cells; increase the number of the Treg cells; and regulate immunological tolerance, thereby effectively preventing, ameliorating, or treating an immune-related disease.

In the present invention, the "individual" is an individual suspected of developing an immune-related disease, and the individual suspected of developing cancer or an immune-related disease means a mammal, such as humans, mice, and domestic animals, who has developed or is likely to develop the disease in question. However, any individual, who is treatable with the binding molecule or antibody-drug conjugate provided in the present invention is included therein without limitation.

The method of the present invention may comprise administering a pharmaceutically effective amount of the binding molecule or antibody-drug conjugate provided in the present invention. An appropriate total daily amount used may be determined by an attending physician or veterinarian within the scope of sound medical judgment, and administration may be made once or several times. However, for the purposes of the present invention, a specific therapeutically effective amount for a particular patient is preferably applied differently depending on various factors, including type and degree of reaction to be achieved, a composition comprising the above-mentioned specific active ingredient, including whether other agents are used therewith as the case may be, the patient's age, body weight, general health status, sex, and diet, time of administration, route of administration, secretion rate of the composition comprising the above-mentioned specific active ingredient, duration of treatment, and drugs used simultaneously or in combination with the specific composition, and similar factors well known in the medical field.

Meanwhile, the method for preventing or treating an immune-related disease may be, but is not limited to, a combination therapy that further comprises administering a compound or substance having therapeutic activity against one or more diseases.

In the present invention, the "combination" should be understood to represent simultaneous, individual, or sequential administration. In a case where the administration is made in a sequential or individual manner, the second component should be administered at intervals such that beneficial effects of the combination are not lost.

In the present invention, the dosage of the binding molecule or antibody-drug conjugate (ADC) may be, but is not limited to, about 0.0001 µg to 500 mg per kg of patient's body weight.

### Advantageous Effects of Invention

The binding molecule, preferably the antibody, specific for the Lrig-1 protein according to the present invention can activate the function, particularly of regulatory T immune cells (Treg cells), among immune cells; increase the number of the Treg cells; and regulate immunological tolerance, thereby effectively preventing, ameliorating, or treating diseases induced by excessive activation and expression of various immune cells and inflammatory cells, for example, immune-related diseases such as autoimmune diseases; graft-versus-host diseases; organ transplant rejection; asthma; atopy; or acute or chronic inflammatory diseases.

In addition, the binding molecule, preferably the antibody, specific for the Lrig-1 protein according to the present invention has advantages of more effectively targeting the Lrig-1 protein as compared with antibodies against Lrig-1 which are previously commercially available, and also possessing very good binding capacity thereto.

### Brief Description of Drawings

FIG. 1 illustrates a structure of the Lrig-1 protein according to an embodiment of the present invention.
FIG. 2 illustrates a structure of the Lrig-1 protein according to an embodiment of the present invention.
FIG. 3 illustrates prediction results for epitopes of the Lrig-1 protein according to an embodiment of the present invention.
FIG. 4 illustrates prediction results for epitopes of the Lrig-1 protein according to an embodiment of the present invention.
FIG. 5 illustrates an expression level of Lrig-1 mRNA according to an embodiment of the present invention.
FIG. 6 illustrates an expression level of Lrig-1 mRNA according to an embodiment of the present invention.
FIG. 7 illustrates an expression level of Lrig-1 mRNA according to an embodiment of the present invention.
FIG. 8 illustrates expression levels of Lrig-1, Lrig-2, and Lrig-3 mRNAs according to an embodiment of the present invention.
FIG. 9 illustrates results obtained by comparing expression levels of Lrig-1 protein in regulatory T cells and non-regulated T cells according to an embodiment of the present invention.
FIG. 10 illustrates expression of the Lrig-1 protein on the surface of regulatory T cells according to an embodiment of the present invention.
FIG. 11 illustrates results obtained by analyzing binding capacity of Lrig-1 protein-specific monoclonal antibodies (A7, C8, E7, and G3) to the Lrig-1 protein according to an embodiment of the present invention.
FIG. 12 illustrates results obtained by analyzing the mechanism of regulating Lrig-1 protein-induced Stat3 phosphorylation, in regulatory T cells, of Lrig-1 protein-specific monoclonal antibodies (A7, C8, E7, and G3) according to an embodiment of the present invention.
FIG. 13 illustrates results obtained by analyzing therapeutic effects, on an autoimmune disease, of Lrig-1 protein-specific monoclonal antibodies (A7, C8, E7, and G3) according to an embodiment of the present invention.

### Detailed Description of Invention

According to an embodiment of the present invention, there is provided a binding molecule selected from the group consisting of the following (1) to (4):
(1) a binding molecule comprising a heavy chain variable region that contains a heavy chain CDR1 represented by SEQ ID NO: 5, a heavy chain CDR2 represented by SEQ ID NO: 6, and a heavy chain CDR3 represented by SEQ ID NO: 7; and a light chain variable region that contains a light chain CDR1 represented by SEQ ID NO: 8, a light chain CDR2 represented by SEQ ID NO: 9, and a light chain CDR3 represented by SEQ ID NO: 10;
(2) a binding molecule comprising a heavy chain variable region that contains a heavy chain CDR1 represented by SEQ ID NO: 13, a heavy chain CDR2 represented by SEQ ID NO: 14, and a heavy chain CDR3 represented by SEQ ID NO: 15; and a light chain variable region that contains a light chain CDR1 represented by SEQ ID NO: 16, a light chain CDR2 represented by SEQ ID NO: 17, and a light chain CDR3 represented by SEQ ID NO: 18;
(3) a binding molecule comprising a heavy chain variable region that contains a heavy chain CDR1 represented by SEQ ID NO: 21, a heavy chain CDR2 represented by SEQ ID NO: 22, and a heavy chain CDR3 represented by SEQ ID NO: 23; and a light chain variable region that contains a light chain CDR1 represented by SEQ ID NO: 24, a light chain CDR2 represented by SEQ ID NO: 25, and a light chain CDR3 represented by SEQ ID NO: 26;
(4) a binding molecule comprising a heavy chain variable region that contains a heavy chain CDR1 represented by SEQ ID NO: 29, a heavy chain CDR2 represented by SEQ ID NO: 30, and a heavy chain CDR3 represented by SEQ ID NO: 31; and a light chain variable region that contains a light chain CDR1 represented by SEQ ID NO: 32, a light chain CDR2 represented by SEQ ID NO: 33, and a light chain CDR3 represented by SEQ ID NO: 34.

Hereinafter, the present invention will be described in more detail by way of examples. These examples are only for describing the present invention in more detail, and it will be apparent to those skilled in the art that according to the gist of the present invention, the scope of the present invention is not limited by these examples.

### Examples

### [Preparation Example 1] T cell subset cell culture

In order to identify whether the Lrig-1 protein is expressed only in regulatory T cells (Treg), the subsets of T cells, Th0, Th1, Th2, Th17, and iTreg, were prepared. The iTreg refers to cells whose differentiation has been artificially induced in a medium containing the following composition, unlike nTreg which has been naturally isolated.

The subsets of the T cells were induced to differentiate into respective cells by first isolating naive T cells obtained from the spleen of mice, causing RPMI1640 (Invitrogen Gibco, Grand Island, NY) nutrient medium that contains 10% fetal bovine serum (FBS; HyClone, Logan, UT) to further contain the respective ingredients of Table 1 below, and performing 72-hour incubation in an incubator at 37°C, 5% CO₂.

**[Table 1]**

| Differentiated cell | Composition |
|---|---|
| Th0 | anti-CD3, anti-CD28 |
| Th1 | IL-12, anti-IL-4 antibody |
| Th2 | IL-4, anti-IFNβ |
| Th17 | IL-6, TGFβ, anti-IFNβ, anti-IL-4 |
| iTreg | IL-2, TGFβ |

### [Example 1] Structural analysis of Lrig-1

A three-dimensional steric structure of the extracellular domain of the Lrig-1 protein was predicted to produce antibodies specific for the Lrig-1 protein, a surface protein of regulatory T cells.

First, in order to predict base sequences of epitopes (epitopes), tools of Uniprot (http://www.uniprot.org) and RCSB Protein Data Bank (http://www.rcsb.org/pdb) were used to predict a three-dimensional steric structure of the extracellular domain (ECD) of the Lrig-1 protein so that the structure of ECD is identified. Then, the results are illustrated in FIGS. 1 and 2.

As illustrated in FIG. 1, a total of 15 leucine-rich regions of LRR1 to LRR15 existed in the Lrig-LRR domain (amino acid sequence at positions 41 to 494) in the extracellular domain of the Lrig-1 protein. Each of the LRR domains is composed of 23 to 27 amino acids, with 3 to 5 leucine being present.

In addition, as illustrated in FIG. 2, three immunoglobulin-like domains exist in amino acid sequences at positions 494 to 781 of the Lrig-1 protein in the extracellular domain of the Lrig-1 protein.

### [Example 2] Prediction of Lrig-1 epitope amino acid sequence

Prediction of the above base sequence was performed using Ellipro server (http://tools.iedb.org/ellipro/) which is an epitope prediction software based on a structure of the Lrig-1 protein. The Ellipro search engine was used because it corresponds to a search engine known to be the most reliable among the existing algorithms for predicting an epitope.

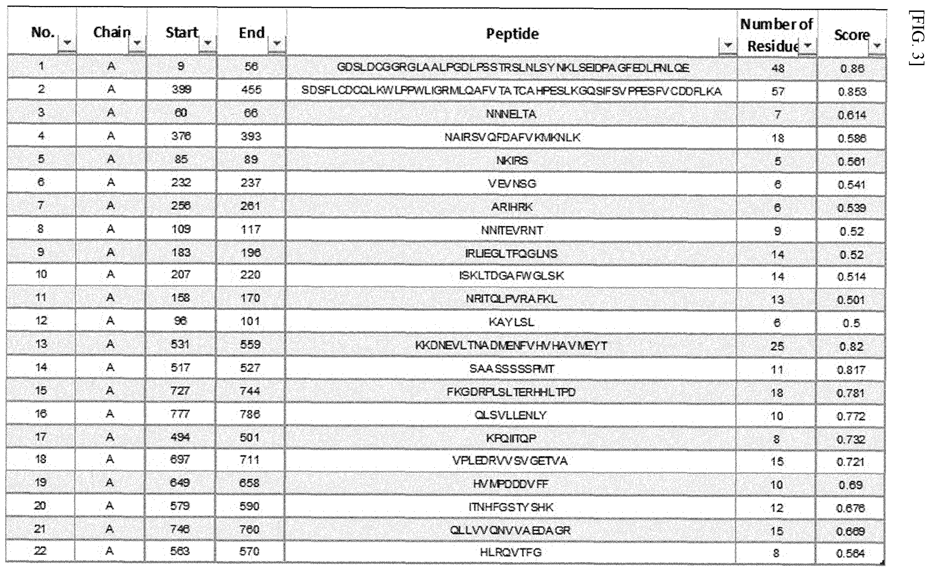

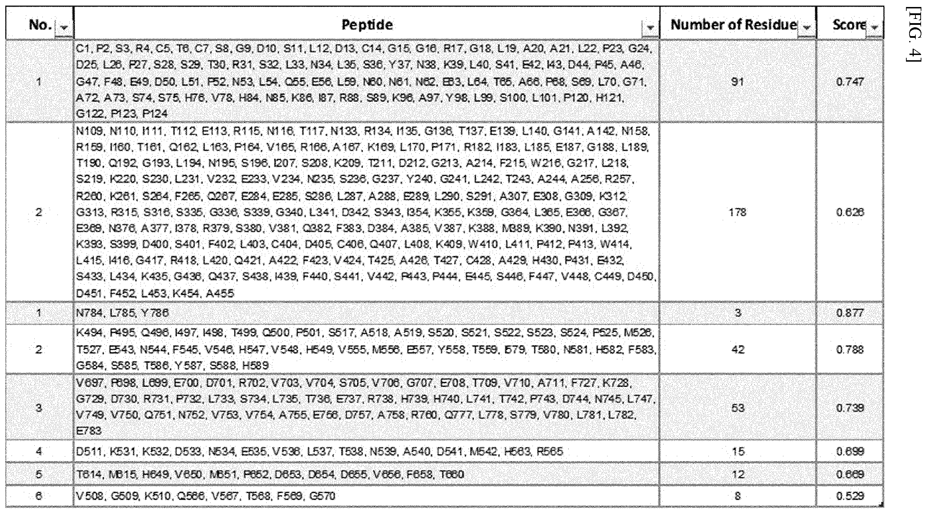

The extracellular domain analyzed in Example 1 was entered into the epitope prediction software, and then predicted contiguous or discontiguous amino acid sequences of the predicted epitopes are illustrated in FIGS. 3 and 4.

As illustrated in FIGS. 3 and 4, a total of 22 contiguous epitope amino acid sequences were predicted, and a total of 8 discontiguous epitope amino acid sequences were predicted.

### [Production Examples 1 to 4] Production of monoclonal antibodies specific to Lrig-1 protein

Antibodies specific for the Lrig-1 protein according to the present invention were produced. The present antibodies were not produced by specifying a certain epitope, but were produced as antibodies capable of binding to any site on the Lrig-1 protein.

In order to produce the antibodies, cells expressing the Lrig-1 protein were produced. More specifically, a DNA fragment corresponding to SEQ ID NO: 2 and pcDNA (hygro) were cleaved with a cleavage enzyme, incubated at 37°C, and ligated to produce pcDNA into which a DNA sequence of the Lrig-1 protein is inserted. The thus produced pcDNA into which SEQ ID NO: 2 is inserted was introduced, through transfection, into L cells, so that the Lrig-1 protein is allowed to be expressed on the surface of the L cells.

Light and heavy chain amino acid sequences capable of binding to Lrig-1 expressed on the cell surface were selected from the Human scFv library so that a total of eight heavy and light chains were selected.

The selected heavy and light chain amino acid sequences were fused with the mlgG2a Fc region, to produce monoclonal antibodies. The sequences of the monoclonal antibodies are shown in Table 2 below.

**[Table 2]**

| Classification | Clone | Location | Amino acid sequence | Sequence information |
|---|---|---|---|---|
| Production Example 1 | A7 clone | Heavy chain | EVQLLESGGGLVQPGGSLRLSCAASGFTFSGYD MSWVRQAPGKGLEWVSLIYPDSGNKYYADSVK GRFTISRDNSKNTLYLQMNSLRAEDTAVYYCAR DAGLSWAGAFDYWGQGTLVTVSSTTAPSVYPL APVCGDTTGSSVTLGCLVKGYFPEPVTLTWNSG SLSSGVHTFPAVLQSDLYTLSSSVTVTSSTWPSQS ITCNVAHPASSTKVDKKIEPRGPTIKPCPPCKCPA PNLLGGPSVFIFPPKIKDVLMISLSPIVTCVVVDVS EDDPDVQISWFVNNVEVHTAQTQTHREDYNSTL RVVSALPIQHQDWMSGKEFKCKVNNKDLPAPIE RTISKPKGSVRAPQVYVLPPPEEEMTKKQVTLTC MVTDFMPEDIYVEWTNNGKTELNYKNTEPVLDS DGSYFMYSKLRVEKKNWVERNSYSCSVVHEGL HNHHTTKSFSRTPGK | SEQ ID NO: 45 |
| | | Light chain | QSVLTQPPSASGTPGQRVTISCSGSSSNIGSNYVT WYQQLPGTAPKLLIYSDSHRPSGVPDRFSGSKSG TSASLAISGLQSEDEADYYCGSWDYSLSAYVFG GGTKLTVLRTVAAPTVSIFPPSSEQLTSGGASVV CFLNNFYPKDINVKWKIDGSERQNGVLNSWTDQ DSKDSTYSMSSTLTLTKDEYERHNSYTCEATHKT | SEQ ID NO: 46 |
| | | | STSPIVKSFNRNEC | |
| Production Example 2 | C8 clone | Heavy chain | EVQLLESGGGLVQPGGSLRLSCAASGFTFSNYY MSWVRQAPGKGLEWVSGISPGDSSTYYADSVK GRFTISRDNSKNTLYLQMNSLRAEDTAVYYCAK GLYSNPNEPFDYWGQGTLVTVSSTTAPSVYPLAP VCGDTTGSSVTLGCLVKGYFPEPVTLTWNSGSLS SGVHTFPAVLQSDLYTLSSSVTVTSSTWPSQSITC NVAHPASSTKVDKKIEPRGPTIKPCPPCKCPAPNL LGGPSVFIFPPKIKDVLMISLSPIVTCVVVDVSED DPDVQISWFVNNVEVHTAQTQTHREDYNSTLRV VSALPIQHQDWMSGKEFKCKVNNKDLPAPIERTI SKPKGSVRAPQVYVLPPPEEEMTKKQVTLTCMV TDFMPEDIYVEWTNNGKTELNYKNTEPVLDSDG SYFMYSKLRVEKKNWVERNSYSCSVVHEGLHN HHTTKSFSRTPGK | SEQ ID NO: 47 |
| | | Light chain | QSVLTQPPSASGTPGQRVTISCTGSSSNIGSNYVS WYQQLPGTAPKLLIYDDSQRPSGVPDRFSGSKSG TSASLAISGLRSEDEADYYCGTWDYSLNGYVFG GGTKLTVLRTVAAPTVSIFPPSSEQLTSGGASVV CFLNNFYPKDINVKWKIDGSERQNGVLNSWTDQ DSKDSTYSMSSTLTLTKDEYERHNSYTCEATHKT STSPIVKSFNRNEC | SEQ ID NO: 48 |
| Production Example 3 | E7 clone | Heavy chain | EVQLLESGGGLVQPGGSLRLSCAASGFTFSSYDM SWVRQAPGKGLEWVSGISPDGSNIYYADSVKGR FTISRDNSKNTLYLQMNSLRAEDTAVYYCAKVG LRCRYEACSYAYGMDVWGQGTLVTVSSTTAPS VYPLAPVCGDTTGSSVTLGCLVKGYFPEPVTLT WNSGSLSSGVHTFPAVLQSDLYTLSSSVTVTSST WPSQSITCNVAHPASSTKVDKKIEPRGPTIKPCPP CKCPAPNLLGGPSVFIFPPKIKDVLMISLSPIVTCV VVDVSEDDPDVQISWFVNNVEVHTAQTQTHRED YNSTLRVVSALPIQHQDWMSGKEFKCKVNNKD LPAPIERTISKPKGSVRAPQVYVLPPPEEEMTKKQ VTLTCMVTDFMPEDIYVEWTNNGKTELNYKNTE PVLDSDGSYFMYSKLRVEKKNWVERNSYSCSV VHEGLHNHHTTKSFSRTPGK | SEQ ID NO: 49 |
| | | Light chain | QSVLTQPPSASGTPGQRVTISCSGSSSNIGSNYVS WYQQLPGTAPKLLIYSDSHRPSGVPDRFSGSKSG TSASLAISGLRSEDEADYYCATWDSSLNGYVFG GGTKLTVLRTVAAPTVSIFPPSSEQLTSGGASVV CFLNNFYPKDINVKWKIDGSERQNGVLNSWTDQ DSKDSTYSMSSTLTLTKDEYERHNSYTCEATHKT STSPIVKSFNRNEC | SEQ ID NO: 50 |
| Production Example 4 | G3 clone | Heavy chain | EVQLLESGGGLVQPGGSLRLSCAASGFTFSNYD MSWVRQAPGKGLEWVSSISPSSGSIYYADSVKG RFTISRDNSKNTLYLQMNSLRAEDTAVYYCAKD LDAFWRPSFDYWGQGTLVTVSSTTAPSVYPLAP VCGDTTGSSVTLGCLVKGYFPEPVTLTWNSGSLS SGVHTFPAVLQSDLYTLSSSVTVTSSTWPSQSITC NVAHPASSTKVDKKIEPRGPTIKPCPPCKCPAPNL LGGPSVFIFPPKIKDVLMISLSPIVTCVVVDVSED DPDVQISWFVNNVEVHTAQTQTHREDYNSTLRV VSALPIQHQDWMSGKEFKCKVNNKDLPAPIERTI SKPKGSVRAPQVYVLPPPEEEMTKKQVTLTCMV TDFMPEDIYVEWTNNGKTELNYKNTEPVLDSDG SYFMYSKLRVEKKNWVERNSYSCSVVHEGLHN | SEQ ID NO: 51 |
| | | | HHTTKSFSRTPGK | |
| | | Light chain | QSVLTQPPSASGTPGQRVTISCTGSSSNIGNNNVN WYQQLPGTAPKLLIYSDSHRPSGVPDRFSGSKSG TSASLAISGLRSEDEADYYCGSWDDSLSAYVFGG GTKLTVLRTVAAPTVSIFPPSSEQLTSGGASVVCF LNNFYPKDINVKWKIDGSERQNGVLNSWTDQDS KDSTYSMSSTLTLTKDEYERHNSYTCEATHKTST SPIVKSFNRNEC | SEQ ID NO: 52 |
| Production Example 5 | A8 clone | Heavy chain | EVQLLESGGGLVQPGGSLRLSCAASGFTFSDYD MSWVRQVPGKGLEWVSWISHGGGSIYYADSVK GRFTISRDNSKNTLYLQMNSLRAEDTAVYYCAR GLGLCKTGLCYYYDAMDVWGQGTLVTVSSTTA PSVYPLAPVCGDTTGSSVTLGCLVKGYFPEPVTL TWNSGSLSSGVHTFPAVLQSDLYTLSSSVTVTSS TWPSQSITCNVAHPASSTKVDKKIEPRGPTIKPCP PCKCPAPNLLGGPSVFIFPPKIKDVLMISLSPIVTC VVVDVSEDDPDVQISWFVNNVEVHTAQTQTHRE DYNSTLRVVSALPIQHQDWMSGKEFKCKVNNK DLPAPIERTISKPKGSVRAPQVYVLPPPEEEMTKK QVTLTCMVTDFMPEDIYVEWTNNGKTELNYKN TEPVLDSDGSYFMYSKLRVEKKNWVERNSYSCS VVHEGLHNHHTTKSFSRTPGK | - |
| | | Light chain | QSVLTQPPSASGTPGQRVTISCTGSSSNIGNNSVT WYQQLPGTAPKLLIYADNNRPSGVPDRFSGSKS GTSASLAISGLRSEDEADYYCAAWDSSLSAYVFG GGTKLTVLRTVAAPTVSIFPPSSEQLTSGGASVV CFLNNFYPKDINVKWKIDGSERQNGVLNSWTDQ DSKDSTYSMSSTLTLTKDEYERHNSYTCEATHKT STSPIVKSFNRNEC | - |
| Production Example 6 | B8 clone | Heavy chain | EVQLLESGGGLVQPGGSLRLSCAASGFTFSDYY MSWVRQAPGKGLEWVSGISHDSGSKYYADSVK GRFTISRDNSKNTLYLQMNSLRAEDTAVYYCAR HWTTFDYWGQGTLVTVSSTTAPSVYPLAPVCGD TTGSSVTLGCLVKGYFPEPVTLTWNSGSLSSGVH TFPAVLQSDLYTLSSSVTVTSSTWPSQSITCNVAH PASSTKVDKKIEPRGPTIKPCPPCKCPAPNLLGGP SVFIFPPKIKDVLMISLSPIVTCVVVDVSEDDPDV QISWFVNNVEVHTAQTQTHREDYNSTLRVVSAL PIQHQDWMSGKEFKCKVNNKDLPAPIERTISKPK GSVRAPQVYVLPPPEEEMTKKQVTLTCMVTDFM PEDIYVEWTNNGKTELNYKNTEPVLDSDGSYFM YSKLRVEKKNWVERNSYSCSVVHEGLHNHHTT KSFSRTPGK | - |
| | | Light chain | QSVLTQPPSASGTPGQRVTISCSGSSSNIGSNNVT WYQQLPGTAPKLLIYANSNRPSGVPDRFSGSKSG TSASLAISGLRSEDEADYYCGAWDYSLSAYVFG GGTKLTVLRTVAAPTVSIFPPSSEQLTSGGASVV CFLNNFYPKDINVKWKIDGSERQNGVLNSWTDQ DSKDSTYSMSSTLTLTKDEYERHNSYTCEATHKT STSPIVKSFNRNEC | - |
| Production Example 7 | D9 clone | Heavy chain | EVQLLESGGGLVQPGGSLRLSCAASGFTFSNYA MSWVRQAPGKGLEWVSAIYPGGGSIYYADSVK GRFTISRDNSKNTLYLQMNSLRAEDTAVYYCAR DILPCPWGRCYYDYAMDVWGQGTLVTVSSTTA PSVYPLAPVCGDTTGSSVTLGCLVKGYFPEPVTL TWNSGSLSSGVHTFPAVLQSDLYTLSSSVTVTSS | - |
| | | | TWPSQSITCNVAHPASSTKVDKKIEPRGPTIKPCP PCKCPAPNLLGGPSVFIFPPKIKDVLMISLSPIVTC VVVDVSEDDPDVQISWFVNNVEVHTAQTQTHRE DYNSTLRVVSALPIQHQDWMSGKEFKCKVNNK DLPAPIERTISKPKGSVRAPQVYVLPPPEEEMTKK QVTLTCMVTDFMPEDIYVEWTNNGKTELNYKN TEPVLDSDGSYFMYSKLRVEKKNWVERNSYSCS VVHEGLHNHHTTKSFSRTPGK | |
| | | Light chain | QSVLTQPPSASGTPGQRVTISCSDSSSNIGSNTVS WYQQLPGTAPKLLIYADNNRPSGVPDRFSGSKS GTSASLAISGLRSEDEADYYCGTWDYSLSGYVF GGGTKLTVLRTVAAPTVSIFPPSSEQLTSGGASV VCFLNNFYPKDINVKWKIDGSERQNGVLNSWTD QDSKDSTYSMSSTLTLTKDEYERHNSYTCEATH KTSTSPIVKSFNRNEC | - |
| Production Example 8 | H6 clone | Heavy chain | EVQLLESGGGLVQPGGSLRLSCAASGFTFSNYA MSWVRQAPGKGLEWVSVISHGGGSTYYADSVK GRFTISRDNSKNTLYLQMNSLRAEDTAVYYCAR VISNCHLGVCYYSNGMDVWGQGTLVTVSSTTAP SVYPLAPVCGDTTGSSVTLGCLVKGYFPEPVTLT WNSGSLSSGVHTFPAVLQSDLYTLSSSVTVTSST WPSQSITCNVAHPASSTKVDKKIEPRGPTIKPCPP CKCPAPNLLGGPSVFIFPPKIKDVLMISLSPIVTCV VVDVSEDDPDVQISWFVNNVEVHTAQTQTHRED YNSTLRVVSALPIQHQDWMSGKEFKCKVNNKD LPAPIERTISKPKGSVRAPQVYVLPPPEEEMTKKQ VTLTCMVTDFMPEDIYVEWTNNGKTELNYKNTE PVLDSDGSYFMYSKLRVEKKNWVERNSYSCSV VHEGLHNHHTTKSFSRTPGK | - |
| | | Light chain | QSVLTQPPSASGTPGQRVTISCSGSSSNIGNNDVY WYQQLPGTAPKLLIYSDSQRPSGVPDRFSGSKSG TSASLAISGLRSEDEADYYCGTWDYSLSGYVFG GGTKLTVLRTVAAPTVSIFPPSSEQLTSGGASVV CFLNNFYPKDINVKWKIDGSERQNGVLNSWTDQ DSKDSTYSMSSTLTLTKDEYERHNSYTCEATHKT STSPIVKSFNRNEC | - |

### [Example 3] Identification of specific expression of Lrig-1 mRNA in regulatory T cells

Verification was made of whether the Lrig-1 protein can act as a biomarker specific for regulatory T cells.

For the verification, CD4⁺ T cells were isolated using magnet-activated cell sorting (MACS), through CD4 beads, from the spleen of rats. Subsequently, regulatory T (CD4⁺CD25⁺ T) cells and non-regulatory T (CD4⁺CD25⁻ T) cells were isolated with a fluorescence-activated cell sorter (FACS) using a CD25 antibody. For the respective cells and the cells differentiated in Preparation Example 1, mRNA was extracted using Trizol, and gDNA was removed from genomic RNA using gDNA extraction kit (Qiagen) according to the protocol provided by the manufacturer. The gDNA-removed mRNA was synthesized into cDNA through the BDsprint cDNA Synthesis Kit (Clonetech).

Real-time polymerase chain reaction (RT PCR) was performed to quantitatively identify an expression level of Lrig-1 mRNA in the cDNA.

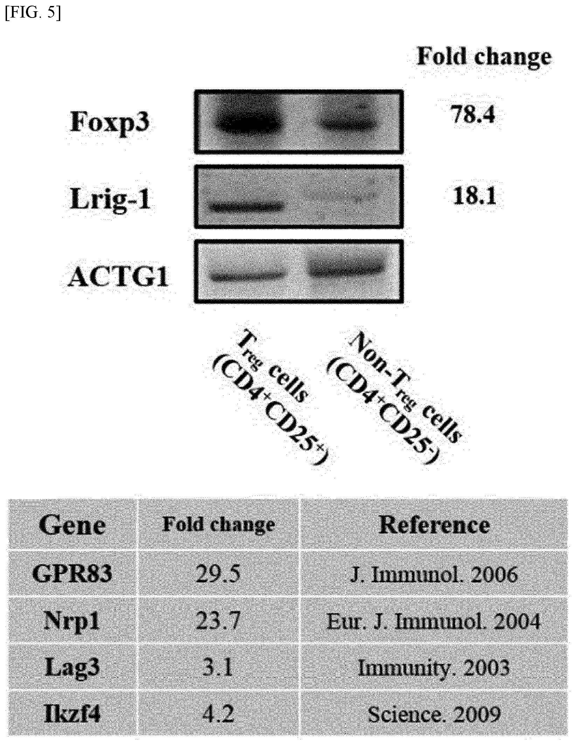

The real-time polymerase chain reaction was performed with primers shown in Table 3 below using SYBR Green (Molecular Probes) by the protocol provided by the manufacturer under conditions of 40 cycles consisting of 95°C for 3 minutes, 61°C for 15 seconds, 72°C for 30 seconds, a relative gene expression level was calculated using the ΔΔCT method, and normalized using HPRT. The results are illustrated in FIGS. 5 to 8.

**[Table 3]**

| Primer | Sequence |
|---|---|
| Mouse Lrig-1 | Forward 5' - GAC GGA ATT CAG TGA GGA GAA CCT - 3' |
| | Reverse 5' - CAA CTG GTA GTG GCA GCT TGT AGG - 3' |
| Mouse Lrig-2 | forward 5' - TCA CAA GGA ACA TTG TCT GAA CCA- 3' |
| | reverse 5' - GCC TGA TCT AAC ACA TCC TCC TCA- 3' |
| Mouse Lrig-3 | forward 5' - CAG CAC CTT GAG CTG AAC AGA AAC - 3' |
| | reverse 5' - CCA GCC TTT GGT AAT CTC GGT TAG - 3' |
| Mouse FOXP3 | forward 5' - CTT TCA CCT ATC CCA CCC TTA TCC - 3' |
| | reverse 5' - ATT CAT CTA CGG TCC ACA CTG CTC - 3' |
| ACTG1 | forward 5' - GGC GTC ATG GTG GGC ATG GG - 3' |
| | reverse 5' - ATG GCG TGG GGA AGG GCG TA - 3' |

As illustrated in FIG. 5, it can be seen that the expression of Lrig-1 in regulatory T (CD4⁺CD25⁺ T) cells is 18.1 times higher than non-regulatory T (CD4⁺CD25⁻ T) cells. This was about 10 times higher expression level than Lag3 and Ikzf4, which are previously known markers for regulatory T cells.

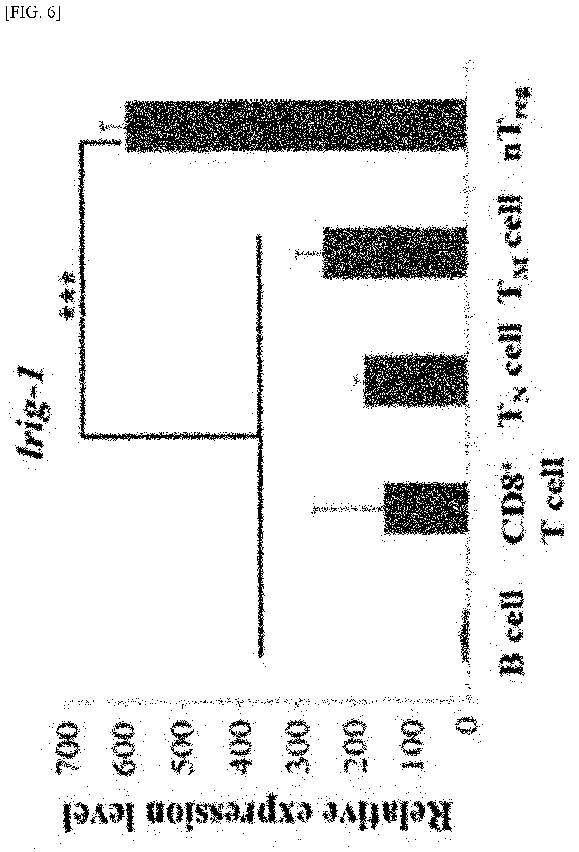

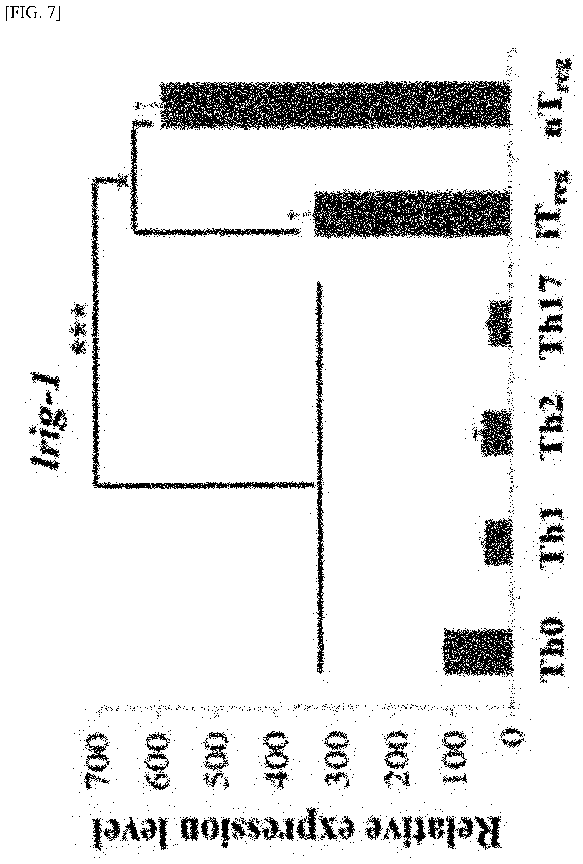

In addition, as illustrated in FIGS. 6 and 7, the expression of Lrig-1 mRNA was remarkably high in regulatory T cells as compared with other types of immune cells, and in particular, was remarkably high in naturally isolated regulatory T cells (nTreg) as compared with induced regulatory T cells (iTreg cells).

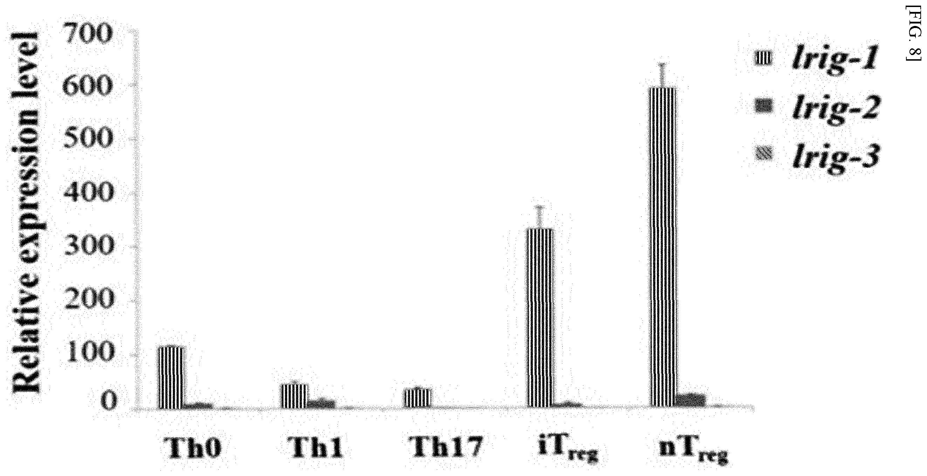

In addition, as illustrated in FIG. 8, expression of Lrig-1 was the highest among Lrig-1, Lrig-2, and Lrig-3 which correspond to the Lrig family.

From the above results, it can be seen that the Lrig-1 protein according to the present invention is specifically expressed in regulatory T cells, in particular, naturally-occurring regulatory T cells.

### [Example 4] Identification of specific expression of Lrig-1 protein in regulatory T cells

It was identified whether the Lrig-1 protein expressed from Lrig-1 mRNA is specifically expressed only in regulatory T cells.

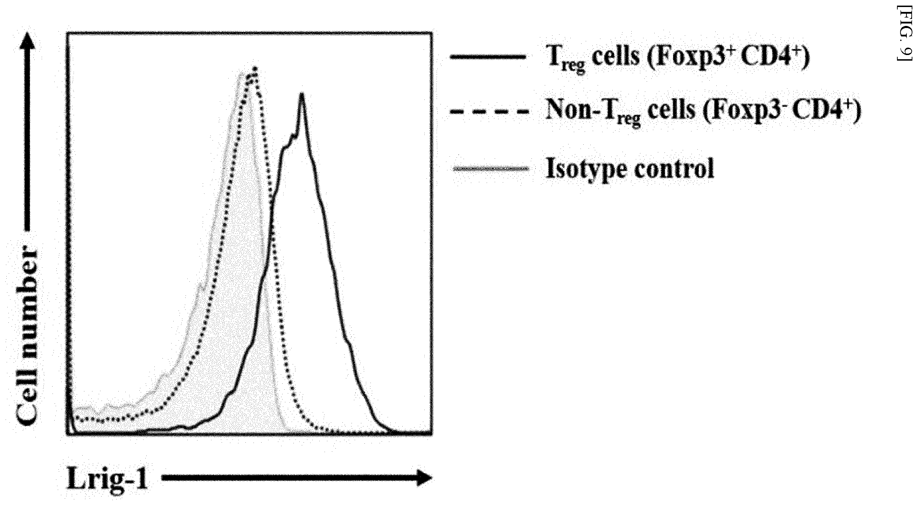

Using FOXP3-RFP-knocked-in mice, the FOXP3-RFP obtained by coupling red fluorescence protein (RFP) to FOXP3 promoter, a transcription factor specific for regulatory T cells, CD4⁺ T cells were isolated using magnet-activated cell sorting (MACS), through CD4 beads, from the spleen of the mice. Subsequently, using RFP protein, regulatory T (CD4⁺RFP⁺ T) cells and non-regulatory T (CD4⁺RFP⁻ T) cells were obtained by performing isolation through a fluorescence-activated cell sorter (FACS). The respective cells were stained with the purchased Lrig-1 antibody and a negative control was stained with an isotype-matched control antibody, to measure an expression level of Lrig-1 with the fluorescence-activated cell sorter. The results are illustrated in FIG. 9.

As illustrated in FIG. 9, the non-regulatory T cells indicated by a dotted line showed almost the same expression level of Lrig-1 as the negative control, whereas there were a large number of cells with high expression level of Lrig-1 in the regulatory T cells.

From the above results, it can be seen that the Lrig-1 protein according to the present invention is specifically expressed in regulatory T cells.

### [Example 5] Identification of specific expression of Lrig-1 protein on surface of regulatory T cells

From the viewpoint that in order to be a target of cell therapy, the Lrig-1 protein must be expressed on the surface of regulatory T cells, which in turn allows a more effective target therapy, it was identified whether the Lrig-1 protein is expressed on the surface of the regulatory T cells.

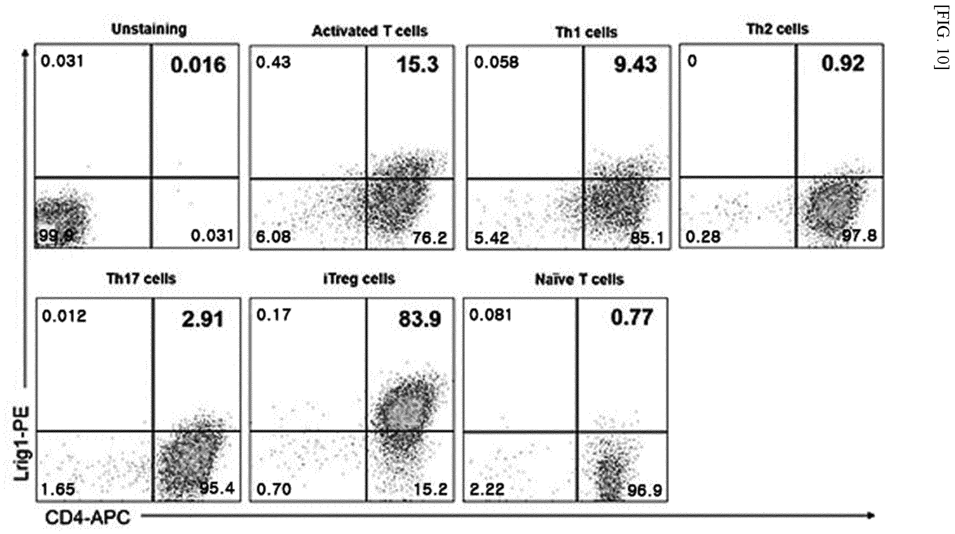

The respective differentiated T cell subsets of Preparation Example 1 were stained with anti-CD4-APC and anti-Lrig-1-PE antibodies, and expression levels of Lrig-1 were measured at the respective cell surfaces using a fluorescence-activated cell sorter (FACS). The results are illustrated in FIG. 10.

As illustrated in FIG. 10, Lrig-1 was expressed in an amount of 0.77 to 15.3 in activated T cells, Th1 cells, Th2 cells, Th17 cells, and naive T cells, whereas Lrig-1 was expressed as high as 83.9 in differentiation-induced T cells (iTreg cells).

From the above results, it can be seen that the Lrig-1 protein according to the present invention is not only specifically expressed in regulatory T (Treg) cells, but also is, in particular, expressed at a higher level on the surface of the Treg cells.

### [Example 6] Evaluation of binding capacity of antibody according to present invention to Lrig-1 protein

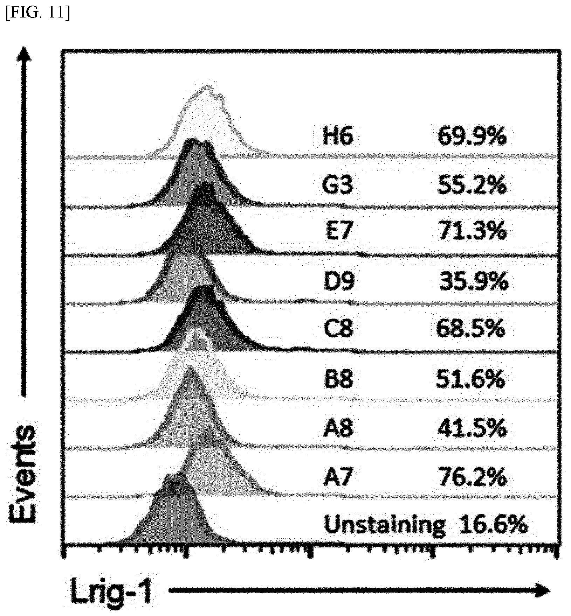

In order to identify whether the monoclonal antibodies according to the present invention produced in Production Examples 1 to 8 well recognize Lrig-1, each of the antibodies of Production Examples 1 to 8 was bound to L cells that stably express Lrig-1. Then, a secondary antibody which is conjugated with eFlour 670 and is capable of recognizing the mouse antibodies was added thereto, and then binding capacity of the monoclonal antibodies to the Lrig-1 protein was analyzed using FACS. The results are illustrated in FIG. 11.

As illustrated in FIG. 11, it was found that all Lrig-1 protein-specific monoclonal antibodies (A7, C8, E7, and G3) according to the present invention effectively recognize and bind to the Lrig-1 protein present on the surface of L cells.

### [Example 7] Regulation of signal transduction pathway in Treg cells, by antibody according to present invention

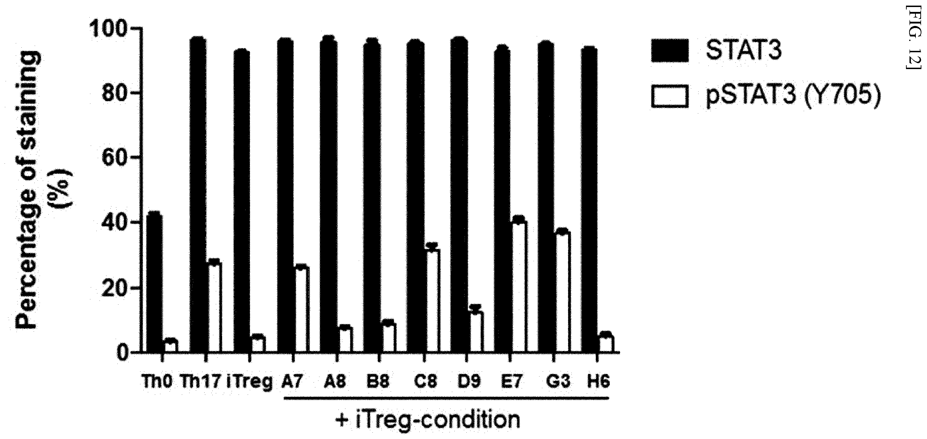

In order to analyze how the monoclonal antibodies according to the present invention produced in Production Examples 1 to 8 affect the signal transduction pathway in Treg cells through the Lrig-1 protein, Lrig-1 present on the surface of the Treg cells was stimulated by treating the Treg cells with the antibodies of Production Examples 1 to 8, and then a level of tyrosine phosphorylation of Stat3 protein present in the stimulated Treg cells was analyzed through phosphotyrosine immunoblot. The results are illustrated in FIG. 12.

As illustrated in FIG. 12, it was found that the Lrig-1 protein-specific monoclonal antibodies (A7, C8, E7, and G3) according to the present invention increase phosphorylation of Stat3 at the same level as Th17 cells.

### [Example 8] Therapeutic effects of antibody according to the present invention on autoimmune disease

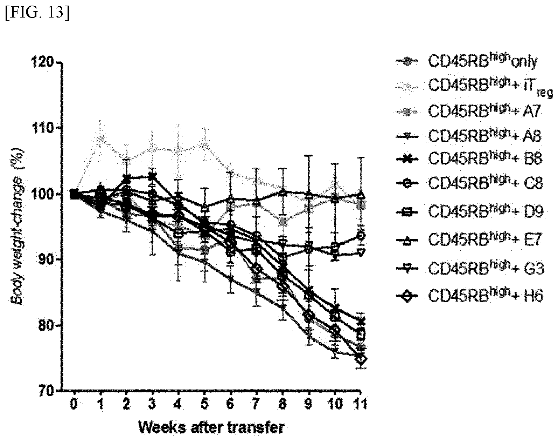

In order to identify therapeutic effects of the monoclonal antibodies (A7, C8, E7, and G3) according to the present invention produced in Production Examples 1 to 4 on an autoimmune disease, RAG-1^{-/-} mice were subjected to adoptive transfer with CD45RB (high) cells so that inflammatory bowel disease (IBD), which is an autoimmune disease, was induced. Then, the antibodies of Production Examples 1 to 4 were intraperitoneally injected in an amount of 200 µg/mouse, and then therapeutic effects thereof on the autoimmune disease were analyzed. The results are illustrated in FIG. 13.

As illustrated in FIG. 13, it was found that the Lrig-1 protein-specific monoclonal antibodies (A7, C8, E7, and G3) according to the present invention remarkably inhibit a body weight-decreasing effect in inflammatory bowel disease-induced mice.

From this, it can be seen that the Lrig-1 protein-specific monoclonal antibody according to the present invention are capable of effectively preventing, ameliorating, or treating immune-related diseases, such as autoimmune diseases, graft-versus-host diseases, organ transplant rejection, asthma, atopy, or acute or chronic inflammatory disease, which are induced by excessive activation and expression of various immune cells and inflammatory cells.

Although the present invention has been described in detail above, the scope of the present invention is not limited thereto. It will be obvious to those skilled in the art that various modifications and changes can be made without departing from the technical spirit of the present invention described in the claims.

### Industrial Applicability

The present invention relates to a binding molecule capable of specifically binding to leucine-rich and immunoglobulin-like domains 1 (Lrig-1) protein, which is a protein present on the surface of regulatory T cells (Treg cells), and a use thereof, specifically, prevention or treatment of immune-related diseases, such as autoimmune diseases, graft-versus-host diseases, organ transplant rejection, asthma, atopy, or acute or chronic inflammatory diseases.

## Claims

1. A binding molecule which specifically binds to Lrig-1 (leucine-rich and immunoglobulin-like domains 1) protein, the binding molecule comprising:
a heavy chain variable region that contains a heavy chain CDR1 consisting of an amino acid sequence selected from the group consisting of SEQ ID NOs: 5, 13, 21, and 29; a heavy chain CDR2 consisting of an amino acid sequence selected from the group consisting of SEQ ID NOs: 6, 14, 22, and 30; a heavy chain CDR3 consisting of an amino acid sequence selected from the group consisting of SEQ ID NOs: 7, 15, 23, and 31; and
a light chain variable region that contains a light chain CDR1 consisting of an amino acid sequence selected from the group consisting of SEQ ID NOs: 8, 16, 24, and 32; a light chain CDR2 consisting of an amino acid sequence selected from the group consisting of SEQ ID NOs: 9, 17, 25, and 33; a light chain CDR3 consisting of an amino acid sequence selected from the group consisting of SEQ ID NO: 10, 18, 26, and 34.

2. The binding molecule according to claim 1,
wherein the Lrig-1 protein consists of an amino acid sequence represented by SEQ ID NO: 1 or 3.

3. The binding molecule according to claim 1,
wherein the Lrig-1 protein is encoded by a polynucleotide represented by SEQ ID NO: 2 or 4.

4. The binding molecule according to claim 1,
wherein the binding molecule comprises:
a heavy chain variable region, selected from the group consisting of the following (a) to (d):
(a) a heavy chain variable region that contains a heavy chain CDR1 represented by SEQ ID NO: 5, a heavy chain CDR2 represented by SEQ ID NO: 6, and a heavy chain CDR3 represented by SEQ ID NO: 7;
(b) a heavy chain variable region that contains a heavy chain CDR1 represented by SEQ ID NO: 13, a heavy chain CDR2 represented by SEQ ID NO: 14, and a heavy chain CDR3 represented by SEQ ID NO: 15;
(c) a heavy chain variable region that contains a heavy chain CDR1 represented by SEQ ID NO: 21, a heavy chain CDR2 represented by SEQ ID NO: 22, and a heavy chain CDR3 represented by SEQ ID NO: 23; and
(d) a heavy chain variable region that contains a heavy chain CDR1 represented by SEQ ID NO: 29, a heavy chain CDR2 represented by SEQ ID NO: 30, and a heavy chain CDR3 represented by SEQ ID NO: 31; and
a light chain variable region, selected from the group consisting of the following (e) to (h):
(e) a light chain variable region that contains a light chain CDR1 represented by SEQ ID NO: 8, a light chain CDR2 represented by SEQ ID NO: 9, and a light chain CDR3 represented by SEQ ID NO: 10;
(f) a light chain variable region that contains a light chain CDR1 represented by SEQ ID NO: 16, a light chain CDR2 represented by SEQ ID NO: 17, and a light chain CDR3 represented by SEQ ID NO: 18;
(g) a light chain variable region that contains a light chain CDR1 represented by SEQ ID NO: 24, a light chain CDR2 represented by SEQ ID NO: 25, and a light chain CDR3 represented by SEQ ID NO: 26;
(h) a light chain variable region that contains a light chain CDR1 represented by SEQ ID NO: 32, a light chain CDR2 represented by SEQ ID NO: 33, and a light chain CDR3 represented by SEQ ID NO: 34.

5. The binding molecule according to claim 1,
wherein the binding molecule is selected from the group consisting of the following (1) to (4):
(1) a binding molecule comprising a heavy chain variable region that contains a heavy chain CDR1 represented by SEQ ID NO: 5, a heavy chain CDR2 represented by SEQ ID NO: 6, and a heavy chain CDR3 represented by SEQ ID NO: 7; and a light chain variable region that contains a light chain CDR1 represented by SEQ ID NO: 8, a light chain CDR2 represented by SEQ ID NO: 9, and a light chain CDR3 represented by SEQ ID NO: 10;
(2) a binding molecule comprising a heavy chain variable region that contains a heavy chain CDR1 represented by SEQ ID NO: 13, a heavy chain CDR2 represented by SEQ ID NO: 14, and a heavy chain CDR3 represented by SEQ ID NO: 15; and a light chain variable region that contains a light chain CDR1 represented by SEQ ID NO: 16, a light chain CDR2 represented by SEQ ID NO: 17, and a light chain CDR3 represented by SEQ ID NO: 18;
(3) a binding molecule comprising a heavy chain variable region that contains a heavy chain CDR1 represented by SEQ ID NO: 21, a heavy chain CDR2 represented by SEQ ID NO: 22, and a heavy chain CDR3 represented by SEQ ID NO: 23; and a light chain variable region that contains a light chain CDR1 represented by SEQ ID NO: 24, a light chain CDR2 represented by SEQ ID NO: 25, and a light chain CDR3 represented by SEQ ID NO: 26;
(4) a binding molecule comprising a heavy chain variable region that contains a heavy chain CDR1 represented by SEQ ID NO: 29, a heavy chain CDR2 represented by SEQ ID NO: 30, and a heavy chain CDR3 represented by SEQ ID NO: 31; and a light chain variable region that contains a light chain CDR1 represented by SEQ ID NO: 32, a light chain CDR2 represented by SEQ ID NO: 33, and a light chain CDR3 represented by SEQ ID NO: 34.

6. The binding molecule according to claim 1,
wherein the binding molecule comprises:
a heavy chain variable region consisting of any one amino acid sequence selected from the group consisting of SEQ ID NOs: 11, 19, 27, and 35; and
a light chain variable region consisting of any one amino acid sequence selected from the group consisting of SEQ ID NO: 12, 20, 28, and 36.

7. The binding molecule according to claim 1,
wherein the binding molecule is selected from the group consisting of the following binding molecules:
(i) a binding molecule comprising a heavy chain variable region represented by SEQ ID NO: 11, and a light chain variable region represented by SEQ ID NO: 12;
(ii) a binding molecule comprising a heavy chain variable region represented by SEQ ID NO: 19, and a light chain variable region represented by SEQ ID NO: 20;
(iii) a binding molecule comprising a heavy chain variable region represented by SEQ ID NO: 27, and a light chain variable region represented by SEQ ID NO: 28; and
(iv) a binding molecule comprising a heavy chain variable region represented by SEQ ID NO: 35, and a light chain variable region represented by SEQ ID NO: 36.

8. The binding molecule according to claim 1, further comprising:
an Fc region or a constant region.

9. The binding molecule according to claim 9,
wherein the Fc region is an Fc region of an IgG1, IgG2, IgG3, or IgG4 antibody, or a hybrid Fc region.

10. The binding molecule according to claim 1, further comprising:
a heavy chain constant region consisting of an amino acid sequence selected from the group consisting of SEQ ID NOs: 37, 39, 41, 42, 43, 44, and 53.

11. The binding molecule according to claim 1, further comprising:
a light chain constant region consisting of an amino acid sequence represented by SEQ ID NO: 38 or 40.

12. The binding molecule according to claim 1, further comprising:
a heavy chain constant region consisting of an amino acid sequence represented by SEQ ID NO: 37; and
a light chain constant region consisting of an amino acid sequence represented by SEQ ID NO: 38.

13. The binding molecule according to claim 1, further comprising:
a heavy chain constant region consisting of an amino acid sequence selected from the group consisting of SEQ ID NO: 39, 41, 42, 43, and 53; and
a light chain constant region consisting of an amino acid sequence represented by SEQ ID NO: 40.

14. The binding molecule according to claim 1, further comprising:
a heavy chain constant region consisting of an amino acid sequence represented by SEQ ID NO: 44.

15. The binding molecule according to claim 1,
wherein the binding molecule is selected from the group consisting of the following binding molecules:
a binding molecule comprising a heavy chain represented by SEQ ID NO: 45, and a light chain represented by SEQ ID NO: 46;
a binding molecule comprising a heavy chain represented by SEQ ID NO: 47, and a light chain represented by SEQ ID NO: 48;
a binding molecule comprising a heavy chain represented by SEQ ID NO: 49, and a light chain represented by SEQ ID NO: 50; and
a binding molecule comprising a heavy chain represented by SEQ ID NO: 51, and a light chain represented by SEQ ID NO: 52.

16. The binding molecule according to claim 1,
wherein the binding molecule is an antibody or a fragment thereof.

17. The binding molecule according to claim 16,
wherein the antibody is a chimeric antibody, a humanized antibody, a bivalent, bispecific molecule, a minibody, a domain antibody, a bispecific antibody, an antibody mimetic, a unibody, a diabody, a triabody, or a tetrabody, or a fragment thereof.

18. A nucleic acid molecule which encodes the binding molecule according to any one of claims 1 to 17.

19. An expression vector into which the nucleic acid molecule of claim 18 is inserted.

20. A host cell line, transfected with the expression vector according to claim 19.

21. An antibody-drug conjugate, comprising:
an antibody; and
a drug,
wherein the antibody comprises:
a heavy chain variable region that contains a heavy chain CDR1 consisting of an amino acid sequence selected from the group consisting of SEQ ID NOs: 5, 13, 21, and 29; a heavy chain CDR2 consisting of an amino acid sequence selected from the group consisting of SEQ ID NOs: 6, 14, 22, and 30; a heavy chain CDR3 consisting of an amino acid sequence selected from the group consisting of SEQ ID NOs: 7, 15, 23, and 31; and
a light chain variable region that contains a light chain CDR1 consisting of an amino acid sequence selected from the group consisting of SEQ ID NOs: 8, 16, 24, and 32; a light chain CDR2 consisting of an amino acid sequence selected from the group consisting of SEQ ID NOs: 9, 17, 25, and 33; a light chain CDR3 consisting of an amino acid sequence selected from the group consisting of SEQ ID NO: 10, 18, 26, and 34.

22. A pharmaceutical composition for preventing or treating an immune-related disease, comprising as an active ingredient:
the binding molecule according to any one of claims 1 to 17.

23. The pharmaceutical composition according to claim 22,
wherein the immune-related disease is an autoimmune disease, a graft versus host disease, organ transplant rejection, asthma, atopy, or an acute or chronic inflammatory disease.

24. A method for preventing or treating an immune-related disease, comprising:
a step of administering, to an individual, a pharmaceutically effective amount of the binding molecule according to any one of claims 1 to 17.

25. A method for preventing or treating an immune-related disease, comprising:
a step of administering, to an individual, a pharmaceutically effective amount of the antibody-drug conjugate according to claim 21.


Sequence List Free Text
<110>	Good T Cells, Inc.
<120>	BINDING MOLECULE SPECIFIC TO LRIG-1 PROTEIN AND USE THEREOF
<130>	POPB194162PCT
<150>	KR 10-2018-0123858
<151>	2018-10-17
<160>	53
<170>	KoPatentIn 3.0
<210>	1
<211>	759
<212>	PRT
<213>	Homo sapiens
<400>	1
Gly Pro Arg Ala Pro Cys Ala Ala Ala Cys Thr Cys Ala Gly Asp Ser
1               5                   10                  15
Leu Asp Cys Gly Gly Arg Gly Leu Ala Ala Leu Pro Gly Asp Leu Pro
            20                  25                  30
Ser Trp Thr Arg Ser Leu Asn Leu Ser Tyr Asn Lys Leu Ser Glu Ile
        35                  40                  45
Asp Pro Ala Gly Phe Glu Asp Leu Pro Asn Leu Gln Glu Val Tyr Leu
    50                  55                  60
Asn Asn Asn Glu Leu Thr Ala Val Pro Ser Leu Gly Ala Ala Ser Ser
65                  70                  75                  80

His Val Val Ser Leu Phe Leu Gln His Asn Lys Ile Arg Ser Val Glu
                85                  90                  95
Gly Ser Gln Leu Lys Ala Tyr Leu Ser Leu Glu Val Leu Asp Leu Ser
            100                 105                 110
Leu Asn Asn Ile Thr Glu Val Arg Asn Thr Cys Phe Pro His Gly Pro
        115                 120                 125
Pro Ile Lys Glu Leu Asn Leu Ala Gly Asn Arg Ile Gly Thr Leu Glu
    130                 135                 140

Leu Gly Ala Phe Asp Gly Leu Ser Arg Ser Leu Leu Thr Leu Arg Leu
145                 150                 155                 160
Ser Lys Asn Arg Ile Thr Gln Leu Pro Val Arg Ala Phe Lys Leu Pro
                165                 170                 175
Arg Leu Thr Gln Leu Asp Leu Asn Arg Asn Arg Ile Arg Leu Ile Glu
            180                 185                 190
Gly Leu Thr Phe Gln Gly Leu Asn Ser Leu Glu Val Leu Lys Leu Gln
        195                 200                 205
Arg Asn Asn Ile Ser Lys Leu Thr Asp Gly Ala Phe Trp Gly Leu Ser
    210                 215                 220
Lys Met His Val Leu His Leu Glu Tyr Asn Ser Leu Val Glu Val Asn
225                 230                 235                 240
Ser Gly Ser Leu Tyr Gly Leu Thr Ala Leu His Gln Leu His Leu Ser
                245                 250                 255
Asn Asn Ser Ile Ala Arg Ile His Arg Lys Gly Trp Ser Phe Cys Gln
            260                 265                 270
Lys Leu His Glu Leu Val Leu Ser Phe Asn Asn Leu Thr Arg Leu Asp
        275                 280                 285
Glu Glu Ser Leu Ala Glu Leu Ser Ser Leu Ser Val Leu Arg Leu Ser
    290                 295                 300
His Asn Ser Ile Ser His Ile Ala Glu Gly Ala Phe Lys Gly Leu Arg
305                 310                 315                 320
Ser Leu Arg Val Leu Asp Leu Asp His Asn Glu Ile Ser Gly Thr Ile
                325                 330                 335
Glu Asp Thr Ser Gly Ala Phe Ser Gly Leu Asp Ser Leu Ser Lys Leu
            340                 345                 350
Thr Leu Phe Gly Asn Lys Ile Lys Ser Val Ala Lys Arg Ala Phe Ser
        355                 360                 365
Gly Leu Glu Gly Leu Glu His Leu Asn Leu Gly Gly Asn Ala Ile Arg
    370                 375                 380
Ser Val Gln Phe Asp Ala Phe Val Lys Met Lys Asn Leu Lys Glu Leu
385                 390                 395                 400

His Ile Ser Ser Asp Ser Phe Leu Cys Asp Cys Gln Leu Lys Trp Leu
                405                 410                 415
Pro Pro Trp Leu Ile Gly Arg Met Leu Gln Ala Phe Val Thr Ala Thr
            420                 425                 430
Cys Ala His Pro Glu Ser Leu Lys Gly Gln Ser Ile Phe Ser Val Pro
        435                 440                 445
Pro Glu Ser Phe Val Cys Asp Asp Phe Leu Lys Pro Gln Ile Ile Thr
    450                 455                 460
Gln Pro Glu Thr Thr Met Ala Met Val Gly Lys Asp Ile Arg Phe Thr
465                 470                 475                 480
Cys Ser Ala Ala Ser Ser Ser Ser Ser Pro Met Thr Phe Ala Trp Lys
                485                 490                 495
Lys Asp Asn Glu Val Leu Thr Asn Ala Asp Met Glu Asn Phe Val His
            500                 505                 510
Val His Ala Gln Asp Gly Glu Val Met Glu Tyr Thr Thr Ile Leu His
        515                 520                 525
Leu Arg Gln Val Thr Phe Gly His Glu Gly Arg Tyr Gln Cys Val Ile
    530                 535                 540
Thr Asn His Phe Gly Ser Thr Tyr Ser His Lys Ala Arg Leu Thr Val
545                 550                 555                 560
Asn Val Leu Pro Ser Phe Thr Lys Thr Pro His Asp Ile Thr Ile Arg
                565                 570                 575
Thr Thr Thr Val Ala Arg Leu Glu Cys Ala Ala Thr Gly His Pro Asn
            580                 585                 590
Pro Gln Ile Ala Trp Gln Lys Asp Gly Gly Thr Asp Phe Pro Ala Ala
        595                 600                 605
Arg Glu Arg Arg Met His Val Met Pro Asp Asp Asp Val Phe Phe Ile
    610                 615                 620
Thr Asp Val Lys Ile Asp Asp Ala Gly Val Tyr Ser Cys Thr Ala Gln
625                 630                 635                 640
Asn Ser Ala Gly Ser Ile Ser Ala Asn Ala Thr Leu Thr Val Leu Glu
                645                 650                 655

Thr Pro Ser Leu Val Val Pro Leu Glu Asp Arg Val Val Ser Val Gly
            660                 665                 670
Glu Thr Val Ala Leu Gln Cys Lys Ala Thr Gly Asn Pro Pro Pro Arg
        675                 680                 685
Ile Thr Trp Phe Lys Gly Asp Arg Pro Leu Ser Leu Thr Glu Arg His
    690                 695                 700
His Leu Thr Pro Asp Asn Gln Leu Leu Val Val Gln Asn Val Val Ala
705                 710                 715                 720
Glu Asp Ala Gly Arg Tyr Thr Cys Glu Met Ser Asn Thr Leu Gly Thr
                725                 730                 735
Glu Arg Ala His Ser Gln Leu Ser Val Leu Pro Ala Ala Gly Cys Arg
            740                 745                 750
Lys Asp Gly Thr Thr Val Gly
        755

<210> 2
<211> 2397
<212> DNA
<213> Homo sapiens
<400> 2
ggcccgcggg cgccctgcgc ggccgcctgc acttgcgctg gggactcgct ggactgcggt 60
gggcgcgggc tggctgcgtt gcccggggac ctgccctcct ggacgcggag cctaaacctg 120
agttacaaca aactctctga gattgaccct gctggttttg aggacttgcc gaacctacag 180
gaagtgtacc tcaataataa tgagttgaca gcggtaccat ccctgggcgc tgcttcatca 240
catgtcgtct ctctctttct gcagcacaac aagattcgca gcgtggaggg gagccagctg 300
aaggcctacc tttccttaga agtgttagat ctgagtttga acaacatcac ggaagtgcgg 360
aacacctgct ttccacacgg accgcctata aaggagctca acctggcagg caatcggatt 420
ggcaccctgg agttgggagc atttgatggt ctgtcacggt cgctgctaac tcttcgcctg 480
agcaaaaaca ggatcaccca gcttcctgta agagcattca agctacccag gctgacacaa 540
ctggacctca atcggaacag gattcggctg atagagggcc tcaccttcca ggggctcaac 600
agcttggagg tgctgaagct tcagcgaaac aacatcagca aactgacaga tggggccttc 660
tggggactgt ccaagatgca tgtgctgcac ctggagtaca acagcctggt agaagtgaac 720

agcggctcgc tctacggcct cacggccctg catcagctcc acctcagcaa caattccatc 780
gctcgcattc accgcaaggg ctggagcttc tgccagaagc tgcatgagtt ggtcctgtcc 840
ttcaacaacc tgacacggct ggacgaggag agcctggccg agctgagcag cctgagtgtc 900
ctgcgtctca gccacaattc catcagccac attgcggagg gtgccttcaa gggactcagg 960
agcctgcgag tcttggatct ggaccataac gagatttcgg gcacaataga ggacacgagc 1020
ggcgccttct cagggctcga cagcctcagc aagctgactc tgtttggaaa caagatcaag 1080
tctgtggcta agagagcatt ctcggggctg gaaggcctgg agcacctgaa ccttggaggg 1140
aatgcgatca gatctgtcca gtttgatgcc tttgtgaaga tgaagaatct taaagagctc 1200
catatcagca gcgacagctt cctgtgtgac tgccagctga agtggctgcc cccgtggcta 1260
attggcagga tgctgcaggc ctttgtgaca gccacctgtg cccacccaga atcactgaag 1320
ggtcagagca ttttctctgt gccaccagag agtttcgtgt gcgatgactt cctgaagcca 1380
cagatcatca cccagccaga aaccaccatg gctatggtgg gcaaggacat ccggtttaca 1440
tgctcagcag ccagcagcag cagctccccc atgacctttg cctggaagaa agacaatgaa 1500
gtcctgacca atgcagacat ggagaacttt gtccacgtcc acgcgcagga cggggaagtg 1560
atggagtaca ccaccatcct gcacctccgt caggtcactt tcgggcacga gggccgctac 1620
caatgtgtca tcaccaacca ctttggctcc acctattcac ataaggccag gctcaccgtg 1680
aatgtgttgc catcattcac caaaacgccc cacgacataa ccatccggac caccaccgtg 1740
gcccgcctcg aatgtgctgc cacaggtcac ccaaaccctc agattgcctg gcagaaggat 1800
ggaggcacgg atttccccgc tgcccgtgag cgacgcatgc atgtcatgcc ggatgacgac 1860
gtgtttttca tcactgatgt gaaaatagat gacgcagggg tttacagctg tactgctcag 1920
aactcagccg gttctatttc agctaatgcc accctgactg tcctagagac cccatccttg 1980
gtggtcccct tggaagaccg tgtggtatct gtgggagaaa cagtggccct ccaatgcaaa 2040
gccacgggga accctccgcc ccgcatcacc tggttcaagg gggaccgccc gctgagcctc 2100
actgagcggc accacctgac ccctgacaac cagctcctgg tggttcagaa cgtggtggca 2160
gaggatgcgg gccgatatac ctgtgagatg tccaacaccc tgggcacgga gcgagctcac 2220
agccagctga gcgtcctgcc cgcagcaggc tgcaggaagg atgggaccac ggtaggcatc 2280
ttcaccattg ctgtcgtgag cagcatcgtc ctgacgtcac tggtctgggt gtgcatcatc 2340
taccagacca ggaagaagag tgaagagtac agtgtcacca acacagatga aaccgtc 2397

<210> 3
<211> 761
<212> PRT

<213>        Mus musculus

<400>        3

Gln Ala Gly Pro Arg Ala Pro Cys Ala Ala Ala Cys Thr Cys Ala Gly
1               5                   10                  15

Asp Ser Leu Asp Cys Ser Gly Arg Gly Leu Ala Thr Leu Pro Arg Asp
            20                  25                  30

Leu Pro Ser Trp Thr Arg Ser Leu Asn Leu Ser Tyr Asn Arg Leu Ser
        35                  40                  45

Glu Ile Asp Ser Ala Ala Phe Glu Asp Leu Thr Asn Leu Gln Glu Val
    50                  55                  60

Tyr Leu Asn Ser Asn Glu Leu Thr Ala Ile Pro Ser Leu Gly Ala Ala
65                  70                  75                  80

Ser Ile Gly Val Val Ser Leu Phe Leu Gln His Asn Lys Ile Leu Ser
                85                  90                  95

Val Asp Gly Ser Gln Leu Lys Ser Tyr Leu Ser Leu Glu Val Leu Asp
            100                 105                 110

Leu Ser Ser Asn Asn Ile Thr Glu Ile Arg Ser Ser Cys Phe Pro Asn
        115                 120                 125

Gly Leu Arg Ile Arg Glu Leu Asn Leu Ala Ser Asn Arg Ile Ser Ile
    130                 135                 140

Leu Glu Ser Gly Ala Phe Asp Gly Leu Ser Arg Ser Leu Leu Thr Leu
145                 150                 155                 160

Arg Leu Ser Lys Asn Arg Ile Thr Gln Leu Pro Val Lys Ala Phe Lys
                165                 170                 175

Leu Pro Arg Leu Thr Gln Leu Asp Leu Asn Arg Asn Arg Ile Arg Leu
            180                 185                 190

Ile Glu Gly Leu Thr Phe Gln Gly Leu Asp Ser Leu Glu Val Leu Arg
        195                 200                 205

Leu Gln Arg Asn Asn Ile Ser Arg Leu Thr Asp Gly Ala Phe Trp Gly
    210                 215                 220

Leu Ser Lys Met His Val Leu His Leu Glu Tyr Asn Ser Leu Val Glu
225                 230                 235                 240

Val Asn Ser Gly Ser Leu Tyr Gly Leu Thr Ala Leu His Gln Leu His
                245                 250                 255
Leu Ser Asn Asn Ser Ile Ser Arg Ile Gln Arg Asp Gly Trp Ser Phe
            260                 265                 270
Cys Gln Lys Leu His Glu Leu Ile Leu Ser Phe Asn Asn Leu Thr Arg
        275                 280                 285
Leu Asp Glu Glu Ser Leu Ala Glu Leu Ser Ser Leu Ser Ile Leu Arg
    290                 295                 300
Leu Ser His Asn Ala Ile Ser His Ile Ala Glu Gly Ala Phe Lys Gly
305                 310                 315                 320
Leu Lys Ser Leu Arg Val Leu Asp Leu Asp His Asn Glu Ile Ser Gly
                325                 330                 335
Thr Ile Glu Asp Thr Ser Gly Ala Phe Thr Gly Leu Asp Asn Leu Ser
            340                 345                 350
Lys Leu Thr Leu Phe Gly Asn Lys Ile Lys Ser Val Ala Lys Arg Ala
        355                 360                 365
Phe Ser Gly Leu Glu Ser Leu Glu His Leu Asn Leu Gly Glu Asn Ala
    370                 375                 380
Ile Arg Ser Val Gln Phe Asp Ala Phe Ala Lys Met Lys Asn Leu Lys
385                 390                 395                 400
Glu Leu Tyr Ile Ser Ser Glu Ser Phe Leu Cys Asp Cys Gln Leu Lys
                405                 410                 415
Trp Leu Pro Pro Trp Leu Met Gly Arg Met Leu Gln Ala Phe Val Thr
            420                 425                 430
Ala Thr Cys Ala His Pro Glu Ser Leu Lys Gly Gln Ser Ile Phe Ser
        435                 440                 445
Val Leu Pro Asp Ser Phe Val Cys Asp Asp Phe Pro Lys Pro Gln Ile
    450                 455                 460
Ile Thr Gln Pro Glu Thr Thr Met Ala Val Val Gly Lys Asp Ile Arg
465                 470                 475                 480
Phe Thr Cys Ser Ala Ala Ser Ser Ser Ser Ser Pro Met Thr Phe Ala
                485                 490                 495

Trp Lys Lys Asp Asn Glu Val Leu Ala Asn Ala Asp Met Glu Asn Phe
            500                 505                 510
Ala His Val Arg Ala Gln Asp Gly Glu Val Met Glu Tyr Thr Thr Ile
        515                 520                 525
Leu His Leu Arg His Val Thr Phe Gly His Glu Gly Arg Tyr Gln Cys
    530                 535                 540
Ile Ile Thr Asn His Phe Gly Ser Thr Tyr Ser His Lys Ala Arg Leu
545                 550                 555                 560
Thr Val Asn Val Leu Pro Ser Phe Thr Lys Ile Pro His Asp Ile Ala
                565                 570                 575
Ile Arg Thr Gly Thr Thr Ala Arg Leu Glu Cys Ala Ala Thr Gly His
            580                 585                 590
Pro Asn Pro Gln Ile Ala Trp Gln Lys Asp Gly Gly Thr Asp Phe Pro
        595                 600                 605
Ala Ala Arg Glu Arg Arg Met His Val Met Pro Asp Asp Asp Val Phe
    610                 615                 620
Phe Ile Thr Asp Val Lys Ile Asp Asp Met Gly Val Tyr Ser Cys Thr
625                 630                 635                 640
Ala Gln Asn Ser Ala Gly Ser Val Ser Ala Asn Ala Thr Leu Thr Val
                645                 650                 655
Leu Glu Thr Pro Ser Leu Ala Val Pro Leu Glu Asp Arg Val Val Thr
            660                 665                 670
Val Gly Glu Thr Val Ala Phe Gln Cys Lys Ala Thr Gly Ser Pro Thr
        675                 680                 685
Pro Arg Ile Thr Trp Leu Lys Gly Gly Arg Pro Leu Ser Leu Thr Glu
    690                 695                 700
Arg His His Phe Thr Pro Gly Asn Gln Leu Leu Val Val Gln Asn Val
705                 710                 715                 720
Met Ile Asp Asp Ala Gly Arg Tyr Thr Cys Glu Met Ser Asn Pro Leu
                725                 730                 735
Gly Thr Glu Arg Ala His Ser Gln Leu Ser Ile Leu Pro Thr Pro Gly
            740                 745                 750

Cys Arg Lys Asp Gly Thr Thr Val Gly
        755                 760

<210> 4
<211> 2283
<212> DNA
<213> Mus musculus

<400> 4
caggctggcc cgcgggcccc ctgcgcggcc gcctgcactt gcgccgggga ctcgctggac 60
tgcagtgggc gcgggctggc gacgctgccc cgggacctgc cctcctggac gcgcagccta 120
aacctgagtt ataacagact ctccgagatc gactctgctg cttttgagga cttgacgaat 180
ctgcaggaag tgtacctcaa cagcaatgag ctgacagcca taccatcact gggcgctgct 240
tccataggag ttgtctctct ctttttgcag cacaacaaga tccttagtgt ggatgggagc 300
cagctgaagt cgtacctgtc cttggaagtg ctggatctga gttccaacaa catcacggaa 360
attcggagct cctgtttccc gaacggcctg cgtataaggg aactcaactt ggcgagcaac 420
cgcatcagca tcctggagtc tggagcattt gatggtctgt cgcggtcact gctgactctc 480
cgtctgagca aaaacaggat cacccagctt cctgtgaaag cgttcaagct acccaggctg 540
acacaactag acctgaatcg gaatcggatt cggctgattg aaggcctcac gttccagggg 600
ctcgacagct tagaggtgct gaggcttcag aggaacaaca tcagcaggct gacggacggg 660
gccttctggg ggctgtctaa gatgcacgtg ctgcacctgg agtacaacag tctggtggaa 720
gtgaacagtg gctccctcta tggcctcaca gccctgcacc agctgcacct cagcaacaac 780
tccatctctc gaattcagcg tgatggctgg agcttctgcc aaaagctgca tgagttgatt 840
ctgtccttca acaacctcac gcggctggat gaggagagtc tagcggagtt gagcagcctc 900
agtatcctgc gcctcagtca caacgccatc agtcacattg ctgaaggcgc cttcaaggga 960
ctcaagagtc tgcgggtctt ggacctggac cataacgaga tctcgggtac aatcgaggat 1020
accagtggtg cctttacggg gcttgacaac ctcagcaagc tgactctgtt tggaaacaag 1080
atcaaatctg tggctaagag agccttctcg ggcctggaaa gcctggaaca cctgaacctt 1140
ggagagaatg caatcaggtc tgtccagttt gatgcctttg caaagatgaa gaaccttaaa 1200
gagctctaca tcagcagtga gagcttcctg tgtgactgcc agctcaagtg gctgccccca 1260
tggctaatgg gtaggatgct gcaggccttt gtgacagcca cctgtgccca tccagagtcg 1320
ctgaagggcc agagcatttt ctcagtgctg ccagacagct ttgtgtgtga tgactttcca 1380

aagccacaga tcatcaccca gcctgagacg accatggctg tggtgggcaa ggacatccgt 1440
ttcacatgct ccgcagccag cagcagcagc tcaccaatga ccttcgcctg gaagaaggac 1500
aatgaggtcc tggccaatgc agacatggag aactttgccc acgtccgtgc acaggacggc 1560
gaagtgatgg agtataccac tatcctgcac ctccgtcacg tcacctttgg gcacgagggc 1620
cgctaccagt gtatcatcac aaaccacttt ggctccacat actcccacaa agccaggctc 1680
actgtgaatg tgttgccatc attcactaaa ataccccatg acattgccat ccggactggc 1740
accacagccc gcctcgagtg tgctgccacg ggccacccta accctcagat tgcctggcag 1800
aaggatggag gcaccgattt cccggcagct cgtgagcgac gcatgcatgt tatgccagac 1860
gatgatgtgt tcttcatcac tgatgtgaaa atagacgaca tgggggtcta cagctgcact 1920
gcccagaact cggcaggctc ggtttcagcc aacgctaccc tcacagtctt agaaactcca 1980
tccttggcag tgcctctgga agaccgtgtg gtaactgtgg gagaaacagt ggccttccag 2040
tgcaaagcaa ccgggagccc cacaccacgc atcacctggc ttaagggagg tcgcccattg 2100
agcctcacag agcgccacca tttcactcca ggcaaccagc tgctggttgt tcagaatgtg 2160
atgatagacg atgcagggcg gtatacctgt gagatgtcta atcccctggg cactgagcga 2220
gcacatagcc agctgagcat tttacctacc cctggctgcc ggaaggatgg gaccaccgta 2280
ggc
2283

<210> 5
<211> 5
<212> PRT
<213> Artificial Sequence
<220>
<223> A7 heavy chain CDR 1
<400> 5
Gly Tyr Asp Met Ser
1               5

<210> 6
<211> 9
<212> PRT
<213> Artificial Sequence

<220>

<223>	A7 heavy chain CDR 2

<400>	6

Leu Ile Tyr Pro Asp Ser Gly Asn Lys
1               5

<210>	7

<211>	13

<212>	PRT

<213>	Artificial Sequence

<220>

<223>	A7 heavy chain CDR 3

<400>	7

Arg Asp Ala Gly Leu Ser Trp Ala Gly Ala Phe Asp Tyr
1               5                   10

<210>	8

<211>	12

<212>	PRT

<213>	Artificial Sequence

<220>

<223>	A7 light chain CDR 1

<400>	8

Ser Gly Ser Ser Ser Asn Ile Gly Ser Asn Tyr Val
1               5                   10

<210>	9

<211>	4

<212>	PRT

<213>	Artificial Sequence

<220>

<223>	A7 light chain CDR 2

<400>	9
Ser Asp Ser His
1

<210>	10
<211>	9
<212>	PRT
<213>	Artificial Sequence
<220>
<223>	A7 light chain CDR 3
<400>	10
Gly Ser Trp Asp Tyr Ser Leu Ser Ala
1               5

<210>	11

<211>	121
<212>	PRT
<213>	Artificial Sequence
<220>
<223>	A7 heavy chain_variable region
<400>	11
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Gly Tyr
            20                  25                  30
Asp Met Ser Trp Val Arg Gln Ala Pro Gly Lys Gly Leu Glu Trp Val
        35                  40                  45
Ser Leu Ile Tyr Pro Asp Ser Gly Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95
Ala Arg Asp Ala Gly Leu Ser Trp Ala Gly Ala Phe Asp Tyr Trp Gly
            100                 105                 110
Gln Gly Thr Leu Val Thr Val Ser Ser
        115                 120

<210> 12
<211> 110
<212> PRT
<213> Artificial Sequence
<220>
<223> A7 light chain_variable region
<400> 12
Gln Ser Val Leu Thr Gln Pro Pro Ser Ala Ser Gly Thr Pro Gly Gln
1               5                   10                  15
Arg Val Thr Ile Ser Cys Ser Gly Ser Ser Ser Asn Ile Gly Ser Asn
            20                  25                  30
Tyr Val Thr Trp Tyr Gln Gln Leu Pro Gly Thr Ala Pro Lys Leu Leu
        35                  40                  45
Ile Tyr Ser Asp Ser His Arg Pro Ser Gly Val Pro Asp Arg Phe Ser
    50                  55                  60
Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Gly Ser Trp Asp Tyr Ser Leu
                85                  90                  95
Ser Ala Tyr Val Phe Gly Gly Gly Thr Lys Leu Thr Val Leu
            100                 105                 110

<210> 13
<211> 5

<212>	PRT
<213>	Artificial Sequence
<220>
<223>	C8 heavy chain CDR 1
<400>	13
Asn Tyr Tyr Met Ser
1               5

<210>	14
<211>	8
<212>	PRT
<213>	Artificial Sequence
<220>
<223>	C8 heavy chain CDR 2
<400>	14
Gly Ile Ser Pro Gly Asp Ser Ser
1               5

<210>	15
<211>	13
<212>	PRT
<213>	Artificial Sequence
<220>
<223>	C8 heavy chain CDR 3
<400>	15
Lys Gly Leu Tyr Ser Asn Pro Asn Glu Pro Phe Asp Tyr
1               5                   10

<210>	16
<211>	13
<212>	PRT
<213>	Artificial Sequence

<220>

<223>	C8 light chain CDR 1

<400>	16

Thr Gly Ser Ser Ser Asn Ile Gly Ser Asn Tyr Val Ser
1               5                   10

<210>	17

<211>	4

<212>	PRT

<213>	Artificial Sequence

<220>

<223>	C8 light chain CDR 2

<400>	17

Asp Asp Ser Gln
1

<210>	18

<211>	9

<212>	PRT

<213>	Artificial Sequence

<220>

<223>	C8 light chain CDR 3

<400>	18

Gly Thr Trp Asp Tyr Ser Leu Asn Gly
1               5

<210>	19

<211>	121

<212>	PRT

<213>	Artificial Sequence

<220>

<223>        C8 heavy chain_variable region

<400>        19

Glu Val Gln Leu Leu Glu Ser Gly Gly Gly Leu Val Gln Pro Gly Gly

1               5                   10                  15

Ser Leu Arg Leu Ser Cys Ala Ala Ser Gly Phe Thr Phe Ser Asn Tyr

            20                  25                  30

Tyr Met Ser Trp Val Arg Gln Ala Pro Gly Lys Gly Leu Glu Trp Val

        35                  40                  45

Ser Gly Ile Ser Pro Gly Asp Ser Ser Thr Tyr Tyr Ala Asp Ser Val

    50                  55                  60

Lys Gly Arg Phe Thr Ile Ser Arg Asp Asn Ser Lys Asn Thr Leu Tyr

65                  70                  75                  80

Leu Gln Met Asn Ser Leu Arg Ala Glu Asp Thr Ala Val Tyr Tyr Cys

                85                  90                  95

Ala Lys Gly Leu Tyr Ser Asn Pro Asn Glu Pro Phe Asp Tyr Trp Gly

            100                 105                 110

Gln Gly Thr Leu Val Thr Val Ser Ser

        115                 120

<210>        20

<211>        110

<212>        PRT

<213>        Artificial Sequence

<220>

<223>        C8 light chain_variable region

<400>        20

Gln Ser Val Leu Thr Gln Pro Pro Ser Ala Ser Gly Thr Pro Gly Gln

1               5                   10                  15

Arg Val Thr Ile Ser Cys Thr Gly Ser Ser Ser Asn Ile Gly Ser Asn

            20                  25                  30

Tyr Val Ser Trp Tyr Gln Gln Leu Pro Gly Thr Ala Pro Lys Leu Leu

        35                  40                  45

Ile Tyr Asp Asp Ser Gln Arg Pro Ser Gly Val Pro Asp Arg Phe Ser
    50                  55                  60
Gly Ser Lys Ser Gly Thr Ser Ala Ser Leu Ala Ile Ser Gly Leu Arg
65                  70                  75                  80
Ser Glu Asp Glu Ala Asp Tyr Tyr Cys Gly Thr Trp Asp Tyr Ser Leu
                85                  90                  95
Asn Gly Tyr Val Phe Gly Gly Gly Thr Lys Leu Thr Val Leu
            100                 105                 110

<210> 21
<211> 5
<212> PRT
<213> Artificial Sequence
<220>
<223> E7 heavy chain CDR 1
<400> 21
Ser Tyr Asp Met Ser
1               5

<210> 22
<211> 9
<212> PRT
<213> Artificial Sequence
<220>
<223> E7 heavy chain CDR 2
<400> 22
Gly Ile Ser Pro Asp Gly Ser Asn Ile
1               5

<210> 23
<211> 19

<212>	PRT
<213>	Artificial Sequence
<220>
<223>	E7 heavy chain CDR 3
<400>	23
Lys Val Gly Leu Arg Cys Arg Tyr Glu Ala Cys Ser Tyr Ala Tyr Gly
1               5                   10                  15

Met Asp Val

<210>	24
<211>	13
<212>	PRT
<213>	Artificial Sequence
<220>
<223>	E7 light chain CDR 1
<400>	24
Ser Gly Ser Ser Ser Asn Ile Gly Ser Asn Tyr Val Ser
1               5                   10

<210>	25
<211>	4
<212>	PRT
<213>	Artificial Sequence
<220>
<223>	E7 light chain CDR 2
<400>	25
Ser Asp Ser His
1

<210>	26
<211>	9

<212>	PRT
<213>	Artificial Sequence
<220>
<223>	E7 light chain CDR 3
<400>	26
Ala Thr Trp Asp Ser Ser Leu Asn Gly
1               5

<210>	27
<211>	127
<212>	PRT
<213>	Artificial Sequence
<220>
<223>	E7 heavy chain_variable region
<400>	27
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Asp Met Ser Trp Val Arg Gln Ala Pro Gly Lys Gly Leu Glu Trp Val
        35                  40                  45
Ser Gly Ile Ser Pro Asp Gly Ser Asn Ile Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Val Gly Leu Arg Cys Arg Tyr Glu Ala Cys Ser Tyr Ala Tyr
            100                 105                 110
Gly Met Asp Val Trp Gly Gln Gly Thr Leu Val Thr Val Ser Ser
        115                 120                 125

<210>	28
<211>	110
<212>	PRT
<213>	Artificial Sequence
<220>
<223>	E7 light chain_variable region
<400>	28
Gln Ser Val Leu Thr Gln Pro Pro Ser Ala Ser Gly Thr Pro Gly Gln
1               5                   10                  15
Arg Val Thr Ile Ser Cys Ser Gly Ser Ser Ser Asn Ile Gly Ser Asn
            20                  25                  30
Tyr Val Ser Trp Tyr Gln Gln Leu Pro Gly Thr Ala Pro Lys Leu Leu
        35                  40                  45
Ile Tyr Ser Asp Ser His Arg Pro Ser Gly Val Pro Asp Arg Phe Ser
    50                  55                  60
Gly Ser Lys Ser Gly Thr Ser Ala Ser Leu Ala Ile Ser Gly Leu Arg
65                  70                  75                  80
Ser Glu Asp Glu Ala Asp Tyr Tyr Cys Ala Thr Trp Asp Ser Ser Leu
                85                  90                  95
Asn Gly Tyr Val Phe Gly Gly Gly Thr Lys Leu Thr Val Leu
            100                 105                 110

<210>	29
<211>	5
<212>	PRT
<213>	Artificial Sequence
<220>
<223>	G3 heavy chain CDR 1
<400>	29
Asn Tyr Asp Met Ser
1               5

<210>	30
<211>	9
<212>	PRT
<213>	Artificial Sequence
<220>
<223>	G3 heavy chain CDR 2
<400>	30
Ser Ile Ser Pro Ser Ser Gly Ser Ile
1               5

<210>	31
<211>	13
<212>	PRT
<213>	Artificial Sequence
<220>
<223>	G3 heavy chain CDR 3
<400>	31
Lys Asp Leu Asp Ala Phe Trp Arg Pro Ser Phe Asp Tyr
1               5                   10

<210>	32
<211>	13
<212>	PRT
<213>	Artificial Sequence
<220>
<223>	G3 light chain CDR 1
<400>	32
Thr Gly Ser Ser Ser Asn Ile Gly Asn Asn Asn Val Asn
1               5                   10

<210>	33
<211>	4

<212>	PRT
<213>	Artificial Sequence
<220>
<223>	G3 light chain CDR 2
<400>	33
Ser Asp Ser His
1

<210>	34
<211>	9
<212>	PRT
<213>	Artificial Sequence
<220>
<223>	G3 light chain CDR 3
<400>	34
Gly Ser Trp Asp Asp Ser Leu Ser Ala
1               5

<210>	35
<211>	121
<212>	PRT
<213>	Artificial Sequence
<220>
<223>	G3 heavy chain_variable region
<400>	35
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Asn Tyr
            20                  25                  30
Asp Met Ser Trp Val Arg Gln Ala Pro Gly Lys Gly Leu Glu Trp Val
        35                  40                  45
Ser Ser Ile Ser Pro Ser Ser Gly Ser Ile Tyr Tyr Ala Asp Ser Val

50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Asp Leu Asp Ala Phe Trp Arg Pro Ser Phe Asp Tyr Trp Gly
            100                 105                 110
Gln Gly Thr Leu Val Thr Val Ser Ser
        115                 120

<210>   36
<211>   110
<212>   PRT
<213>   Artificial Sequence
<220>
<223>   G3 light chain_variable region
<400>   36
Gln Ser Val Leu Thr Gln Pro Pro Ser Ala Ser Gly Thr Pro Gly Gln
1               5                   10                  15
Arg Val Thr Ile Ser Cys Thr Gly Ser Ser Ser Asn Ile Gly Asn Asn
            20                  25                  30
Asn Val Asn Trp Tyr Gln Gln Leu Pro Gly Thr Ala Pro Lys Leu Leu
        35                  40                  45
Ile Tyr Ser Asp Ser His Arg Pro Ser Gly Val Pro Asp Arg Phe Ser
    50                  55                  60
Gly Ser Lys Ser Gly Thr Ser Ala Ser Leu Ala Ile Ser Gly Leu Arg
65                  70                  75                  80
Ser Glu Asp Glu Ala Asp Tyr Tyr Cys Gly Ser Trp Asp Asp Ser Leu
                85                  90                  95
Ser Ala Tyr Val Phe Gly Gly Gly Thr Lys Leu Thr Val Leu
            100                 105                 110

<210> 37

<211> 328

<212> PRT

<213> Mus musculus

<400> 37

Thr Thr Ala Pro Ser Val Tyr Pro Leu Ala Pro Val Cys Gly Asp Thr
1               5                   10                  15

Thr Gly Ser Ser Val Thr Leu Gly Cys Leu Val Lys Gly Tyr Phe Pro
            20                  25                  30

Glu Pro Val Thr Leu Thr Trp Asn Ser Gly Ser Leu Ser Ser Gly Val
        35                  40                  45

His Thr Phe Pro Ala Val Leu Gln Ser Asp Leu Tyr Thr Leu Ser Ser
    50                  55                  60

Ser Val Thr Val Thr Ser Ser Thr Trp Pro Ser Gln Ser Ile Thr Cys
65                  70                  75                  80

Asn Val Ala His Pro Ala Ser Ser Thr Lys Val Asp Lys Lys Ile Glu
                85                  90                  95

Pro Arg Gly Pro Thr Ile Lys Pro Cys Pro Pro Cys Lys Cys Pro Ala
            100                 105                 110

Pro Asn Leu Leu Gly Gly Pro Ser Val Phe Ile Phe Pro Pro Lys Ile
        115                 120                 125

Lys Asp Val Leu Met Ile Ser Leu Ser Pro Ile Val Thr Cys Val Val
    130                 135                 140

Val Asp Val Ser Glu Asp Asp Pro Asp Val Gln Ile Ser Trp Phe Val
145                 150                 155                 160

Asn Asn Val Glu Val His Thr Ala Gln Thr Gln Thr His Arg Glu Asp
                165                 170                 175

Tyr Asn Ser Thr Leu Arg Val Val Ser Ala Leu Pro Ile Gln His Gln
            180                 185                 190

Asp Trp Met Ser Gly Lys Glu Phe Lys Cys Lys Val Asn Asn Lys Asp
        195                 200                 205

Leu Pro Ala Pro Ile Glu Arg Thr Ile Ser Lys Pro Lys Gly Ser Val
    210                 215                 220
Arg Ala Pro Gln Val Tyr Val Leu Pro Pro Pro Glu Glu Glu Met Thr
225                 230                 235                 240
Lys Lys Gln Val Thr Leu Thr Cys Met Val Thr Asp Phe Met Pro Glu
                245                 250                 255
Asp Ile Tyr Val Glu Trp Thr Asn Asn Gly Lys Thr Glu Leu Asn Tyr
            260                 265                 270
Lys Asn Thr Glu Pro Val Leu Asp Ser Asp Gly Ser Tyr Phe Met Tyr
        275                 280                 285
Ser Lys Leu Arg Val Glu Lys Lys Asn Trp Val Glu Arg Asn Ser Tyr
    290                 295                 300
Ser Cys Ser Val Val His Glu Gly Leu His Asn His His Thr Thr Lys
305                 310                 315                 320
Ser Phe Ser Arg Thr Pro Gly Lys
                325

<210> 38
<211> 107
<212> PRT
<213> Mus musculus
<400> 38
Arg Thr Val Ala Ala Pro Thr Val Ser Ile Phe Pro Pro Ser Ser Glu
1               5                   10                  15
Gln Leu Thr Ser Gly Gly Ala Ser Val Val Cys Phe Leu Asn Asn Phe
            20                  25                  30
Tyr Pro Lys Asp Ile Asn Val Lys Trp Lys Ile Asp Gly Ser Glu Arg
        35                  40                  45
Gln Asn Gly Val Leu Asn Ser Trp Thr Asp Gln Asp Ser Lys Asp Ser
    50                  55                  60
Thr Tyr Ser Met Ser Ser Thr Leu Thr Leu Thr Lys Asp Glu Tyr Glu
65                  70                  75                  80

Arg His Asn Ser Tyr Thr Cys Glu Ala Thr His Lys Thr Ser Thr Ser
                85                  90                  95
Pro Ile Val Lys Ser Phe Asn Arg Asn Glu Cys
            100                 105

<210> 39
<211> 330
<212> PRT
<213> homo sapiens
<400> 39

<210> 40
<211> 107
<212> PRT
<213> Homo sapiens
<400> 40

<210> 41
<211> 326
<212> PRT
<213> Homo sapiens
<400> 41
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Asn Phe Gly Thr Gln Thr
65                  70                  75                  80
Tyr Thr Cys Asn Val Asp His Lys Pro Ser Asn Thr Lys Val Asp Lys
                85                  90                  95
Thr Val Glu Arg Lys Cys Cys Val Glu Cys Pro Pro Cys Pro Ala Pro
            100                 105                 110
Pro Val Ala Gly Pro Ser Val Phe Leu Phe Pro Pro Lys Pro Lys Asp
        115                 120                 125
Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160
Val Glu Val His Asn Ala Lys Thr Lys Pro Arg Glu Glu Gln Phe Asn
                165                 170                 175
Ser Thr Phe Arg Val Val Ser Val Leu Thr Val Val His Gln Asp Trp
            180                 185                 190
Leu Asn Gly Lys Glu Tyr Lys Cys Lys Val Ser Asn Lys Gly Leu Pro
        195                 200                 205
Ala Pro Ile Glu Lys Thr Ile Ser Lys Thr Lys Gly Gln Pro Arg Glu
    210                 215                 220
Pro Gln Val Tyr Thr Leu Pro Pro Ser Arg Glu Glu Met Thr Lys Asn
225                 230                 235                 240
Gln Val Ser Leu Thr Cys Leu Val Lys Gly Phe Tyr Pro Ser Asp Ile
                245                 250                 255
Ser Val Glu Trp Glu Ser Asn Gly Gln Pro Glu Asn Asn Tyr Lys Thr
            260                 265                 270
Thr Pro Pro Met Leu Asp Ser Asp Gly Ser Phe Phe Leu Tyr Ser Lys
        275                 280                 285
Leu Thr Val Asp Lys Ser Arg Trp Gln Gln Gly Asn Val Phe Ser Cys
    290                 295                 300
Ser Val Met His Glu Ala Leu His Asn His Tyr Thr Gln Lys Ser Leu
305                 310                 315                 320
Ser Leu Ser Pro Gly Lys
                325

<210>    42
<211>    377
<212>    PRT
<213>    Homo sapiens
<400>    42
Ala Ser Thr Lys Gly Pro Ser Val Phe Pro Leu Ala Pro Cys Ser Arg
1               5                   10                  15

Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Thr Cys Asn Val Asn His Lys Pro Ser Asn Thr Lys Val Asp Lys
                85                  90                  95
Arg Val Glu Leu Lys Thr Pro Leu Gly Asp Thr Thr His Thr Cys Pro
            100                 105                 110
Arg Cys Pro Glu Pro Lys Ser Cys Asp Thr Pro Pro Pro Cys Pro Arg
        115                 120                 125
Cys Pro Glu Pro Lys Ser Cys Asp Thr Pro Pro Pro Cys Pro Arg Cys
    130                 135                 140
Pro Glu Pro Lys Ser Cys Asp Thr Pro Pro Pro Cys Pro Arg Cys Pro
145                 150                 155                 160
Ala Pro Glu Leu Leu Gly Gly Pro Ser Val Phe Leu Phe Pro Pro Lys
                165                 170                 175
Pro Lys Asp Thr Leu Met Ile Ser Arg Thr Pro Glu Val Thr Cys Val
            180                 185                 190
Val Val Asp Val Ser His Glu Asp Pro Glu Val Gln Phe Lys Trp Tyr
        195                 200                 205
Val Asp Gly Val Glu Val His Asn Ala Lys Thr Lys Pro Arg Glu Glu
    210                 215                 220
Gln Tyr Asn Ser Thr Phe Arg Val Val Ser Val Leu Thr Val Leu His
225                 230                 235                 240
Gln Asp Trp Leu Asn Gly Lys Glu Tyr Lys Cys Lys Val Ser Asn Lys
                245                 250                 255
Ala Leu Pro Ala Pro Ile Glu Lys Thr Ile Ser Lys Thr Lys Gly Gln
            260                 265                 270

Pro Arg Glu Pro Gln Val Tyr Thr Leu Pro Pro Ser Arg Glu Glu Met
        275                 280                 285
Thr Lys Asn Gln Val Ser Leu Thr Cys Leu Val Lys Gly Phe Tyr Pro
    290                 295                 300
Ser Asp Ile Ala Val Glu Trp Glu Ser Ser Gly Gln Pro Glu Asn Asn
305                 310                 315                 320
Tyr Asn Thr Thr Pro Pro Met Leu Asp Ser Asp Gly Ser Phe Phe Leu
                325                 330                 335
Tyr Ser Lys Leu Thr Val Asp Lys Ser Arg Trp Gln Gln Gly Asn Ile
            340                 345                 350
Phe Ser Cys Ser Val Met His Glu Ala Leu His Asn Arg Phe Thr Gln
        355                 360                 365
Lys Ser Leu Ser Leu Ser Pro Gly Lys
    370                 375

<210> 43
<211> 327
<212> PRT
<213> Homo sapiens
<400> 43
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325

<210> 44

<211>	245
<212>	PRT
<213>	Artificial Sequence
<220>
<223>	Hybrid Fc_Heavy region
<400>	44
Arg Asn Thr Gly Arg Gly Gly Glu Glu Lys Lys Lys Glu Lys Glu Lys
1               5                   10                  15
Glu Glu Gln Glu Glu Arg Glu Thr Lys Thr Pro Glu Cys Pro Ser His
            20                  25                  30
Thr Gln Pro Leu Gly Val Phe Leu Phe Pro Pro Lys Pro Lys Asp Thr
        35                  40                  45
Leu Met Ile Ser Arg Thr Pro Glu Val Thr Cys Val Val Val Asp Val
    50                  55                  60
Ser Gln Glu Asp Pro Glu Val Gln Phe Asn Trp Tyr Val Asp Gly Val
65                  70                  75                  80
Glu Val His Asn Ala Lys Thr Lys Pro Arg Glu Glu Gln Phe Asn Ser
                85                  90                  95
Thr Tyr Arg Val Val Ser Val Leu Thr Val Leu His Gln Asp Trp Leu
            100                 105                 110
Asn Gly Lys Glu Tyr Lys Cys Lys Val Ser Asn Lys Gly Leu Pro Ser
        115                 120                 125
Ser Ile Glu Lys Thr Ile Ser Lys Ala Lys Gly Gln Pro Arg Glu Pro
    130                 135                 140
Gln Val Tyr Thr Leu Pro Pro Ser Gln Glu Glu Met Thr Lys Asn Gln
145                 150                 155                 160
Val Ser Leu Thr Cys Leu Val Lys Gly Phe Tyr Pro Ser Asp Ile Ala
                165                 170                 175
Val Glu Trp Glu Ser Asn Gly Gln Pro Glu Asn Asn Tyr Lys Thr Thr
            180                 185                 190
Pro Pro Val Leu Asp Ser Asp Gly Ser Phe Phe Leu Tyr Ser Arg Leu
        195                 200                 205

Thr Val Asp Lys Ser Arg Trp Gln Glu Gly Asn Val Phe Ser Cys Ser
    210                 215                 220
Val Met His Glu Ala Leu His Asn His Tyr Thr Gln Lys Ser Leu Ser
225                 230                 235                 240
Leu Ser Leu Gly Lys
                245

<210> 45
<211> 449
<212> PRT
<213> Artificial Sequence
<220>
<223> A7 heavy chain_mouse IgG2 Fc_full sequence
<400> 45
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Gly Tyr
            20                  25                  30
Asp Met Ser Trp Val Arg Gln Ala Pro Gly Lys Gly Leu Glu Trp Val
        35                  40                  45
Ser Leu Ile Tyr Pro Asp Ser Gly Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asp Ala Gly Leu Ser Trp Ala Gly Ala Phe Asp Tyr Trp Gly
            100                 105                 110
Gln Gly Thr Leu Val Thr Val Ser Ser Thr Thr Ala Pro Ser Val Tyr
        115                 120                 125
Pro Leu Ala Pro Val Cys Gly Asp Thr Thr Gly Ser Ser Val Thr Leu
    130                 135                 140

Gly Cys Leu Val Lys Gly Tyr Phe Pro Glu Pro Val Thr Leu Thr Trp
145                 150                 155                 160
Asn Ser Gly Ser Leu Ser Ser Gly Val His Thr Phe Pro Ala Val Leu
                165                 170                 175
Gln Ser Asp Leu Tyr Thr Leu Ser Ser Ser Val Thr Val Thr Ser Ser
            180                 185                 190
Thr Trp Pro Ser Gln Ser Ile Thr Cys Asn Val Ala His Pro Ala Ser
        195                 200                 205
Ser Thr Lys Val Asp Lys Lys Ile Glu Pro Arg Gly Pro Thr Ile Lys
    210                 215                 220
Pro Cys Pro Pro Cys Lys Cys Pro Ala Pro Asn Leu Leu Gly Gly Pro
225                 230                 235                 240
Ser Val Phe Ile Phe Pro Pro Lys Ile Lys Asp Val Leu Met Ile Ser
                245                 250                 255
Leu Ser Pro Ile Val Thr Cys Val Val Val Asp Val Ser Glu Asp Asp
            260                 265                 270
Pro Asp Val Gln Ile Ser Trp Phe Val Asn Asn Val Glu Val His Thr
        275                 280                 285
Ala Gln Thr Gln Thr His Arg Glu Asp Tyr Asn Ser Thr Leu Arg Val
    290                 295                 300
Val Ser Ala Leu Pro Ile Gln His Gln Asp Trp Met Ser Gly Lys Glu
305                 310                 315                 320
Phe Lys Cys Lys Val Asn Asn Lys Asp Leu Pro Ala Pro Ile Glu Arg
                325                 330                 335
Thr Ile Ser Lys Pro Lys Gly Ser Val Arg Ala Pro Gln Val Tyr Val
            340                 345                 350
Leu Pro Pro Pro Glu Glu Glu Met Thr Lys Lys Gln Val Thr Leu Thr
        355                 360                 365
Cys Met Val Thr Asp Phe Met Pro Glu Asp Ile Tyr Val Glu Trp Thr
    370                 375                 380
Asn Asn Gly Lys Thr Glu Leu Asn Tyr Lys Asn Thr Glu Pro Val Leu
385                 390                 395                 400

Asp Ser Asp Gly Ser Tyr Phe Met Tyr Ser Lys Leu Arg Val Glu Lys
                405                 410                 415
Lys Asn Trp Val Glu Arg Asn Ser Tyr Ser Cys Ser Val Val His Glu
            420                 425                 430
Gly Leu His Asn His His Thr Thr Lys Ser Phe Ser Arg Thr Pro Gly
        435                 440                 445
Lys

<210> 46
<211> 217
<212> PRT
<213> Artificial Sequence
<220>
<223> A7 light chain_mouse IgG2 Fc_full sequence
<400> 46
Gln Ser Val Leu Thr Gln Pro Pro Ser Ala Ser Gly Thr Pro Gly Gln
1               5                   10                  15
Arg Val Thr Ile Ser Cys Ser Gly Ser Ser Ser Asn Ile Gly Ser Asn
            20                  25                  30
Tyr Val Thr Trp Tyr Gln Gln Leu Pro Gly Thr Ala Pro Lys Leu Leu
        35                  40                  45
Ile Tyr Ser Asp Ser His Arg Pro Ser Gly Val Pro Asp Arg Phe Ser
    50                  55                  60
Gly Ser Lys Ser Gly Thr Ser Ala Ser Leu Ala Ile Ser Gly Leu Gln
65                  70                  75                  80
Ser Glu Asp Glu Ala Asp Tyr Tyr Cys Gly Ser Trp Asp Tyr Ser Leu
                85                  90                  95
Ser Ala Tyr Val Phe Gly Gly Gly Thr Lys Leu Thr Val Leu Arg Thr
            100                 105                 110
Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140
Lys Asp Ile Asn Val Lys Trp Lys Ile Asp Gly Ser Glu Arg Gln Asn
145                 150                 155                 160
Gly Val Leu Asn Ser Trp Thr Asp Gln Asp Ser Lys Asp Ser Thr Tyr
                165                 170                 175
Ser Met Ser Ser Thr Leu Thr Leu Thr Lys Asp Glu Tyr Glu Arg His
            180                 185                 190
Asn Ser Tyr Thr Cys Glu Ala Thr His Lys Thr Ser Thr Ser Pro Ile
        195                 200                 205
Val Lys Ser Phe Asn Arg Asn Glu Cys
    210                 215

<210> 47
<211> 449
<212> PRT
<213> Artificial Sequence
<220>
<223> C8 heavy chain_mouse IgG2 Fc_full sequence
<400> 47
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Asn Tyr
            20                  25                  30
Tyr Met Ser Trp Val Arg Gln Ala Pro Gly Lys Gly Leu Glu Trp Val
        35                  40                  45
Ser Gly Ile Ser Pro Gly Asp Ser Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95

Ala Lys Gly Leu Tyr Ser Asn Pro Asn Glu Pro Phe Asp Tyr Trp Gly
            100                 105                 110
Gln Gly Thr Leu Val Thr Val Ser Ser Thr Thr Ala Pro Ser Val Tyr
        115                 120                 125
Pro Leu Ala Pro Val Cys Gly Asp Thr Thr Gly Ser Ser Val Thr Leu
    130                 135                 140
Gly Cys Leu Val Lys Gly Tyr Phe Pro Glu Pro Val Thr Leu Thr Trp
145                 150                 155                 160
Asn Ser Gly Ser Leu Ser Ser Gly Val His Thr Phe Pro Ala Val Leu
                165                 170                 175
Gln Ser Asp Leu Tyr Thr Leu Ser Ser Ser Val Thr Val Thr Ser Ser
            180                 185                 190
Thr Trp Pro Ser Gln Ser Ile Thr Cys Asn Val Ala His Pro Ala Ser
        195                 200                 205
Ser Thr Lys Val Asp Lys Lys Ile Glu Pro Arg Gly Pro Thr Ile Lys
    210                 215                 220
Pro Cys Pro Pro Cys Lys Cys Pro Ala Pro Asn Leu Leu Gly Gly Pro
225                 230                 235                 240
Ser Val Phe Ile Phe Pro Pro Lys Ile Lys Asp Val Leu Met Ile Ser
                245                 250                 255
Leu Ser Pro Ile Val Thr Cys Val Val Val Asp Val Ser Glu Asp Asp
            260                 265                 270
Pro Asp Val Gln Ile Ser Trp Phe Val Asn Asn Val Glu Val His Thr
        275                 280                 285
Ala Gln Thr Gln Thr His Arg Glu Asp Tyr Asn Ser Thr Leu Arg Val
    290                 295                 300
Val Ser Ala Leu Pro Ile Gln His Gln Asp Trp Met Ser Gly Lys Glu
305                 310                 315                 320
Phe Lys Cys Lys Val Asn Asn Lys Asp Leu Pro Ala Pro Ile Glu Arg
                325                 330                 335
Thr Ile Ser Lys Pro Lys Gly Ser Val Arg Ala Pro Gln Val Tyr Val
            340                 345                 350

Leu Pro Pro Pro Glu Glu Glu Met Thr Lys Lys Gln Val Thr Leu Thr
        355                 360                 365
Cys Met Val Thr Asp Phe Met Pro Glu Asp Ile Tyr Val Glu Trp Thr
    370                 375                 380
Asn Asn Gly Lys Thr Glu Leu Asn Tyr Lys Asn Thr Glu Pro Val Leu
385                 390                 395                 400
Asp Ser Asp Gly Ser Tyr Phe Met Tyr Ser Lys Leu Arg Val Glu Lys
                405                 410                 415
Lys Asn Trp Val Glu Arg Asn Ser Tyr Ser Cys Ser Val Val His Glu
            420                 425                 430
Gly Leu His Asn His His Thr Thr Lys Ser Phe Ser Arg Thr Pro Gly
        435                 440                 445
Lys

<210> 48
<211> 217
<212> PRT
<213> Artificial Sequence
<220>
<223> C8 light chain_mouse IgG2 Fc_full sequence
<400> 48
Gln Ser Val Leu Thr Gln Pro Pro Ser Ala Ser Gly Thr Pro Gly Gln
1               5                   10                  15
Arg Val Thr Ile Ser Cys Thr Gly Ser Ser Ser Asn Ile Gly Ser Asn
            20                  25                  30
Tyr Val Ser Trp Tyr Gln Gln Leu Pro Gly Thr Ala Pro Lys Leu Leu
        35                  40                  45
Ile Tyr Asp Asp Ser Gln Arg Pro Ser Gly Val Pro Asp Arg Phe Ser
    50                  55                  60
Gly Ser Lys Ser Gly Thr Ser Ala Ser Leu Ala Ile Ser Gly Leu Arg
65                  70                  75                  80
Ser Glu Asp Glu Ala Asp Tyr Tyr Cys Gly Thr Trp Asp Tyr Ser Leu

85                                  90                                  95

Asn Gly Tyr Val Phe Gly Gly Gly Thr Lys Leu Thr Val Leu Arg Thr

                100                                 105                                 110

Val Ala Ala Pro Thr Val Ser Ile Phe Pro Pro Ser Ser Glu Gln Leu

            115                                 120                                 125

Thr Ser Gly Gly Ala Ser Val Val Cys Phe Leu Asn Asn Phe Tyr Pro

        130                                 135                                 140

Lys Asp Ile Asn Val Lys Trp Lys Ile Asp Gly Ser Glu Arg Gln Asn

145                                 150                                 155                                 160

Gly Val Leu Asn Ser Trp Thr Asp Gln Asp Ser Lys Asp Ser Thr Tyr

                        165                                 170                                 175

Ser Met Ser Ser Thr Leu Thr Leu Thr Lys Asp Glu Tyr Glu Arg His

                180                                 185                                 190

Asn Ser Tyr Thr Cys Glu Ala Thr His Lys Thr Ser Thr Ser Pro Ile

            195                                 200                                 205

Val Lys Ser Phe Asn Arg Asn Glu Cys

        210                                 215

<210>        49

<211>        455

<212>        PRT

<213>        Artificial Sequence

<220>

<223>        E7 heavy chain_mouse IgG2 Fc_full sequence

<400>        49

Glu Val Gln Leu Leu Glu Ser Gly Gly Gly Leu Val Gln Pro Gly Gly

  1                                 5                                   10                                  15

Ser Leu Arg Leu Ser Cys Ala Ala Ser Gly Phe Thr Phe Ser Ser Tyr

                    20                                  25                                  30

Asp Met Ser Trp Val Arg Gln Ala Pro Gly Lys Gly Leu Glu Trp Val

            35                                  40                                  45

Ser Gly Ile Ser Pro Asp Gly Ser Asn Ile Tyr Tyr Ala Asp Ser Val

    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Val Gly Leu Arg Cys Arg Tyr Glu Ala Cys Ser Tyr Ala Tyr
            100                 105                 110
Gly Met Asp Val Trp Gly Gln Gly Thr Leu Val Thr Val Ser Ser Thr
        115                 120                 125
Thr Ala Pro Ser Val Tyr Pro Leu Ala Pro Val Cys Gly Asp Thr Thr
    130                 135                 140
Gly Ser Ser Val Thr Leu Gly Cys Leu Val Lys Gly Tyr Phe Pro Glu
145                 150                 155                 160
Pro Val Thr Leu Thr Trp Asn Ser Gly Ser Leu Ser Ser Gly Val His
                165                 170                 175
Thr Phe Pro Ala Val Leu Gln Ser Asp Leu Tyr Thr Leu Ser Ser Ser
            180                 185                 190
Val Thr Val Thr Ser Ser Thr Trp Pro Ser Gln Ser Ile Thr Cys Asn
        195                 200                 205
Val Ala His Pro Ala Ser Ser Thr Lys Val Asp Lys Lys Ile Glu Pro
    210                 215                 220
Arg Gly Pro Thr Ile Lys Pro Cys Pro Pro Cys Lys Cys Pro Ala Pro
225                 230                 235                 240
Asn Leu Leu Gly Gly Pro Ser Val Phe Ile Phe Pro Pro Lys Ile Lys
                245                 250                 255
Asp Val Leu Met Ile Ser Leu Ser Pro Ile Val Thr Cys Val Val Val
            260                 265                 270
Asp Val Ser Glu Asp Asp Pro Asp Val Gln Ile Ser Trp Phe Val Asn
        275                 280                 285
Asn Val Glu Val His Thr Ala Gln Thr Gln Thr His Arg Glu Asp Tyr
    290                 295                 300
Asn Ser Thr Leu Arg Val Val Ser Ala Leu Pro Ile Gln His Gln Asp

305                    310                    315                    320

Trp Met Ser Gly Lys Glu Phe Lys Cys Lys Val Asn Asn Lys Asp Leu

                325                    330                    335

Pro Ala Pro Ile Glu Arg Thr Ile Ser Lys Pro Lys Gly Ser Val Arg

            340                    345                    350

Ala Pro Gln Val Tyr Val Leu Pro Pro Pro Glu Glu Glu Met Thr Lys

        355                    360                    365

Lys Gln Val Thr Leu Thr Cys Met Val Thr Asp Phe Met Pro Glu Asp

    370                    375                    380

Ile Tyr Val Glu Trp Thr Asn Asn Gly Lys Thr Glu Leu Asn Tyr Lys

385                    390                    395                    400

Asn Thr Glu Pro Val Leu Asp Ser Asp Gly Ser Tyr Phe Met Tyr Ser

                405                    410                    415

Lys Leu Arg Val Glu Lys Lys Asn Trp Val Glu Arg Asn Ser Tyr Ser

            420                    425                    430

Cys Ser Val Val His Glu Gly Leu His Asn His His Thr Thr Lys Ser

        435                    440                    445

Phe Ser Arg Thr Pro Gly Lys

    450                    455

<210>    50

<211>    217

<212>    PRT

<213>    Artificial Sequence

<220>

<223>    E7 light chain_mouse IgG2 Fc_full sequence

<400>    50

Gln Ser Val Leu Thr Gln Pro Pro Ser Ala Ser Gly Thr Pro Gly Gln

1                5                    10                    15

Arg Val Thr Ile Ser Cys Ser Gly Ser Ser Ser Asn Ile Gly Ser Asn

            20                    25                    30

Tyr Val Ser Trp Tyr Gln Gln Leu Pro Gly Thr Ala Pro Lys Leu Leu

35                    40                    45
Ile Tyr Ser Asp Ser His Arg Pro Ser Gly Val Pro Asp Arg Phe Ser
    50                    55                    60
Gly Ser Lys Ser Gly Thr Ser Ala Ser Leu Ala Ile Ser Gly Leu Arg
65                    70                    75                    80
Ser Glu Asp Glu Ala Asp Tyr Tyr Cys Ala Thr Trp Asp Ser Ser Leu
                85                    90                    95
Asn Gly Tyr Val Phe Gly Gly Gly Thr Lys Leu Thr Val Leu Arg Thr
            100                    105                    110
Val Ala Ala Pro Thr Val Ser Ile Phe Pro Pro Ser Ser Glu Gln Leu
        115                    120                    125
Thr Ser Gly Gly Ala Ser Val Val Cys Phe Leu Asn Asn Phe Tyr Pro
    130                    135                    140
Lys Asp Ile Asn Val Lys Trp Lys Ile Asp Gly Ser Glu Arg Gln Asn
145                    150                    155                    160
Gly Val Leu Asn Ser Trp Thr Asp Gln Asp Ser Lys Asp Ser Thr Tyr
                165                    170                    175
Ser Met Ser Ser Thr Leu Thr Leu Thr Lys Asp Glu Tyr Glu Arg His
            180                    185                    190
Asn Ser Tyr Thr Cys Glu Ala Thr His Lys Thr Ser Thr Ser Pro Ile
        195                    200                    205
Val Lys Ser Phe Asn Arg Asn Glu Cys
    210                    215

<210>    51
<211>    449
<212>    PRT
<213>    Artificial Sequence
<220>
<223>    G3 heavy chain_mouse IgG2 Fc_full sequence
<400>    51
Glu Val Gln Leu Leu Glu Ser Gly Gly Gly Leu Val Gln Pro Gly Gly

1                   5                   10                  15
Ser Leu Arg Leu Ser Cys Ala Ala Ser Gly Phe Thr Phe Ser Asn Tyr
                20                  25                  30
Asp Met Ser Trp Val Arg Gln Ala Pro Gly Lys Gly Leu Glu Trp Val
            35                  40                  45
Ser Ser Ile Ser Pro Ser Ser Gly Ser Ile Tyr Tyr Ala Asp Ser Val
        50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Asp Leu Asp Ala Phe Trp Arg Pro Ser Phe Asp Tyr Trp Gly
                100                 105                 110
Gln Gly Thr Leu Val Thr Val Ser Ser Thr Thr Ala Pro Ser Val Tyr
            115                 120                 125
Pro Leu Ala Pro Val Cys Gly Asp Thr Thr Gly Ser Ser Val Thr Leu
        130                 135                 140
Gly Cys Leu Val Lys Gly Tyr Phe Pro Glu Pro Val Thr Leu Thr Trp
145                 150                 155                 160
Asn Ser Gly Ser Leu Ser Ser Gly Val His Thr Phe Pro Ala Val Leu
                165                 170                 175
Gln Ser Asp Leu Tyr Thr Leu Ser Ser Ser Val Thr Val Thr Ser Ser
                180                 185                 190
Thr Trp Pro Ser Gln Ser Ile Thr Cys Asn Val Ala His Pro Ala Ser
            195                 200                 205
Ser Thr Lys Val Asp Lys Lys Ile Glu Pro Arg Gly Pro Thr Ile Lys
        210                 215                 220
Pro Cys Pro Pro Cys Lys Cys Pro Ala Pro Asn Leu Leu Gly Gly Pro
225                 230                 235                 240
Ser Val Phe Ile Phe Pro Pro Lys Ile Lys Asp Val Leu Met Ile Ser
                245                 250                 255
Leu Ser Pro Ile Val Thr Cys Val Val Val Asp Val Ser Glu Asp Asp

            260                 265                 270
Pro Asp Val Gln Ile Ser Trp Phe Val Asn Asn Val Glu Val His Thr
        275                 280                 285
Ala Gln Thr Gln Thr His Arg Glu Asp Tyr Asn Ser Thr Leu Arg Val
    290                 295                 300
Val Ser Ala Leu Pro Ile Gln His Gln Asp Trp Met Ser Gly Lys Glu
305                 310                 315                 320
Phe Lys Cys Lys Val Asn Asn Lys Asp Leu Pro Ala Pro Ile Glu Arg
                325                 330                 335
Thr Ile Ser Lys Pro Lys Gly Ser Val Arg Ala Pro Gln Val Tyr Val
            340                 345                 350
Leu Pro Pro Pro Glu Glu Glu Met Thr Lys Lys Gln Val Thr Leu Thr
        355                 360                 365
Cys Met Val Thr Asp Phe Met Pro Glu Asp Ile Tyr Val Glu Trp Thr
    370                 375                 380
Asn Asn Gly Lys Thr Glu Leu Asn Tyr Lys Asn Thr Glu Pro Val Leu
385                 390                 395                 400
Asp Ser Asp Gly Ser Tyr Phe Met Tyr Ser Lys Leu Arg Val Glu Lys
                405                 410                 415
Lys Asn Trp Val Glu Arg Asn Ser Tyr Ser Cys Ser Val Val His Glu
            420                 425                 430
Gly Leu His Asn His His Thr Thr Lys Ser Phe Ser Arg Thr Pro Gly
        435                 440                 445
Lys

<210> 52
<211> 217
<212> PRT
<213> Artificial Sequence
<220>
<223> G3 light chain_mouse IgG2 Fc_full sequence
<400> 52

Gln Ser Val Leu Thr Gln Pro Pro Ser Ala Ser Gly Thr Pro Gly Gln
1               5                   10                  15
Arg Val Thr Ile Ser Cys Thr Gly Ser Ser Ser Asn Ile Gly Asn Asn
            20                  25                  30
Asn Val Asn Trp Tyr Gln Gln Leu Pro Gly Thr Ala Pro Lys Leu Leu
        35                  40                  45
Ile Tyr Ser Asp Ser His Arg Pro Ser Gly Val Pro Asp Arg Phe Ser
    50                  55                  60
Gly Ser Lys Ser Gly Thr Ser Ala Ser Leu Ala Ile Ser Gly Leu Arg
65                  70                  75                  80
Ser Glu Asp Glu Ala Asp Tyr Tyr Cys Gly Ser Trp Asp Asp Ser Leu
                85                  90                  95
Ser Ala Tyr Val Phe Gly Gly Gly Thr Lys Leu Thr Val Leu Arg Thr
            100                 105                 110
Val Ala Ala Pro Thr Val Ser Ile Phe Pro Pro Ser Ser Glu Gln Leu
        115                 120                 125
Thr Ser Gly Gly Ala Ser Val Val Cys Phe Leu Asn Asn Phe Tyr Pro
    130                 135                 140
Lys Asp Ile Asn Val Lys Trp Lys Ile Asp Gly Ser Glu Arg Gln Asn
145                 150                 155                 160
Gly Val Leu Asn Ser Trp Thr Asp Gln Asp Ser Lys Asp Ser Thr Tyr
                165                 170                 175
Ser Met Ser Ser Thr Leu Thr Leu Thr Lys Asp Glu Tyr Glu Arg His
            180                 185                 190
Asn Ser Tyr Thr Cys Glu Ala Thr His Lys Thr Ser Thr Ser Pro Ile
        195                 200                 205
Val Lys Ser Phe Asn Arg Asn Glu Cys
    210                 215

<210>   53
<211>   330
<212>   PRT

<213>	Homo sapiens
<400>	53
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
                325                 330

<110>    Good T Cells, Inc.

<120>    BINDING MOLECULE SPECIFIC TO LRIG-1 PROTEIN AND USE THEREOF

<130>    POPB194162PCT

<150>    KR 10-2018-0123858
<151>    2018-10-17

<160>    53

<170>    KoPatentIn 3.0

<210>    1
<211>    759
<212>    PRT
<213>    Homo sapiens

<400>    1
Gly Pro Arg Ala Pro Cys Ala Ala Ala Cys Thr Cys Ala Gly Asp Ser
  1               5                  10                  15

Leu Asp Cys Gly Gly Arg Gly Leu Ala Ala Leu Pro Gly Asp Leu Pro
             20                  25                  30

Ser Trp Thr Arg Ser Leu Asn Leu Ser Tyr Asn Lys Leu Ser Glu Ile
         35                  40                  45

Asp Pro Ala Gly Phe Glu Asp Leu Pro Asn Leu Gln Glu Val Tyr Leu
     50                  55                  60

Asn Asn Asn Glu Leu Thr Ala Val Pro Ser Leu Gly Ala Ala Ser Ser
 65                  70                  75                  80

His Val Val Ser Leu Phe Leu Gln His Asn Lys Ile Arg Ser Val Glu
                 85                  90                  95

Gly Ser Gln Leu Lys Ala Tyr Leu Ser Leu Glu Val Leu Asp Leu Ser
            100                 105                 110

Leu Asn Asn Ile Thr Glu Val Arg Asn Thr Cys Phe Pro His Gly Pro
        115                 120                 125

Pro Ile Lys Glu Leu Asn Leu Ala Gly Asn Arg Ile Gly Thr Leu Glu
    130                 135                 140

Leu Gly Ala Phe Asp Gly Leu Ser Arg Ser Leu Leu Thr Leu Arg Leu
145                 150                 155                 160

Ser Lys Asn Arg Ile Thr Gln Leu Pro Val Arg Ala Phe Lys Leu Pro
                165                 170                 175

Arg Leu Thr Gln Leu Asp Leu Asn Arg Asn Arg Ile Arg Leu Ile Glu
            180                 185                 190

Gly Leu Thr Phe Gln Gly Leu Asn Ser Leu Glu Val Leu Lys Leu Gln
        195                 200                 205

Arg Asn Asn Ile Ser Lys Leu Thr Asp Gly Ala Phe Trp Gly Leu Ser
    210                 215                 220

Lys Met His Val Leu His Leu Glu Tyr Asn Ser Leu Val Glu Val Asn
225                 230                 235                 240

Ser Gly Ser Leu Tyr Gly Leu Thr Ala Leu His Gln Leu His Leu Ser
                245                 250                 255

Asn Asn Ser Ile Ala Arg Ile His Arg Lys Gly Trp Ser Phe Cys Gln
            260                 265                 270

Lys Leu His Glu Leu Val Leu Ser Phe Asn Asn Leu Thr Arg Leu Asp
        275                 280                 285

Glu Glu Ser Leu Ala Glu Leu Ser Ser Leu Ser Val Leu Arg Leu Ser
    290                 295                 300

His Asn Ser Ile Ser His Ile Ala Glu Gly Ala Phe Lys Gly Leu Arg
305                 310                 315                 320

Ser Leu Arg Val Leu Asp Leu Asp His Asn Glu Ile Ser Gly Thr Ile
                325                 330                 335

Glu Asp Thr Ser Gly Ala Phe Ser Gly Leu Asp Ser Leu Ser Lys Leu
            340                 345                 350

Thr Leu Phe Gly Asn Lys Ile Lys Ser Val Ala Lys Arg Ala Phe Ser
        355                 360                 365

Gly Leu Glu Gly Leu Glu His Leu Asn Leu Gly Gly Asn Ala Ile Arg
    370                 375                 380

Ser Val Gln Phe Asp Ala Phe Val Lys Met Lys Asn Leu Lys Glu Leu
385                 390                 395                 400

His Ile Ser Ser Asp Ser Phe Leu Cys Asp Cys Gln Leu Lys Trp Leu
                405                 410                 415

Pro Pro Trp Leu Ile Gly Arg Met Leu Gln Ala Phe Val Thr Ala Thr
            420                 425                 430

Cys Ala His Pro Glu Ser Leu Lys Gly Gln Ser Ile Phe Ser Val Pro
        435                 440                 445

Pro Glu Ser Phe Val Cys Asp Asp Phe Leu Lys Pro Gln Ile Ile Thr
    450                 455                 460

Gln Pro Glu Thr Thr Met Ala Met Val Gly Lys Asp Ile Arg Phe Thr
465                 470                 475                 480

Cys Ser Ala Ala Ser Ser Ser Ser Ser Pro Met Thr Phe Ala Trp Lys
                485                 490                 495

Lys Asp Asn Glu Val Leu Thr Asn Ala Asp Met Glu Asn Phe Val His
            500                 505                 510

Val His Ala Gln Asp Gly Glu Val Met Glu Tyr Thr Thr Ile Leu His
        515                 520                 525

Leu Arg Gln Val Thr Phe Gly His Glu Gly Arg Tyr Gln Cys Val Ile
    530                 535                 540

Thr Asn His Phe Gly Ser Thr Tyr Ser His Lys Ala Arg Leu Thr Val
545                 550                 555                 560

Asn Val Leu Pro Ser Phe Thr Lys Thr Pro His Asp Ile Thr Ile Arg
                565                 570                 575

Thr Thr Thr Val Ala Arg Leu Glu Cys Ala Ala Thr Gly His Pro Asn
                580                 585                 590

Pro Gln Ile Ala Trp Gln Lys Asp Gly Gly Thr Asp Phe Pro Ala Ala
            595                 600                 605

Arg Glu Arg Arg Met His Val Met Pro Asp Asp Asp Val Phe Phe Ile
    610                 615                 620

Thr Asp Val Lys Ile Asp Asp Ala Gly Val Tyr Ser Cys Thr Ala Gln
625                 630                 635                 640

Asn Ser Ala Gly Ser Ile Ser Ala Asn Ala Thr Leu Thr Val Leu Glu
                645                 650                 655

Thr Pro Ser Leu Val Val Pro Leu Glu Asp Arg Val Val Ser Val Gly
                660                 665                 670

Glu Thr Val Ala Leu Gln Cys Lys Ala Thr Gly Asn Pro Pro Pro Arg
            675                 680                 685

Ile Thr Trp Phe Lys Gly Asp Arg Pro Leu Ser Leu Thr Glu Arg His
    690                 695                 700

His Leu Thr Pro Asp Asn Gln Leu Leu Val Val Gln Asn Val Val Ala
705                 710                 715                 720

Glu Asp Ala Gly Arg Tyr Thr Cys Glu Met Ser Asn Thr Leu Gly Thr
                725                 730                 735

Glu Arg Ala His Ser Gln Leu Ser Val Leu Pro Ala Ala Gly Cys Arg
                740                 745                 750

Lys Asp Gly Thr Thr Val Gly
            755


<210>    2
<211>    2397
<212>    DNA
<213>    Homo sapiens

<400>    2
ggcccgcggg cgccctgcgc ggccgcctgc acttgcgctg gggactcgct ggactgcggt        60

gggcgcgggc tggctgcgtt gcccggggac ctgccctcct ggacgcggag cctaaacctg       120

agttacaaca aactctctga gattgaccct gctggttttg aggacttgcc gaacctacag       180

gaagtgtacc tcaataataa tgagttgaca gcggtaccat ccctgggcgc tgcttcatca       240

catgtcgtct ctctctttct gcagcacaac aagattcgca gcgtggaggg gagccagctg       300

aaggcctacc tttccttaga agtgttagat ctgagtttga acaacatcac ggaagtgcgg       360

aacacctgct ttccacacgg accgcctata aaggagctca acctggcagg caatcggatt       420

ggcaccctgg agttgggagc atttgatggt ctgtcacggt cgctgctaac tcttcgcctg       480

agcaaaaaca ggatcaccca gcttcctgta agagcattca agctacccag gctgacacaa       540

ctggacctca atcggaacag gattcggctg atagagggcc tcaccttcca ggggctcaac       600

agcttggagg tgctgaagct tcagcgaaac aacatcagca aactgacaga tggggccttc 660

tggggactgt ccaagatgca tgtgctgcac ctggagtaca acagcctggt agaagtgaac 720

agcggctcgc tctacggcct cacggccctg catcagctcc acctcagcaa caattccatc 780

gctcgcattc accgcaaggg ctggagcttc tgccagaagc tgcatgagtt ggtcctgtcc 840

ttcaacaacc tgacacggct ggacgaggag agcctggccg agctgagcag cctgagtgtc 900

ctgcgtctca gccacaattc catcagccac attgcggagg gtgccttcaa gggactcagg 960

agcctgcgag tcttggatct ggaccataac gagatttcgg gcacaataga ggacacgagc 1020

ggcgccttct cagggctcga cagcctcagc aagctgactc tgtttggaaa caagatcaag 1080

tctgtggcta agagagcatt ctcggggctg gaaggcctgg agcacctgaa ccttggaggg 1140

aatgcgatca gatctgtcca gtttgatgcc tttgtgaaga tgaagaatct taaagagctc 1200

catatcagca gcgacagctt cctgtgtgac tgccagctga agtggctgcc cccgtggcta 1260

attggcagga tgctgcaggc ctttgtgaca gccacctgtg cccacccaga atcactgaag 1320

ggtcagagca ttttctctgt gccaccagag agtttcgtgt gcgatgactt cctgaagcca 1380

cagatcatca cccagccaga aaccaccatg gctatggtgg gcaaggacat ccggtttaca 1440

tgctcagcag ccagcagcag cagctccccc atgacctttg cctggaagaa agacaatgaa 1500

gtcctgacca atgcagacat ggagaacttt gtccacgtcc acgcgcagga cggggaagtg 1560

atggagtaca ccaccatcct gcacctccgt caggtcactt tcgggcacga gggccgctac 1620

caatgtgtca tcaccaacca ctttggctcc acctattcac ataaggccag gctcaccgtg 1680

aatgtgttgc catcattcac caaaacgccc cacgacataa ccatccggac caccaccgtg 1740

gcccgcctcg aatgtgctgc cacaggtcac ccaaaccctc agattgcctg gcagaaggat 1800

ggaggcacgg atttccccgc tgcccgtgag cgacgcatgc atgtcatgcc ggatgacgac 1860

gtgtttttca tcactgatgt gaaaatagat gacgcagggg tttacagctg tactgctcag 1920

aactcagccg gttctatttc agctaatgcc accctgactg tcctagagac cccatccttg 1980

gtggtcccct tggaagaccg tgtggtatct gtgggagaaa cagtggccct ccaatgcaaa 2040

gccacgggga accctccgcc ccgcatcacc tggttcaagg gggaccgccc gctgagcctc 2100

actgagcggc accacctgac ccctgacaac cagctcctgg tggttcagaa cgtggtggca 2160

gaggatgcgg gccgatatac ctgtgagatg tccaacaccc tgggcacgga gcgagctcac 2220

agccagctga gcgtcctgcc ccgcagcagc tgcaggaagg atgggaccac ggtaggcatc 2280

ttcaccattg ctgtcgtgag cagcatcgtc ctgacgtcac tggtctgggt gtgcatcatc 2340

taccagacca ggaagaagag tgaagagtac agtgtcacca acacagatga aaccgtc 2397

<210> 3

<211>    761
<212>    PRT
<213>    Mus musculus

<400>    3
Gln Ala Gly Pro Arg Ala Pro Cys Ala Ala Ala Cys Thr Cys Ala Gly
1               5                   10                  15

Asp Ser Leu Asp Cys Ser Gly Arg Gly Leu Ala Thr Leu Pro Arg Asp
            20                  25                  30

Leu Pro Ser Trp Thr Arg Ser Leu Asn Leu Ser Tyr Asn Arg Leu Ser
        35                  40                  45

Glu Ile Asp Ser Ala Ala Phe Glu Asp Leu Thr Asn Leu Gln Glu Val
    50                  55                  60

Tyr Leu Asn Ser Asn Glu Leu Thr Ala Ile Pro Ser Leu Gly Ala Ala
65                  70                  75                  80

Ser Ile Gly Val Val Ser Leu Phe Leu Gln His Asn Lys Ile Leu Ser
                85                  90                  95

Val Asp Gly Ser Gln Leu Lys Ser Tyr Leu Ser Leu Glu Val Leu Asp
            100                 105                 110

Leu Ser Ser Asn Asn Ile Thr Glu Ile Arg Ser Ser Cys Phe Pro Asn
        115                 120                 125

Gly Leu Arg Ile Arg Glu Leu Asn Leu Ala Ser Asn Arg Ile Ser Ile
    130                 135                 140

Leu Glu Ser Gly Ala Phe Asp Gly Leu Ser Arg Ser Leu Leu Thr Leu
145                 150                 155                 160

Arg Leu Ser Lys Asn Arg Ile Thr Gln Leu Pro Val Lys Ala Phe Lys
                165                 170                 175

Leu Pro Arg Leu Thr Gln Leu Asp Leu Asn Arg Asn Arg Ile Arg Leu
            180                 185                 190

Ile Glu Gly Leu Thr Phe Gln Gly Leu Asp Ser Leu Glu Val Leu Arg
        195                 200                 205

Leu Gln Arg Asn Asn Ile Ser Arg Leu Thr Asp Gly Ala Phe Trp Gly
    210                 215                 220

Leu Ser Lys Met His Val Leu His Leu Glu Tyr Asn Ser Leu Val Glu
225                 230                 235                 240

Val Asn Ser Gly Ser Leu Tyr Gly Leu Thr Ala Leu His Gln Leu His
                245                 250                 255

Leu Ser Asn Asn Ser Ile Ser Arg Ile Gln Arg Asp Gly Trp Ser Phe
            260                 265                 270

Cys Gln Lys Leu His Glu Leu Ile Leu Ser Phe Asn Asn Leu Thr Arg
        275                 280                 285

Leu Asp Glu Glu Ser Leu Ala Glu Leu Ser Ser Leu Ser Ile Leu Arg
    290                 295                 300

Leu Ser His Asn Ala Ile Ser His Ile Ala Glu Gly Ala Phe Lys Gly

305                 310                 315                 320

Leu Lys Ser Leu Arg Val Leu Asp Leu Asp His Asn Glu Ile Ser Gly
                325                 330                 335

Thr Ile Glu Asp Thr Ser Gly Ala Phe Thr Gly Leu Asp Asn Leu Ser
            340                 345                 350

Lys Leu Thr Leu Phe Gly Asn Lys Ile Lys Ser Val Ala Lys Arg Ala
        355                 360                 365

Phe Ser Gly Leu Glu Ser Leu Glu His Leu Asn Leu Gly Glu Asn Ala
    370                 375                 380

Ile Arg Ser Val Gln Phe Asp Ala Phe Ala Lys Met Lys Asn Leu Lys
385                 390                 395                 400

Glu Leu Tyr Ile Ser Ser Glu Ser Phe Leu Cys Asp Cys Gln Leu Lys
                405                 410                 415

Trp Leu Pro Pro Trp Leu Met Gly Arg Met Leu Gln Ala Phe Val Thr
            420                 425                 430

Ala Thr Cys Ala His Pro Glu Ser Leu Lys Gly Gln Ser Ile Phe Ser
        435                 440                 445

Val Leu Pro Asp Ser Phe Val Cys Asp Asp Phe Pro Lys Pro Gln Ile
    450                 455                 460

Ile Thr Gln Pro Glu Thr Thr Met Ala Val Val Gly Lys Asp Ile Arg
465                 470                 475                 480

Phe Thr Cys Ser Ala Ala Ser Ser Ser Ser Ser Pro Met Thr Phe Ala
                485                 490                 495

Trp Lys Lys Asp Asn Glu Val Leu Ala Asn Ala Asp Met Glu Asn Phe
            500                 505                 510

Ala His Val Arg Ala Gln Asp Gly Glu Val Met Glu Tyr Thr Thr Ile
        515                 520                 525

Leu His Leu Arg His Val Thr Phe Gly His Glu Gly Arg Tyr Gln Cys
    530                 535                 540

Ile Ile Thr Asn His Phe Gly Ser Thr Tyr Ser His Lys Ala Arg Leu
545                 550                 555                 560

Thr Val Asn Val Leu Pro Ser Phe Thr Lys Ile Pro His Asp Ile Ala
                565                 570                 575

Ile Arg Thr Gly Thr Thr Ala Arg Leu Glu Cys Ala Ala Thr Gly His
            580                 585                 590

Pro Asn Pro Gln Ile Ala Trp Gln Lys Asp Gly Gly Thr Asp Phe Pro
        595                 600                 605

Ala Ala Arg Glu Arg Arg Met His Val Met Pro Asp Asp Asp Val Phe
    610                 615                 620

Phe Ile Thr Asp Val Lys Ile Asp Asp Met Gly Val Tyr Ser Cys Thr
625                 630                 635                 640

Ala Gln Asn Ser Ala Gly Ser Val Ser Ala Asn Ala Thr Leu Thr Val

645                 650                 655

Leu Glu Thr Pro Ser Leu Ala Val Pro Leu Glu Asp Arg Val Val Thr
            660                 665                 670

Val Gly Glu Thr Val Ala Phe Gln Cys Lys Ala Thr Gly Ser Pro Thr
        675                 680                 685

Pro Arg Ile Thr Trp Leu Lys Gly Gly Arg Pro Leu Ser Leu Thr Glu
    690                 695                 700

Arg His His Phe Thr Pro Gly Asn Gln Leu Leu Val Val Gln Asn Val
705                 710                 715                 720

Met Ile Asp Asp Ala Gly Arg Tyr Thr Cys Glu Met Ser Asn Pro Leu
                725                 730                 735

Gly Thr Glu Arg Ala His Ser Gln Leu Ser Ile Leu Pro Thr Pro Gly
            740                 745                 750

Cys Arg Lys Asp Gly Thr Thr Val Gly
        755                 760

<210>    4
<211>    2283
<212>    DNA
<213>    Mus musculus

<400>    4
caggctggcc cgcgggcccc ctgcgcggcc gcctgcactt gcgccgggga ctcgctggac      60

tgcagtgggc gcgggctggc gacgctgccc cgggacctgc cctcctggac gcgcagccta     120

aacctgagtt ataacagact ctccgagatc gactctgctg cttttgagga cttgacgaat     180

ctgcaggaag tgtacctcaa cagcaatgag ctgacagcca taccatcact gggcgctgct     240

tccataggag ttgtctctct ctttttgcag cacaacaaga tccttagtgt ggatgggagc     300

cagctgaagt cgtacctgtc cttggaagtg ctggatctga gttccaacaa catcacggaa     360

attcggagct cctgtttccc gaacggcctg cgtataaggg aactcaactt ggcgagcaac     420

cgcatcagca tcctggagtc tggagcattt gatggtctgt cgcggtcact gctgactctc     480

cgtctgagca aaaacaggat cacccagctt cctgtgaaag cgttcaagct acccaggctg     540

acacaactag acctgaatcg gaatcggatt cggctgattg aaggcctcac gttccagggg     600

ctcgacagct tagaggtgct gaggcttcag aggaacaaca tcagcaggct gacggacggg     660

gccttctggg ggctgtctaa gatgcacgtg ctgcacctgg agtacaacag tctggtggaa     720

gtgaacagtg gctccctcta tggcctcaca gccctgcacc agctgcacct cagcaacaac     780

tccatctctc gaattcagcg tgatggctgg agcttctgcc aaaagctgca tgagttgatt     840

ctgtccttca acaacctcac gcggctggat gaggagagtc tagcggagtt gagcagcctc     900

agtatcctgc gcctcagtca caacgccatc agtcacattg ctgaaggcgc cttcaaggga     960

ctcaagagtc tgcgggtctt ggacctggac cataacgaga tctcgggtac aatcgaggat    1020

accagtggtg cctttacggg gcttgacaac ctcagcaagc tgactctgtt tggaaacaag 1080

atcaaatctg tggctaagag agccttctcg ggcctggaaa gcctggaaca cctgaacctt 1140

ggagagaatg caatcaggtc tgtccagttt gatgcctttg caaagatgaa gaaccttaaa 1200

gagctctaca tcagcagtga gagcttcctg tgtgactgcc agctcaagtg gctgccccca 1260

tggctaatgg gtaggatgct gcaggccttt gtgacagcca cctgtgccca tccagagtcg 1320

ctgaagggcc agagcatttt ctcagtgctg ccagacagct ttgtgtgtga tgactttcca 1380

aagccacaga tcatcaccca gcctgagacg accatggctg tggtgggcaa ggacatccgt 1440

ttcacatgct ccgcagccag cagcagcagc tcaccaatga ccttcgcctg gaagaaggac 1500

aatgaggtcc tggccaatgc agacatggag aactttgccc acgtccgtgc acaggacggc 1560

gaagtgatgg agtataccac tatcctgcac ctccgtcacg tcacctttgg gcacgagggc 1620

cgctaccagt gtatcatcac aaaccacttt ggctccacat actcccacaa agccaggctc 1680

actgtgaatg tgttgccatc attcactaaa ataccccatg acattgccat ccggactggc 1740

accacagccc gcctcgagtg tgctgccacg ggccacccta accctcagat tgcctggcag 1800

aaggatggag gcaccgattt cccggcagct cgtgagcgac gcatgcatgt tatgccagac 1860

gatgatgtgt tcttcatcac tgatgtgaaa atagacgaca tgggggtcta cagctgcact 1920

gcccagaact cggcaggctc ggtttcagcc aacgctaccc tcacagtctt agaaactcca 1980

tccttggcag tgcctctgga agaccgtgtg gtaactgtgg gagaaacagt ggccttccag 2040

tgcaaagcaa ccgggagccc cacaccacgc atcacctggc ttaagggagg tcgcccattg 2100

agcctcacag agcgccacca tttcactcca ggcaaccagc tgctggttgt tcagaatgtg 2160

atgatagacg atgcagggcg gtatacctgt gagatgtcta atcccctggg cactgagcga 2220

gcacatagcc agctgagcat tttacctacc cctggctgcc ggaaggatgg gaccaccgta 2280

ggc 2283

<210> 5
<211> 5
<212> PRT
<213> Artificial Sequence

<220>
<223> A7 heavy chain CDR 1


<400> 5
Gly Tyr Asp Met Ser
1               5


<210> 6
<211> 9
<212> PRT

<213>    Artificial Sequence

<220>
<223>    A7 heavy chain CDR 2


<400>    6
Leu Ile Tyr Pro Asp Ser Gly Asn Lys
1               5


<210>    7
<211>    13
<212>    PRT
<213>    Artificial Sequence

<220>
<223>    A7 heavy chain CDR 3


<400>    7
Arg Asp Ala Gly Leu Ser Trp Ala Gly Ala Phe Asp Tyr
1               5                   10


<210>    8
<211>    12
<212>    PRT
<213>    Artificial Sequence

<220>
<223>    A7 light chain CDR 1


<400>    8
Ser Gly Ser Ser Ser Asn Ile Gly Ser Asn Tyr Val
1               5                   10


<210>    9
<211>    4
<212>    PRT
<213>    Artificial Sequence

<220>
<223>    A7 light chain CDR 2


<400>    9
Ser Asp Ser His
1


<210>    10
<211>    9
<212>    PRT
<213>    Artificial Sequence

<220>
<223>    A7 light chain CDR 3


<400>    10

Gly Ser Trp Asp Tyr Ser Leu Ser Ala
1               5

<210>   11
<211>   121
<212>   PRT
<213>   Artificial Sequence

<220>
<223>   A7 heavy chain_variable region

<400>   11
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Gly Tyr
            20                  25                  30

Asp Met Ser Trp Val Arg Gln Ala Pro Gly Lys Gly Leu Glu Trp Val
        35                  40                  45

Ser Leu Ile Tyr Pro Asp Ser Gly Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Ala Gly Leu Ser Trp Ala Gly Ala Phe Asp Tyr Trp Gly
            100                 105                 110

Gln Gly Thr Leu Val Thr Val Ser Ser
        115                 120

<210>   12
<211>   110
<212>   PRT
<213>   Artificial Sequence

<220>
<223>   A7 light chain_variable region

<400>   12
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Tyr Val Thr Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Gly Ser Trp Asp Tyr Ser Leu
                85                  90                  95

Ser Ala Tyr Val Phe Gly Gly Gly Thr Lys Leu Thr Val Leu
            100                 105                 110

<210>    13
<211>    5
<212>    PRT
<213>    Artificial Sequence

<220>
<223>    C8 heavy chain CDR 1

<400>    13
Asn Tyr Tyr Met Ser
1               5

<210>    14
<211>    8
<212>    PRT
<213>    Artificial Sequence

<220>
<223>    C8 heavy chain CDR 2

<400>    14
Gly Ile Ser Pro Gly Asp Ser Ser
1               5

<210>    15
<211>    13
<212>    PRT
<213>    Artificial Sequence

<220>
<223>    C8 heavy chain CDR 3

<400>    15
Lys Gly Leu Tyr Ser Asn Pro Asn Glu Pro Phe Asp Tyr
1               5                   10

<210>    16
<211>    13
<212>    PRT
<213>    Artificial Sequence

<220>
<223>    C8 light chain CDR 1

<400>    16
Thr Gly Ser Ser Ser Asn Ile Gly Ser Asn Tyr Val Ser
1               5                   10

<210>    17
<211>    4
<212>    PRT
<213>    Artificial Sequence

<220>
<223>    C8 light chain CDR 2


<400>    17
Asp Asp Ser Gln
1


<210>    18
<211>    9
<212>    PRT
<213>    Artificial Sequence

<220>
<223>    C8 light chain CDR 3


<400>    18
Gly Thr Trp Asp Tyr Ser Leu Asn Gly
1               5


<210>    19
<211>    121
<212>    PRT
<213>    Artificial Sequence

<220>
<223>    C8 heavy chain_variable region


<400>    19
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Tyr Met Ser Trp Val Arg Gln Ala Pro Gly Lys Gly Leu Glu Trp Val
        35                  40                  45

Ser Gly Ile Ser Pro Gly Asp Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Leu Tyr Ser Asn Pro Asn Glu Pro Phe Asp Tyr Trp Gly
            100                 105                 110

Gln Gly Thr Leu Val Thr Val Ser Ser
        115                 120

<210>    20
<211>    110
<212>    PRT
<213>    Artificial Sequence

<220>
<223>    C8 light chain_variable region


<400>    20
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Asp Asp Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Asn Gly Tyr Val Phe Gly Gly Gly Thr Lys Leu Thr Val Leu
            100                 105                 110


<210>    21
<211>    5
<212>    PRT
<213>    Artificial Sequence

<220>
<223>    E7 heavy chain CDR 1


<400>    21
Ser Tyr Asp Met Ser
1               5


<210>    22
<211>    9
<212>    PRT
<213>    Artificial Sequence

<220>
<223>    E7 heavy chain CDR 2


<400>    22
Gly Ile Ser Pro Asp Gly Ser Asn Ile
1               5


<210>    23
<211>    19

<212>    PRT
<213>    Artificial Sequence

<220>
<223>    E7 heavy chain CDR 3


<400>    23
Lys Val Gly Leu Arg Cys Arg Tyr Glu Ala Cys Ser Tyr Ala Tyr Gly
1               5                   10                  15

Met Asp Val




<210>    24
<211>    13
<212>    PRT
<213>    Artificial Sequence

<220>
<223>    E7 light chain CDR 1


<400>    24
Ser Gly Ser Ser Ser Asn Ile Gly Ser Asn Tyr Val Ser
1               5                   10


<210>    25
<211>    4
<212>    PRT
<213>    Artificial Sequence

<220>
<223>    E7 light chain CDR 2


<400>    25
Ser Asp Ser His
1


<210>    26
<211>    9
<212>    PRT
<213>    Artificial Sequence

<220>
<223>    E7 light chain CDR 3


<400>    26
Ala Thr Trp Asp Ser Ser Leu Asn Gly
1               5


<210>    27
<211>    127
<212>    PRT
<213>    Artificial Sequence

<220>

<223>    E7 heavy chain_variable region

<400>    27
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Asp Met Ser Trp Val Arg Gln Ala Pro Gly Lys Gly Leu Glu Trp Val
        35                  40                  45

Ser Gly Ile Ser Pro Asp Gly Ser Asn Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Gly Leu Arg Cys Arg Tyr Glu Ala Cys Ser Tyr Ala Tyr
            100                 105                 110

Gly Met Asp Val Trp Gly Gln Gly Thr Leu Val Thr Val Ser Ser
        115                 120                 125

<210>    28
<211>    110
<212>    PRT
<213>    Artificial Sequence

<220>
<223>    E7 light chain_variable region

<400>    28
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Thr Trp Asp Ser Ser Leu
                85                  90                  95

Asn Gly Tyr Val Phe Gly Gly Gly Thr Lys Leu Thr Val Leu
            100                 105                 110

<210>    29
<211>    5
<212>    PRT
<213>    Artificial Sequence

<220>
<223>    G3 heavy chain CDR 1


<400>    29
Asn Tyr Asp Met Ser
1               5


<210>    30
<211>    9
<212>    PRT
<213>    Artificial Sequence

<220>
<223>    G3 heavy chain CDR 2


<400>    30
Ser Ile Ser Pro Ser Ser Gly Ser Ile
1               5


<210>    31
<211>    13
<212>    PRT
<213>    Artificial Sequence

<220>
<223>    G3 heavy chain CDR 3


<400>    31
Lys Asp Leu Asp Ala Phe Trp Arg Pro Ser Phe Asp Tyr
1               5                   10


<210>    32
<211>    13
<212>    PRT
<213>    Artificial Sequence

<220>
<223>    G3 light chain CDR 1


<400>    32
Thr Gly Ser Ser Ser Asn Ile Gly Asn Asn Asn Val Asn
1               5                   10


<210>    33
<211>    4
<212>    PRT
<213>    Artificial Sequence

<220>
<223>    G3 light chain CDR 2

<400>    33
Ser Asp Ser His
1

<210>    34
<211>    9
<212>    PRT
<213>    Artificial Sequence

<220>
<223>    G3 light chain CDR 3

<400>    34
Gly Ser Trp Asp Asp Ser Leu Ser Ala
1               5

<210>    35
<211>    121
<212>    PRT
<213>    Artificial Sequence

<220>
<223>    G3 heavy chain_variable region

<400>    35
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Asp Met Ser Trp Val Arg Gln Ala Pro Gly Lys Gly Leu Glu Trp Val
        35                  40                  45

Ser Ser Ile Ser Pro Ser Ser Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Leu Asp Ala Phe Trp Arg Pro Ser Phe Asp Tyr Trp Gly
            100                 105                 110

Gln Gly Thr Leu Val Thr Val Ser Ser
        115                 120

<210>    36
<211>    110
<212>    PRT
<213>    Artificial Sequence

<220>
<223>    G3 light chain_variable region

<400>    36
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Asn Val Asn Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Asp Ser Leu
                85                  90                  95

Ser Ala Tyr Val Phe Gly Gly Gly Thr Lys Leu Thr Val Leu
            100                 105                 110


<210>    37
<211>    328
<212>    PRT
<213>    Mus musculus

<400>    37
Thr Thr Ala Pro Ser Val Tyr Pro Leu Ala Pro Val Cys Gly Asp Thr
1               5                   10                  15

Thr Gly Ser Ser Val Thr Leu Gly Cys Leu Val Lys Gly Tyr Phe Pro
            20                  25                  30

Glu Pro Val Thr Leu Thr Trp Asn Ser Gly Ser Leu Ser Ser Gly Val
        35                  40                  45

His Thr Phe Pro Ala Val Leu Gln Ser Asp Leu Tyr Thr Leu Ser Ser
    50                  55                  60

Ser Val Thr Val Thr Ser Ser Thr Trp Pro Ser Gln Ser Ile Thr Cys
65                  70                  75                  80

Asn Val Ala His Pro Ala Ser Ser Thr Lys Val Asp Lys Lys Ile Glu
                85                  90                  95

Pro Arg Gly Pro Thr Ile Lys Pro Cys Pro Pro Cys Lys Cys Pro Ala
            100                 105                 110

Pro Asn Leu Leu Gly Gly Pro Ser Val Phe Ile Phe Pro Pro Lys Ile
        115                 120                 125

Lys Asp Val Leu Met Ile Ser Leu Ser Pro Ile Val Thr Cys Val Val
    130                 135                 140

Val Asp Val Ser Glu Asp Asp Pro Asp Val Gln Ile Ser Trp Phe Val
145                 150                 155                 160

Asn Asn Val Glu Val His Thr Ala Gln Thr Gln Thr His Arg Glu Asp
                165                 170                 175

Tyr Asn Ser Thr Leu Arg Val Val Ser Ala Leu Pro Ile Gln His Gln
            180                 185                 190

Asp Trp Met Ser Gly Lys Glu Phe Lys Cys Lys Val Asn Asn Lys Asp
        195                 200                 205

Leu Pro Ala Pro Ile Glu Arg Thr Ile Ser Lys Pro Lys Gly Ser Val
    210                 215                 220

Arg Ala Pro Gln Val Tyr Val Leu Pro Pro Pro Glu Glu Glu Met Thr
225                 230                 235                 240

Lys Lys Gln Val Thr Leu Thr Cys Met Val Thr Asp Phe Met Pro Glu
                245                 250                 255

Asp Ile Tyr Val Glu Trp Thr Asn Asn Gly Lys Thr Glu Leu Asn Tyr
            260                 265                 270

Lys Asn Thr Glu Pro Val Leu Asp Ser Asp Gly Ser Tyr Phe Met Tyr
        275                 280                 285

Ser Lys Leu Arg Val Glu Lys Lys Asn Trp Val Glu Arg Asn Ser Tyr
    290                 295                 300

Ser Cys Ser Val Val His Glu Gly Leu His Asn His His Thr Thr Lys
305                 310                 315                 320

Ser Phe Ser Arg Thr Pro Gly Lys
                325


<210>    38
<211>    107
<212>    PRT
<213>    Mus musculus

<400>    38
Arg Thr Val Ala Ala Pro Thr Val Ser Ile Phe Pro Pro Ser Ser Glu
1               5                   10                  15

Gln Leu Thr Ser Gly Gly Ala Ser Val Val Cys Phe Leu Asn Asn Phe
            20                  25                  30

Tyr Pro Lys Asp Ile Asn Val Lys Trp Lys Ile Asp Gly Ser Glu Arg
        35                  40                  45

Gln Asn Gly Val Leu Asn Ser Trp Thr Asp Gln Asp Ser Lys Asp Ser
    50                  55                  60

Thr Tyr Ser Met Ser Ser Thr Leu Thr Leu Thr Lys Asp Glu Tyr Glu
65                  70                  75                  80

Arg His Asn Ser Tyr Thr Cys Glu Ala Thr His Lys Thr Ser Thr Ser
                85                  90                  95

Pro Ile Val Lys Ser Phe Asn Arg Asn Glu Cys
            100                 105


<210>    39
<211>    330
<212>    PRT

<213>    homo sapiens

<400>    39

<210>    40
<211>    107
<212>    PRT
<213>    Homo sapiens

<400>    40

<210>    41
<211>    326
<212>    PRT
<213>    Homo sapiens

<400>    41
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp

130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ser Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325

<210>    42
<211>    377
<212>    PRT
<213>    Homo sapiens

<400>    42
Ala Ser Thr Lys Gly Pro Ser Val Phe Pro Leu Ala Pro Cys Ser Arg
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                85                  90                  95

Arg Val Glu Leu Lys Thr Pro Leu Gly Asp Thr Thr His Thr Cys Pro
            100                 105                 110

Arg Cys Pro Glu Pro Lys Ser Cys Asp Thr Pro Pro Pro Cys Pro Arg
        115                 120                 125

Cys Pro Glu Pro Lys Ser Cys Asp Thr Pro Pro Pro Cys Pro Arg Cys
    130                 135                 140

Pro Glu Pro Lys Ser Cys Asp Thr Pro Pro Pro Cys Pro Arg Cys Pro
145                 150                 155                 160

Ala Pro Glu Leu Leu Gly Gly Pro Ser Val Phe Leu Phe Pro Pro Lys
                165                 170                 175

Pro Lys Asp Thr Leu Met Ile Ser Arg Thr Pro Glu Val Thr Cys Val
            180                 185                 190

Val Val Asp Val Ser His Glu Asp Pro Glu Val Gln Phe Lys Trp Tyr
        195                 200                 205

Val Asp Gly Val Glu Val His Asn Ala Lys Thr Lys Pro Arg Glu Glu
    210                 215                 220

Gln Tyr Asn Ser Thr Phe Arg Val Val Ser Val Leu Thr Val Leu His
225                 230                 235                 240

Gln Asp Trp Leu Asn Gly Lys Glu Tyr Lys Cys Lys Val Ser Asn Lys
                245                 250                 255

Ala Leu Pro Ala Pro Ile Glu Lys Thr Ile Ser Lys Thr Lys Gly Gln
            260                 265                 270

Pro Arg Glu Pro Gln Val Tyr Thr Leu Pro Pro Ser Arg Glu Glu Met
        275                 280                 285

Thr Lys Asn Gln Val Ser Leu Thr Cys Leu Val Lys Gly Phe Tyr Pro
    290                 295                 300

Ser Asp Ile Ala Val Glu Trp Glu Ser Ser Gly Gln Pro Glu Asn Asn
305                 310                 315                 320

Tyr Asn Thr Thr Pro Pro Met Leu Asp Ser Asp Gly Ser Phe Phe Leu
                325                 330                 335

Tyr Ser Lys Leu Thr Val Asp Lys Ser Arg Trp Gln Gln Gly Asn Ile
            340                 345                 350

Phe Ser Cys Ser Val Met His Glu Ala Leu His Asn Arg Phe Thr Gln
        355                 360                 365

Lys Ser Leu Ser Leu Ser Pro Gly Lys
    370                 375


<210>   43
<211>   327
<212>   PRT
<213>   Homo sapiens

<400>   43
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>    44

<211>    245
<212>    PRT
<213>    Artificial Sequence

<220>
<223>    Hybrid Fc_Heavy region


<400>    44
Arg Asn Thr Gly Arg Gly Gly Glu Glu Lys Lys Lys Glu Lys Glu Lys
1               5                   10                  15

Glu Glu Gln Glu Glu Arg Glu Thr Lys Thr Pro Glu Cys Pro Ser His
            20                  25                  30

Thr Gln Pro Leu Gly Val Phe Leu Phe Pro Pro Lys Pro Lys Asp Thr
        35                  40                  45

Leu Met Ile Ser Arg Thr Pro Glu Val Thr Cys Val Val Val Asp Val
    50                  55                  60

Ser Gln Glu Asp Pro Glu Val Gln Phe Asn Trp Tyr Val Asp Gly Val
65                  70                  75                  80

Glu Val His Asn Ala Lys Thr Lys Pro Arg Glu Glu Gln Phe Asn Ser
                85                  90                  95

Thr Tyr Arg Val Val Ser Val Leu Thr Val Leu His Gln Asp Trp Leu
            100                 105                 110

Asn Gly Lys Glu Tyr Lys Cys Lys Val Ser Asn Lys Gly Leu Pro Ser
        115                 120                 125

Ser Ile Glu Lys Thr Ile Ser Lys Ala Lys Gly Gln Pro Arg Glu Pro
    130                 135                 140

Gln Val Tyr Thr Leu Pro Pro Ser Gln Glu Glu Met Thr Lys Asn Gln
145                 150                 155                 160

Val Ser Leu Thr Cys Leu Val Lys Gly Phe Tyr Pro Ser Asp Ile Ala
                165                 170                 175

Val Glu Trp Glu Ser Asn Gly Gln Pro Glu Asn Asn Tyr Lys Thr Thr
            180                 185                 190

Pro Pro Val Leu Asp Ser Asp Gly Ser Phe Phe Leu Tyr Ser Arg Leu
        195                 200                 205

Thr Val Asp Lys Ser Arg Trp Gln Glu Gly Asn Val Phe Ser Cys Ser
    210                 215                 220

Val Met His Glu Ala Leu His Asn His Tyr Thr Gln Lys Ser Leu Ser
225                 230                 235                 240

Leu Ser Leu Gly Lys
                245


<210>    45
<211>    449
<212>    PRT
<213>    Artificial Sequence

<220>
<223>    A7 heavy chain_mouse IgG2 Fc_full sequence

<400>    45
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Gly Tyr
            20                  25                  30

Asp Met Ser Trp Val Arg Gln Ala Pro Gly Lys Gly Leu Glu Trp Val
        35                  40                  45

Ser Leu Ile Tyr Pro Asp Ser Gly Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Ala Gly Leu Ser Trp Ala Gly Ala Phe Asp Tyr Trp Gly
            100                 105                 110

Gln Gly Thr Leu Val Thr Val Ser Ser Thr Thr Ala Pro Ser Val Tyr
        115                 120                 125

Pro Leu Ala Pro Val Cys Gly Asp Thr Thr Gly Ser Ser Val Thr Leu
    130                 135                 140

Gly Cys Leu Val Lys Gly Tyr Phe Pro Glu Pro Val Thr Leu Thr Trp
145                 150                 155                 160

Asn Ser Gly Ser Leu Ser Ser Gly Val His Thr Phe Pro Ala Val Leu
                165                 170                 175

Gln Ser Asp Leu Tyr Thr Leu Ser Ser Ser Val Thr Val Thr Ser Ser
            180                 185                 190

Thr Trp Pro Ser Gln Ser Ile Thr Cys Asn Val Ala His Pro Ala Ser
        195                 200                 205

Ser Thr Lys Val Asp Lys Lys Ile Glu Pro Arg Gly Pro Thr Ile Lys
    210                 215                 220

Pro Cys Pro Pro Cys Lys Cys Pro Ala Pro Asn Leu Leu Gly Gly Pro
225                 230                 235                 240

Ser Val Phe Ile Phe Pro Pro Lys Ile Lys Asp Val Leu Met Ile Ser
                245                 250                 255

Leu Ser Pro Ile Val Thr Cys Val Val Val Asp Val Ser Glu Asp Asp
            260                 265                 270

Pro Asp Val Gln Ile Ser Trp Phe Val Asn Asn Val Glu Val His Thr
        275                 280                 285

Ala Gln Thr Gln Thr His Arg Glu Asp Tyr Asn Ser Thr Leu Arg Val
    290                 295                 300

Val Ser Ala Leu Pro Ile Gln His Gln Asp Trp Met Ser Gly Lys Glu

305                 310                 315                 320

Phe Lys Cys Lys Val Asn Asn Lys Asp Leu Pro Ala Pro Ile Glu Arg
                325                 330                 335

Thr Ile Ser Lys Pro Lys Gly Ser Val Arg Ala Pro Gln Val Tyr Val
            340                 345                 350

Leu Pro Pro Pro Glu Glu Glu Met Thr Lys Lys Gln Val Thr Leu Thr
        355                 360                 365

Cys Met Val Thr Asp Phe Met Pro Glu Asp Ile Tyr Val Glu Trp Thr
    370                 375                 380

Asn Asn Gly Lys Thr Glu Leu Asn Tyr Lys Asn Thr Glu Pro Val Leu
385                 390                 395                 400

Asp Ser Asp Gly Ser Tyr Phe Met Tyr Ser Lys Leu Arg Val Glu Lys
                405                 410                 415

Lys Asn Trp Val Glu Arg Asn Ser Tyr Ser Cys Ser Val Val His Glu
            420                 425                 430

Gly Leu His Asn His His Thr Thr Lys Ser Phe Ser Arg Thr Pro Gly
        435                 440                 445

Lys

<210>    46
<211>    217
<212>    PRT
<213>    Artificial Sequence

<220>
<223>    A7 light chain_mouse IgG2 Fc_full sequence

<400>    46
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Tyr Val Thr Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Gly Ser Trp Asp Tyr Ser Leu
                85                  90                  95

Ser Ala Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>    47
<211>    449
<212>    PRT
<213>    Artificial Sequence

<220>
<223>    C8 heavy chain_mouse IgG2 Fc_full sequence


<400>    47
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Tyr Met Ser Trp Val Arg Gln Ala Pro Gly Lys Gly Leu Glu Trp Val
        35                  40                  45

Ser Gly Ile Ser Pro Gly Asp Ser Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Leu Tyr Ser Asn Pro Asn Glu Pro Phe Asp Tyr Trp Gly
            100                 105                 110

Gln Gly Thr Leu Val Thr Val Ser Ser Thr Thr Ala Pro Ser Val Tyr
        115                 120                 125

Pro Leu Ala Pro Val Cys Gly Asp Thr Thr Gly Ser Ser Val Thr Leu
    130                 135                 140

Gly Cys Leu Val Lys Gly Tyr Phe Pro Glu Pro Val Thr Leu Thr Trp
145                 150                 155                 160

Asn Ser Gly Ser Leu Ser Ser Gly Val His Thr Phe Pro Ala Val Leu
                165                 170                 175

Gln Ser Asp Leu Tyr Thr Leu Ser Ser Ser Val Thr Val Thr Ser Ser
            180                 185                 190

Thr Trp Pro Ser Gln Ser Ile Thr Cys Asn Val Ala His Pro Ala Ser
        195                 200                 205

Ser Thr Lys Val Asp Lys Lys Ile Glu Pro Arg Gly Pro Thr Ile Lys
    210                 215                 220

Pro Cys Pro Pro Cys Lys Cys Pro Ala Pro Asn Leu Leu Gly Gly Pro
225                 230                 235                 240

Ser Val Phe Ile Phe Pro Pro Lys Ile Lys Asp Val Leu Met Ile Ser
                245                 250                 255

Leu Ser Pro Ile Val Thr Cys Val Val Val Asp Val Ser Glu Asp Asp
            260                 265                 270

Pro Asp Val Gln Ile Ser Trp Phe Val Asn Asn Val Glu Val His Thr
        275                 280                 285

Ala Gln Thr Gln Thr His Arg Glu Asp Tyr Asn Ser Thr Leu Arg Val
    290                 295                 300

Val Ser Ala Leu Pro Ile Gln His Gln Asp Trp Met Ser Gly Lys Glu
305                 310                 315                 320

Phe Lys Cys Lys Val Asn Asn Lys Asp Leu Pro Ala Pro Ile Glu Arg
                325                 330                 335

Thr Ile Ser Lys Pro Lys Gly Ser Val Arg Ala Pro Gln Val Tyr Val
            340                 345                 350

Leu Pro Pro Pro Glu Glu Glu Met Thr Lys Lys Gln Val Thr Leu Thr
        355                 360                 365

Cys Met Val Thr Asp Phe Met Pro Glu Asp Ile Tyr Val Glu Trp Thr
    370                 375                 380

Asn Asn Gly Lys Thr Glu Leu Asn Tyr Lys Asn Thr Glu Pro Val Leu
385                 390                 395                 400

Asp Ser Asp Gly Ser Tyr Phe Met Tyr Ser Lys Leu Arg Val Glu Lys
                405                 410                 415

Lys Asn Trp Val Glu Arg Asn Ser Tyr Ser Cys Ser Val Val His Glu
            420                 425                 430

Gly Leu His Asn His His Thr Thr Lys Ser Phe Ser Arg Thr Pro Gly
        435                 440                 445

Lys

<210>  48
<211>  217
<212>  PRT
<213>  Artificial Sequence

<220>
<223>  C8 light chain_mouse IgG2 Fc_full sequence

<400>    48
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Asp Asp Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Thr Trp Asp Tyr Ser Leu
                85                  90                  95

Asn Gly Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>    49
<211>    455
<212>    PRT
<213>    Artificial Sequence

<220>
<223>    E7 heavy chain_mouse IgG2 Fc_full sequence


<400>    49
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Asp Met Ser Trp Val Arg Gln Ala Pro Gly Lys Gly Leu Glu Trp Val
        35                  40                  45

Ser Gly Ile Ser Pro Asp Gly Ser Asn Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Gly Leu Arg Cys Arg Tyr Glu Ala Cys Ser Tyr Ala Tyr
            100                 105                 110

Gly Met Asp Val Trp Gly Gln Gly Thr Leu Val Thr Val Ser Ser Thr
        115                 120                 125

Thr Ala Pro Ser Val Tyr Pro Leu Ala Pro Val Cys Gly Asp Thr Thr
    130                 135                 140

Gly Ser Ser Val Thr Leu Gly Cys Leu Val Lys Gly Tyr Phe Pro Glu
145                 150                 155                 160

Pro Val Thr Leu Thr Trp Asn Ser Gly Ser Leu Ser Ser Gly Val His
                165                 170                 175

Thr Phe Pro Ala Val Leu Gln Ser Asp Leu Tyr Thr Leu Ser Ser Ser
            180                 185                 190

Val Thr Val Thr Ser Ser Thr Trp Pro Ser Gln Ser Ile Thr Cys Asn
        195                 200                 205

Val Ala His Pro Ala Ser Ser Thr Lys Val Asp Lys Lys Ile Glu Pro
    210                 215                 220

Arg Gly Pro Thr Ile Lys Pro Cys Pro Pro Cys Lys Cys Pro Ala Pro
225                 230                 235                 240

Asn Leu Leu Gly Gly Pro Ser Val Phe Ile Phe Pro Pro Lys Ile Lys
                245                 250                 255

Asp Val Leu Met Ile Ser Leu Ser Pro Ile Val Thr Cys Val Val Val
            260                 265                 270

Asp Val Ser Glu Asp Asp Pro Asp Val Gln Ile Ser Trp Phe Val Asn
        275                 280                 285

Asn Val Glu Val His Thr Ala Gln Thr Gln Thr His Arg Glu Asp Tyr
    290                 295                 300

Asn Ser Thr Leu Arg Val Val Ser Ala Leu Pro Ile Gln His Gln Asp
305                 310                 315                 320

Trp Met Ser Gly Lys Glu Phe Lys Cys Lys Val Asn Asn Lys Asp Leu
                325                 330                 335

Pro Ala Pro Ile Glu Arg Thr Ile Ser Lys Pro Lys Gly Ser Val Arg
            340                 345                 350

Ala Pro Gln Val Tyr Val Leu Pro Pro Pro Glu Glu Glu Met Thr Lys
        355                 360                 365

Lys Gln Val Thr Leu Thr Cys Met Val Thr Asp Phe Met Pro Glu Asp
    370                 375                 380

Ile Tyr Val Glu Trp Thr Asn Asn Gly Lys Thr Glu Leu Asn Tyr Lys
385                 390                 395                 400

Asn Thr Glu Pro Val Leu Asp Ser Asp Gly Ser Tyr Phe Met Tyr Ser
                405                 410                 415

Lys Leu Arg Val Glu Lys Lys Asn Trp Val Glu Arg Asn Ser Tyr Ser
            420                 425                 430

Cys Ser Val Val His Glu Gly Leu His Asn His His Thr Thr Lys Ser
        435                 440                 445

Phe Ser Arg Thr Pro Gly Lys
    450                 455


<210>    50
<211>    217
<212>    PRT
<213>    Artificial Sequence

<220>
<223>    E7 light chain_mouse IgG2 Fc_full sequence


<400>    50
Gln Ser Val Leu Thr Gln Pro Pro Ser Ala Ser Gly Thr Pro Gly Gln
 1               5                  10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Thr Trp Asp Ser Ser Leu
                85                  90                  95

Asn Gly Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215

<210>  51
<211>  449
<212>  PRT
<213>  Artificial Sequence

<220>
<223>  G3 heavy chain_mouse IgG2 Fc_full sequence

<400>  51
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Asp Met Ser Trp Val Arg Gln Ala Pro Gly Lys Gly Leu Glu Trp Val
        35                  40                  45

Ser Ser Ile Ser Pro Ser Ser Gly Ser Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Leu Asp Ala Phe Trp Arg Pro Ser Phe Asp Tyr Trp Gly
            100                 105                 110

Gln Gly Thr Leu Val Thr Val Ser Ser Thr Thr Ala Pro Ser Val Tyr
        115                 120                 125

Pro Leu Ala Pro Val Cys Gly Asp Thr Thr Gly Ser Ser Val Thr Leu
    130                 135                 140

Gly Cys Leu Val Lys Gly Tyr Phe Pro Glu Pro Val Thr Leu Thr Trp
145                 150                 155                 160

Asn Ser Gly Ser Leu Ser Ser Gly Val His Thr Phe Pro Ala Val Leu
                165                 170                 175

Gln Ser Asp Leu Tyr Thr Leu Ser Ser Ser Val Thr Val Thr Ser Ser
            180                 185                 190

Thr Trp Pro Ser Gln Ser Ile Thr Cys Asn Val Ala His Pro Ala Ser
        195                 200                 205

Ser Thr Lys Val Asp Lys Lys Ile Glu Pro Arg Gly Pro Thr Ile Lys
    210                 215                 220

Pro Cys Pro Pro Cys Lys Cys Pro Ala Pro Asn Leu Leu Gly Gly Pro
225                 230                 235                 240

Ser Val Phe Ile Phe Pro Pro Lys Ile Lys Asp Val Leu Met Ile Ser

245                 250                 255

Leu Ser Pro Ile Val Thr Cys Val Val Val Asp Val Ser Glu Asp Asp
            260                 265                 270

Pro Asp Val Gln Ile Ser Trp Phe Val Asn Asn Val Glu Val His Thr
        275                 280                 285

Ala Gln Thr Gln Thr His Arg Glu Asp Tyr Asn Ser Thr Leu Arg Val
    290                 295                 300

Val Ser Ala Leu Pro Ile Gln His Gln Asp Trp Met Ser Gly Lys Glu
305                 310                 315                 320

Phe Lys Cys Lys Val Asn Asn Lys Asp Leu Pro Ala Pro Ile Glu Arg
                325                 330                 335

Thr Ile Ser Lys Pro Lys Gly Ser Val Arg Ala Pro Gln Val Tyr Val
            340                 345                 350

Leu Pro Pro Pro Glu Glu Glu Met Thr Lys Lys Gln Val Thr Leu Thr
        355                 360                 365

Cys Met Val Thr Asp Phe Met Pro Glu Asp Ile Tyr Val Glu Trp Thr
    370                 375                 380

Asn Asn Gly Lys Thr Glu Leu Asn Tyr Lys Asn Thr Glu Pro Val Leu
385                 390                 395                 400

Asp Ser Asp Gly Ser Tyr Phe Met Tyr Ser Lys Leu Arg Val Glu Lys
                405                 410                 415

Lys Asn Trp Val Glu Arg Asn Ser Tyr Ser Cys Ser Val Val His Glu
            420                 425                 430

Gly Leu His Asn His His Thr Thr Lys Ser Phe Ser Arg Thr Pro Gly
        435                 440                 445

Lys

<210>   52
<211>   217
<212>   PRT
<213>   Artificial Sequence

<220>
<223>   G3 light chain_mouse IgG2 Fc_full sequence

<400>   52
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Asn Val Asn Trp Tyr Gln Gln Leu Pro Gly Thr Ala Pro Lys Leu Leu
        35                  40                  45

Ile Tyr Ser Asp Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Asp Ser Leu
                85                  90                  95

Ser Ala Tyr Val Phe Gly Gly Gly Thr Lys Leu Thr Val Leu Arg Thr
            100                 105                 110

Val Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>    53
<211>    330
<212>    PRT
<213>    Homo sapiens

<400>    53
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys

130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330